# EUROPEAN PATENT SPECIFICATION

(11) **EP 3 307 742 B1**
(45) Date of publication and mention of the grant of the patent: **18.05.2022**
(21) Application number: 16808401.0
(22) Date of filing: 10.06.2016
(51) Int. Cl.: A61K 31/5025, A61K 31/5377, A61K 31/541, A61K 31/551, A61P 25/28, C07D 487/04, C07D 495/04

(54) **METHODS OF TREATING OR PREVENTING A PROTEOPATHY**
VERFAHREN ZUR BEHANDLUNG ODER PRÄVENTION EINER PROTEOPATHIE
MÉTHODES POUR TRAITER OU PRÉVENIR UNE PROTÉOPATHIE

(30) Priority: 11.06.2015 US 201562174332 P; 11.06.2015 US 201562174338 P
(43) Date of publication of application: 18.04.2018
(73) Proprietor: Usher III Initiative, Inc., Chicago, Illinois 60606 (US)
(72) Inventor: SAPERSTEIN, David, Seattle, Washington 98105 (US)
(74) Representative: Cooley (UK) LLP
(86) International application number: PCT/US2016/036945
(87) International publication number: WO 2016/201266

(56) References cited:
- US-A1- 2013 252 936
- US-A1- 2014 121 197
- US-A1- 2014 121 205
- US-A1- 2014 288 005
- US-A1- 2014 288 093
- SHELKOVNIKOVA T A ET AL: "Proteinopathies, neurodegenerative disorders with protein aggregation-based pathology", MOLECULAR BIOLOGY, KLUWER ACADEMIC PUBLISHERS-PLENUM PUBLISHERS, NE, vol. 46, no. 3, 14 June 2012 (2012-06-14), pages 362-374, XP035072111, ISSN: 1608-3245, DOI: 10.1134/S0026893312020161

## Description

### CROSS REFERENCE TO RELATED APPLICATIONS

This application claims the benefit of U.S provisional application no. 62/174,332, filed June 11, 2015, and U.S. provisional application no. 62/174,338, filed June 11, 2015.

### BACKGROUND OF THE INVENTION

The proper functioning of organs and cells within an organism relies on the proper function of proteins. A protein is a biological entity that has a primary amino acid sequence; a secondary structure that forms protein domains and includes, most importantly, alpha helices and beta sheets; and a tertiary structure, a result of a complex folding of the peptide chain in three dimensions that involve the polypeptide chain backbone and amino acid side chain interactions. Some proteins work in a multi-subunit complex, where the arrangement of multiple proteins into a quaternary structure becomes crucial for their proper function.

The failure of proteins to fold into correct three-dimensional structures can lead to diseases called proteopathies (sometimes also referred to as proteinopathies or protein conformational disorders). The failure may be due to one or more mutations in the proteins' gene or to environmental factors such as oxidative stress, alkalosis, acidosis, pH shift and osmotic shock. The misfolding of proteins can sometimes lead to clumping or aggregation into amyloid plaques or fibrils that can exacerbate a disease. Proteopathies cover a wide spectrum of afflictions, including neurodegenerative diseases (e.g., Alzheimer's, Parkinson's, polyglutamine diseases, prion diseases); amyloidosis of other non-nervous system proteins such as α1-antitrypsin, immunoglobulin light and heavy chains, lactadherin, apolipoprotein, gelsolin, lysozyme, fibrinogen, atrial natriuretic factor, keratin, lactoferrin and beta-2 microglobulin, among others); sickle cell disease; cataracts; cystic fibrosis; retinitis pigmentosa; and nephrogenic diabetes insipidus.

Molecular chaperones are biological molecules that assist in proper protein folding, protein translocation, and/or protein degradation. Examples of molecular chaperones include the heat shock proteins, which are classified into seven different families in the human genome and include HSPH (Hsp110), HSPC (Hsp90), HSPA (Hsp70), DNAJ (Hsp40), HSPB (small Hsp (sHsp)), the human chaperonins HSPD/E (HSP60/HSP10) and CCT (TRiC).

US2014/288093 A1 discloses methods and compositions for the treatment and/or prophylaxis of neurodegenerative proteinopathies.

### SUMMARY OF THE INVENTION

The invention provides a compound of Formula II for use in a method for treating or preventing a proteopathy, comprising administering to a subject in need thereof an effective amount of the compound of Formula II: 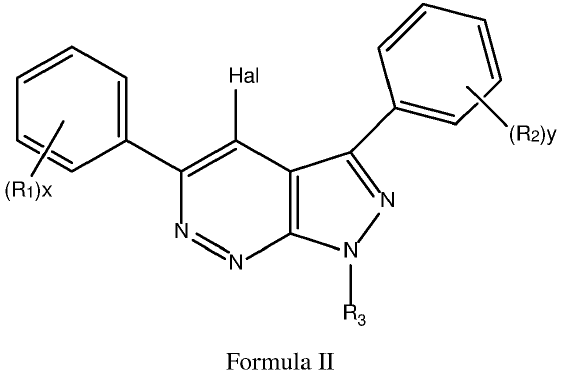 or a pharmaceutically acceptable salt thereof,
wherein Hal is -Cl, -F, -I, or -Br;
x is an integer ranging from 0 to 5;
each R₁ is independently -Cl, -F, -I, -Br, -C₁-C₃ alkyl, -O- C₁-C₃ alkyl, -CN, -CF₃, -C(O)NH(CH₃), or -C≡CCH₂OH;
y is an integer ranging from 0 to 5;
each R₂ is independently -Cl, -F, -Br, -C₁-C₃ alkyl, -O- C₁-C₃ alkyl, -CN, -CF₃, -C(O)NH(CH₃), or -C≡CCH₂OH;
R₃ is -H, -C₁-C₆ alkyl, -(C₁-C₆ alkylene)-OH, -(C₁-C₆ alkylene)-phenyl, -(C₁-C₆ alkylene)-O-(C₁-C₆ alkyl), -C₂-C₆ alkenyl, -(C₁-C₆ alkylene)-C(O)R₄,
-(C₁-C₆ alkylene)-R₅, 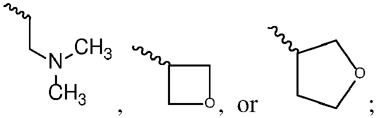
R₄ is -OH, -O-(C₁-C₆ alkyl), -NH₂, -NH(C₁-C₆ alkyl), -NH((C₁-C₆ alkylene)-OH), -NH((C₁-C₆ alkylene)N(C₁-C₆ alkyl)₂), -N(C₁-C₆ alkyl)((C₁-C₆ alkylene)-CN),-N(C₁-C₆ alkyl)((C₁-C₆ alkylene)N(C₁-C₆ alkyl)₂), -NH(C₁-C₆ alkylene)-O-(C₁-C₆ alkyl), 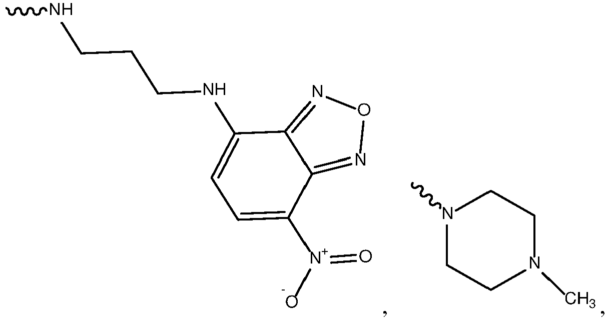 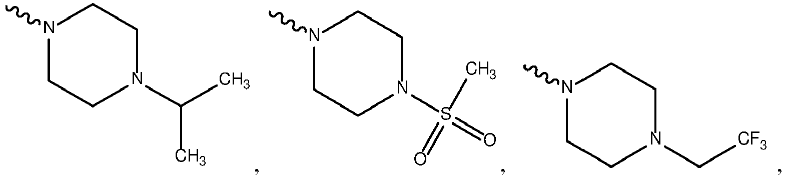 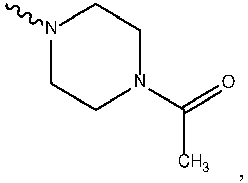 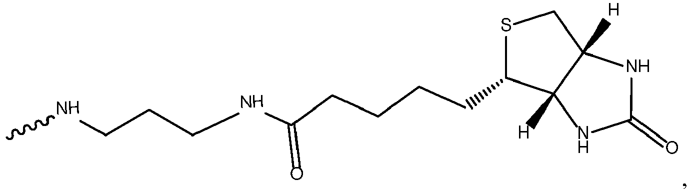 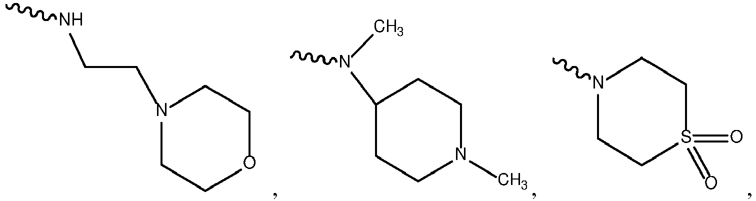 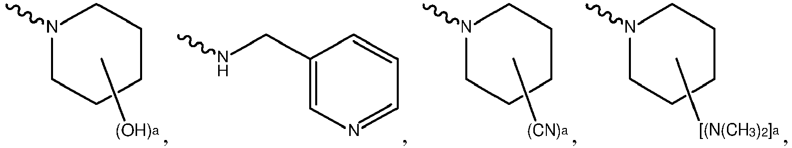 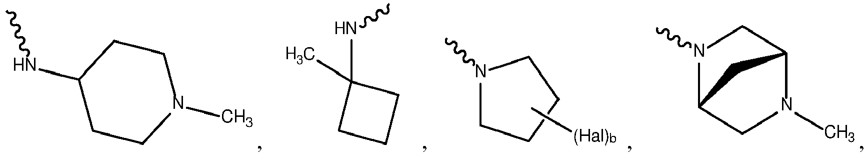 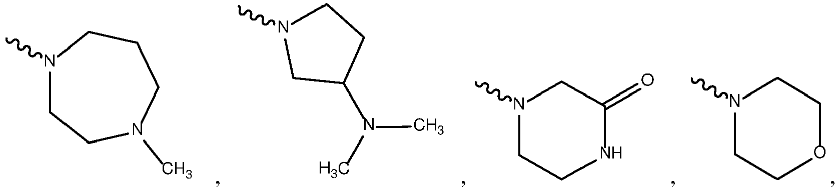 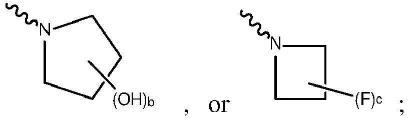
a is an integer ranging from 0 to 10 ;
b is an integer ranging from 0 to 8 ;
c is an integer ranging from 0 to 6; and
R₅ is 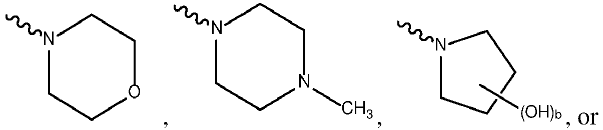 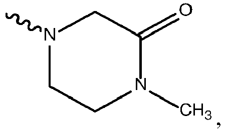
wherein the proteopathy is not retinitis pigmentosa, Leber's congenital amaurosis, a syndromic retinal degeneration, age-related macular degeneration, Usher syndrome or a retinal degenerative disease.

In some embodiments, the compound has the structure: 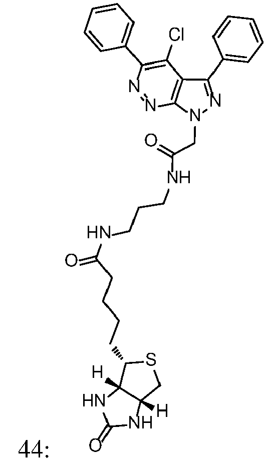 , or a pharmaceutically acceptable salt thereof.

Also described herein (not in accordance with the invention) are methods for treating or preventing a proteopathy, comprising administering to a subject in need thereof an effective amount of a compound of Formula I: 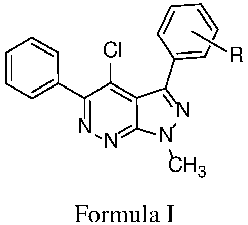 or a pharmaceutically acceptable salt thereof,
wherein R is fluoro, chloro, iodo, methyl, methoxy, cyano, trifluoromethyl, or -(CO)NH(CH₃).

Also described herein (not in accordance with the invention) are methods for treating or preventing a proteopathy, comprising administering to a subject in need thereof an effective amount of a compound of Formula III: 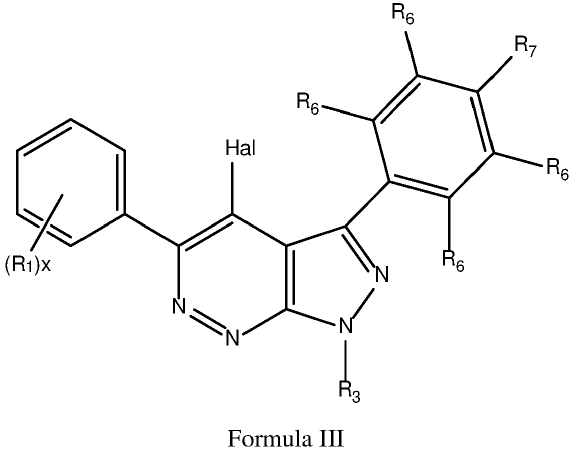 or a pharmaceutically acceptable salt thereof,
wherein Hal is -Cl, -F, -I, or -Br;
x is an integer ranging from 0 to 5;
each R₁ is independently -Cl, -F, -I, -Br, -C₁-C₃ alkyl, -O- C₁-C₃ alkyl, -CN, -CF₃, -C(O)NH(CH₃), or -C≡CCH₂OH;
R₃ is -H, -C₁-C₆ alkyl, -(C₁-C₆ alkylene)-OH, -(C₁-C₆ alkylene)-phenyl, -(C₁-C₆ alkylene)-O-(C₁-C₆ alkyl), -C₂-C₆ alkenyl, -(C₁-C₆ alkylene)-C(O)R₄,
-(C₁-C₆ alkylene)-R₅, 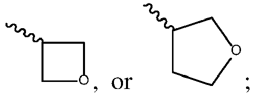
R₄ is -OH, -O-(C₁-C₆ alkyl), -NH₂, -NH(C₁-C₆ alkyl), -NH((C₁-C₆ alkylene)-OH), -NH((C₁-C₆ alkylene)N(C₁-C₆ alkyl)₂), -N(C₁-C₆ alkyl)((C₁-C₆ alkylene)-CN),-N(C₁-C₆ alkyl)((C₁-C₆ alkylene)N(C₁-C₆ alkyl)₂), -NH(C₁-C₆ alkylene)-O-(C₁-C₆ alkyl), 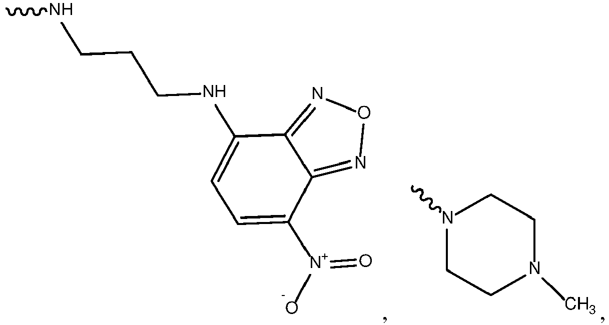 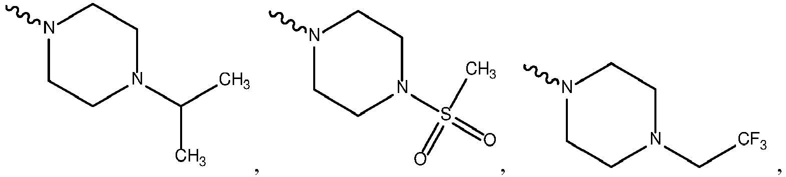 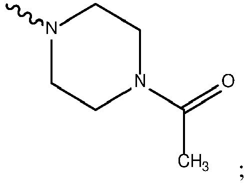 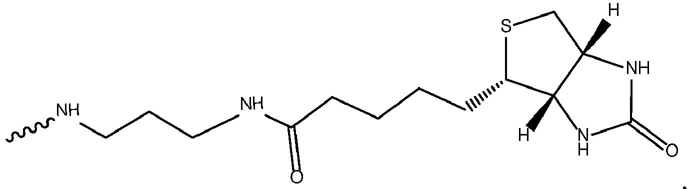 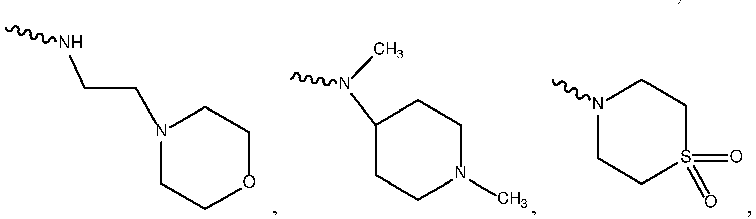 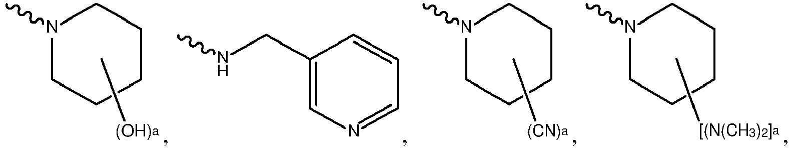 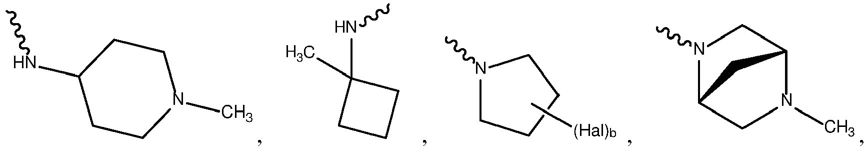 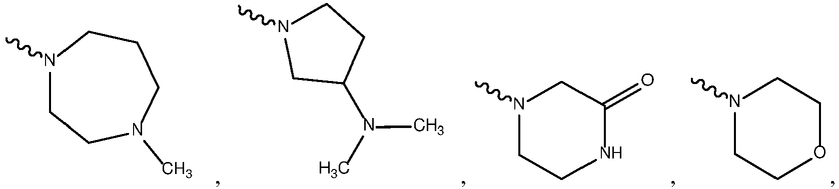 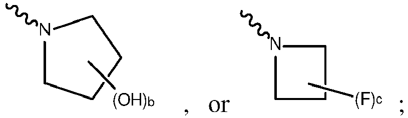
a is an integer ranging from 0 to 10;
b is an integer ranging from 0 to 8;
c is an integer ranging from 0 to 6;
R₅ is 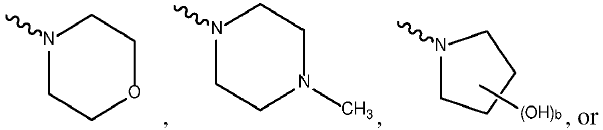 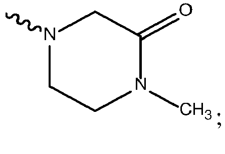 and
each R₆ and R₇ is independently -H or -I, wherein at least one of R₆ and R₇ is -I,
and wherein when R₃ is -C₁-C₃ alkyl, R₇ is -H.

Also described herein (not in accordance with the invention) are methods for treating or preventing a proteopathy, comprising administering to a subject in need thereof an effective amount of a compound of Formula IV: 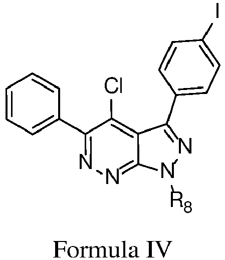 or a pharmaceutically effective salt thereof,
wherein R₈ is -C₁-C₃ alkyl.

Also described herein (not in accordance with the invention) are methods for treating or preventing a proteopathy, comprising administering to a subject in need thereof an effective amount of a compound having the structure: 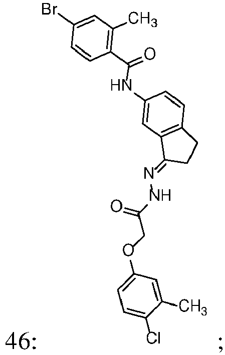 or a pharmaceutically acceptable salt thereof.

Also described herein (not in accordance with the invention) are methods for treating or preventing a proteopathy, comprising administering to a subject in need thereof an effective amount of a compound having the structure: 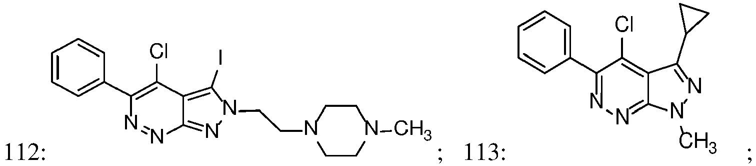 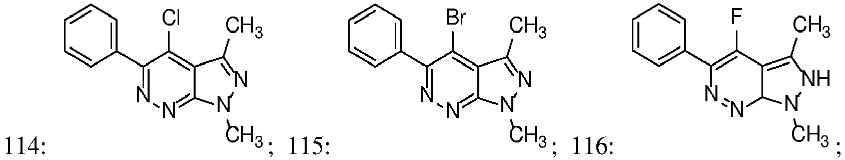 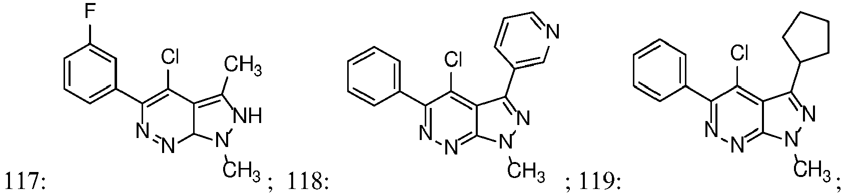 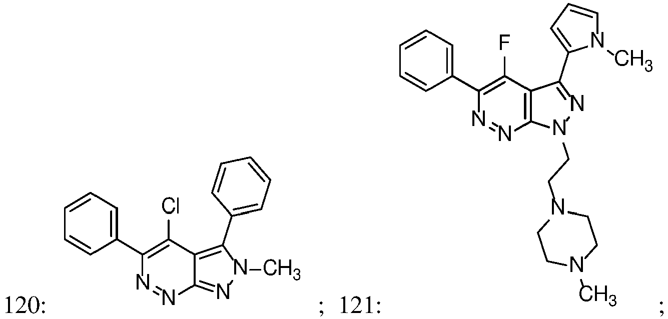 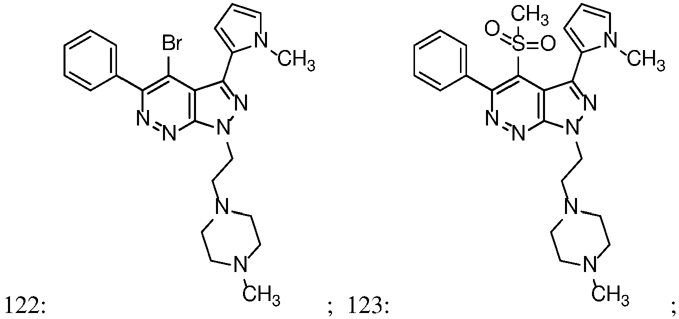 or a pharmaceutically acceptable salt thereof.

Also described herein (not in accordance with the invention) are methods for treating or preventing a proteopathy, comprising administering to a subject in need thereof an effective amount of a compound of Formula V: 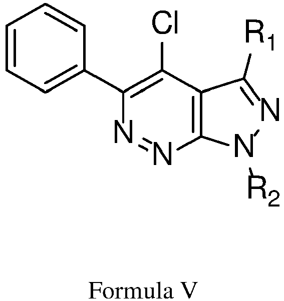 or a pharmaceutically acceptable salt thereof,
wherein R₁ is: 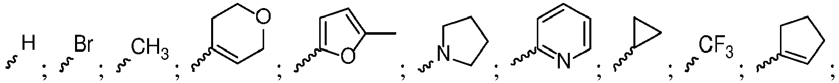 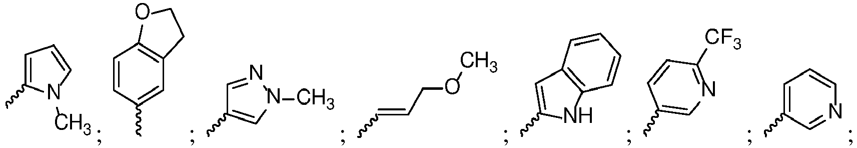 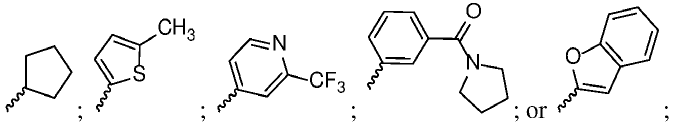
R₂ is: 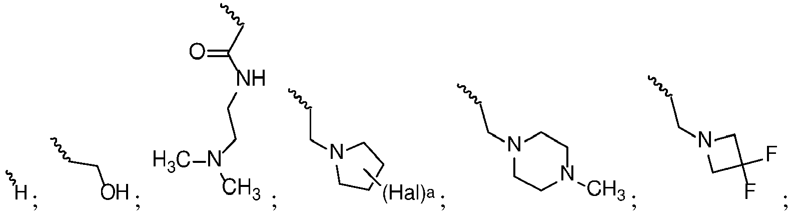 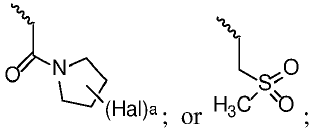
Hal is -Cl, -F, -I, or -Br; and
and a is 0, 1, or 2.

Also described herein (not in accordance with the inention) are methods for treating or preventing a proteopathy, comprising administering to a subject in need thereof an effective amount of a compound of Formula VI: 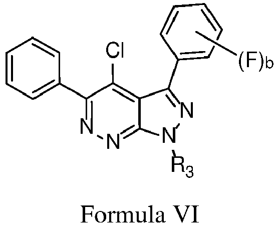 or a pharmaceutically acceptable salt thereof,
wherein R₃ is: 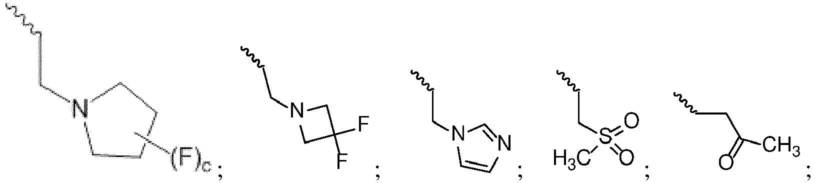 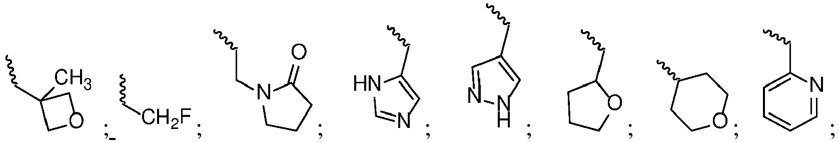 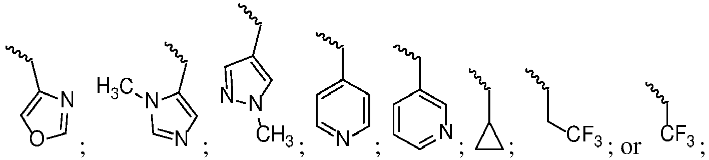
b is 0 or 1; and
c is 1 or 2.

Also described herein (not in accordance with the invention) are methods for treating or preventing a proteopathy, comprising administering to a subject in need thereof an effective amount of a compound of Formula VII: 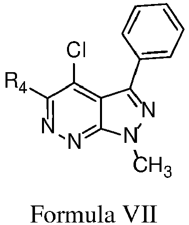 or a pharmaceutically acceptable salt thereof,
wherein R₄ is

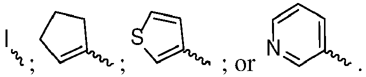

Also described herein (not in accordance with the invention) are methods for treating or preventing a proteopathy, comprising administering to a subject in need thereof an effective amount of a compound of Formula XIII: 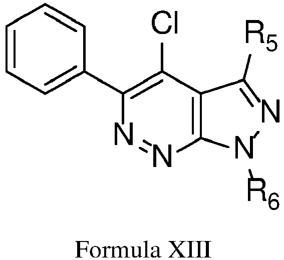 or a pharmaceutically acceptable salt thereof,
wherein R₅ is: 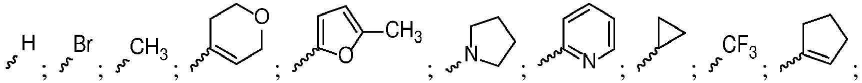 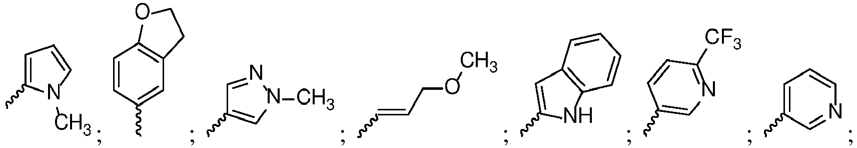 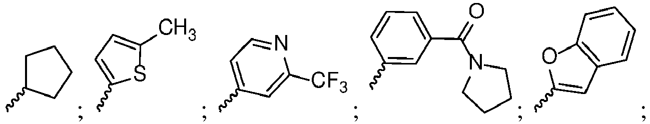 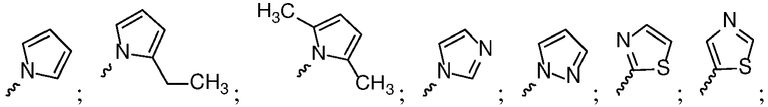 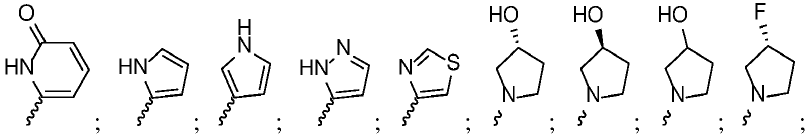 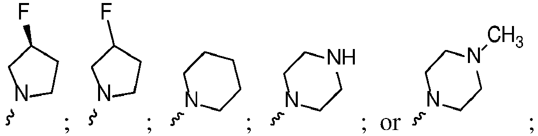
R₆ is: 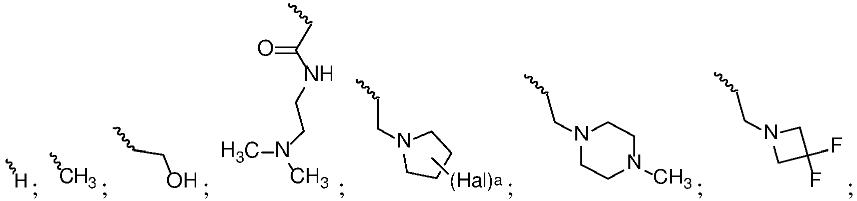 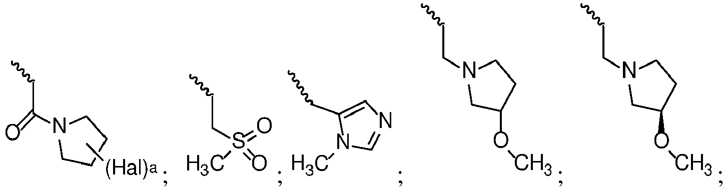 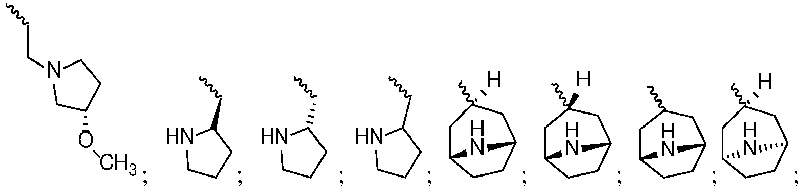 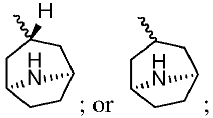
Hal is -Cl, -F, -I, or -Br; and
a is 0, 1, or 2.

Also described herein (not in accordance with the invention) are methods for treating or preventing a proteopathy, comprising administering to a subject in need thereof an effective amount of a compound of Formula XIV: 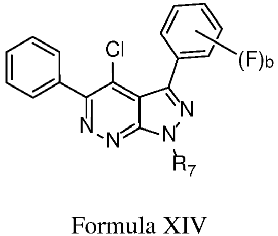 or a pharmaceutically acceptable salt thereof,
wherein R₇ is: 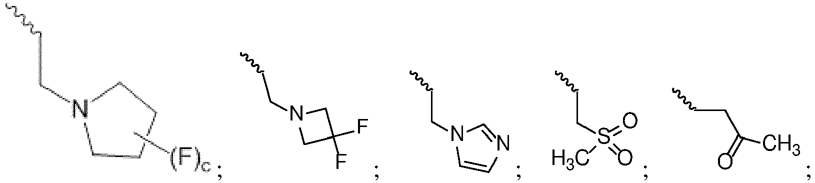 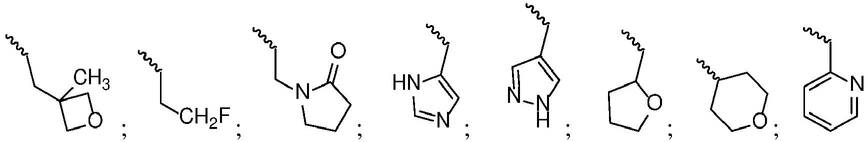 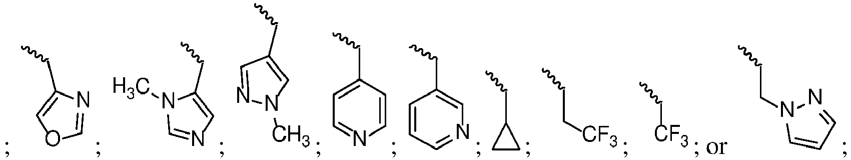
b is 0 or 1; and
c is 1 or 2.

Also described herein (not in accordance with the invention) are methods for treating or preventing a proteopathy, comprising administering to a subject in need thereof an effective amount of a compound of Formula XV: 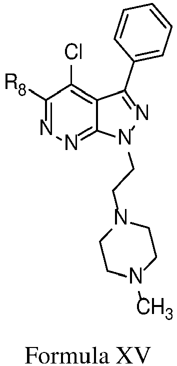 or a pharmaceutically acceptable salt thereof,
wherein R₈ is:

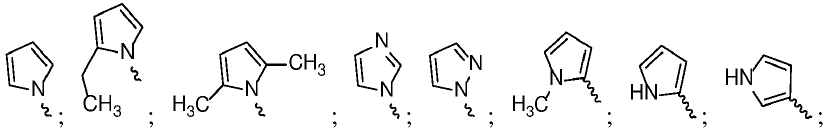

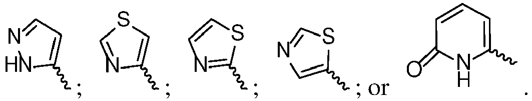

A "Pyrazolopyridazine compound" is: a compound of Formula I, II, III, IV, V, VI, VII, XIII, XIV or XV; Compound 1-35, 37-39, 42, 43, 44, 45, 46, 47-97, 98-123, 124a, 124b; or a pharmaceutically acceptable salt of any of the foregoing. A Pyrazolopyridazine compound is useful for treating or preventing a proteopathy.

### DETAILED DESCRIPTION OF THE INVENTION

The invention provides Pyrazolopyridazine compounds of Formula II, or pharmaceutically acceptable salts thereof, for use in a method for treating or preventing a proteopathy, comprising administering to a subject in need thereof an effective amount of the Pyrazolopyridazine compound.

### Definitions

The term "alkyl" refers to a straight or branched saturated hydrocarbon group. Illustrative alkyl groups include -CH3, -CH2CH3, -CH2CH2CH3, -CH(CH3)2, -CH₂CH₂CH₂CH₃, -CH(CH₃)CH₂CH₃, -CH₂CH(CH₃)₂, -C(CH₃)₃, -CH₂CH₂CH₂CH₂CH₃, -CH(CH₃)CH₂CH₂CH₃, -CH₂CH₂CH(CH₃)₂, -CH₂C(CH₃)₃, -CH₂CH₂CH₂CH₂CH₃, -CH(CH3)CH2CH2CH3, -CH2CH2CH(CH3)2 and -CH(CH3)C(CH3)3 groups.

The term "alkylene" refers to an alkyl group bonded to another atom or group. Illustrative alkylene groups include -CH2-, -CH2CH2-, -CH2CH2CH2-, -C(CH3)2-, -CH(CH3), -CH₂CH₂CH₂CH₂-, -CH(CH₃)CH₂CH₂-, -CH₂C(CH₃)₂-, -C(CH₃)₂CH₂-, -CH₂CH₂CH₂CH₂CH₂-, -CH(CH₃)CH₂CH₂CH₂-, -CH₂CH₂C(CH₃)₂-, -CH₂CH(CH₃)CH₂CH₂, -CH₂CH₂CH(CH₃)CH₂-, -CH₂CH₂CH₂CH₂CH₂CH₂-, -CH(CH₃)CH₂CH₂CH₂CH₂-, -CH₂CH₂CH₂C(CH₃)₂-, -CH₂CH(CH₃)CH₂CH₂CH₂-, -CH₂CH₂CH₂CH(CH₃)CH₂- and -C(CH3)2C(CH3)2 - groups.

The term "alkenyl" refers to a straight or branched hydrocarbon group having one or more double bonds. Illustrative alkenyl groups include -CH=CH2, -CH2CH=CH2, *cis* -CH=CHCH₃, *trans* -CH=CHCH₃, -C(CH₃)=CH₂, *cis* -CH=CHCH₂CH₃, *trans* -CH=CHCH₂CH₃, *cis* -CH₂CH=CHCH₃, *trans* -CH₂CH=CHCH₃, -CH₂CH₂CH=CH₂, *cis* -CH=CHCH₂CH₂CH₃, *trans* -CH=CHCH₂CH₂CH₃, *cis* -CH₂CH₂CH=CHCH₃, *trans* -CH₂CH₂CH=CHCH₃, -CH₂CH₂CH₂CH=CH₂, -CH₂CH=C(CH₃)₂, *cis* -CH=CHCH₂CH₂CH₂CH₃, *trans* -CH=CHCH₂CH₂CH₂CH₃, *cis* -CH₂CH₂CH₂CH=CHCH₃, *trans* -CH₂CH₂CH₂CH=CHCH₃, -CH₂CH₂CH₂CH₂CH=CH₂, and -CH2CH2CH=C(CH3)2, groups.

The word "about" when immediately preceding a numerical value means a range of plus or minus 10% of that value, e.g., "about 100 mg" means 90 mg to 110 mg, "about 300 mg" means 270 mg to 330 mg.

### Abbreviations:

- APCI: Atmospheric Pressure Chemical Ionization
- DAPI: 4',6-diamidino-2-phenylindole
- DCM: dichloromethane
- DEAD: diethyl azodicarboxylate
- DIPEA: diisopropylethylamine
- DMEM: Dulbecco's Modified Eagle Medium
- DMF: dimethylformamide
- DMSO: Dimethyl sulfoxide
- EDAC: 1-(3-dimethylaminopropyl)-3-ethylcarbodiimide hydrochloride
- ESI: Electrospray ionization
- ESI-TOF: Electrospray ionization-Time-of-flight
- HATU: 2-(7-Aza-1H-benzotriazole-1-yl)-1,1,3,3-tetramethyluronium hexafluorophosphate
- HOPO: 2-hydroxypyridine-N-oxide
- HPLC: High-performance liquid chromatography
- LCMS: Liquid chromatography-mass spectrometry
- LDA: lithium diisopropyl amide
- m/z: Mass-to-charge ratio
- MALDI-TOF: Matrix Assisted Laser Desorption Ionization-Time-of-flight
- MS: Mass spectrometry
- PBS: phosphate-buffered saline
- Rt: Retention time
- SDS: sodium dodecylsulfate
- TFA: trifluoroacetic acid
- THF: tetrahydrofuran

The term "effective amount" means an amount of a Pyrazolopyridazine compound or non-Pyrazolopyridazine compound that is effective to treat or prevent a proteopathy in a subject. In some embodiments, where another therapeutic or prophylactic agent is administered prior to, subsequent to or concurrently with administration of a Pyrazolopyridizne compound or non-Pyrazolopyridazine compound, the "effective amount" is the total amount of (i) Pyrazolopyridazine compound or non-Pyrazolopyridazine compound and (ii) the other therapeutic or prophylactic agent that is effective to treat or prevent a protopathy in a subject.

The terms "proteopathy", "proteinopathy" and "protein conformational disorder" refer to a disease or a disorder resulting from the misfolding of one or more proteins.

The term "protein aggregate" refers to a biological phenomenon in which misfolded proteins accumulate and clump together.

A "subject" is a mammal, including a species-rich order, e.g., a primate, such as a human; a Rodentia species, such as a mouse, a rat or a guinea pig; a Carnivora species such as a dog, cat, weasel, bear or seal; a non-human primate, such as a monkey, chimpanzee, baboon or rhesus; a Chiroptera species, such as a bat; a Soricomorpha species, such as a shrew, mole or solenodon; and a Cetartiodactyla species, such as a whale. In one embodiment, the subject is a human. In another embodiment, the human is a human fetus.

### Pyrazolopyridazine Compounds of Formula II

The invention provides a compound of Formula II for use in a method for treating or preventing a proteopathy, comprising administering to a subject in need thereof an effective amount of the compound of Formula II: 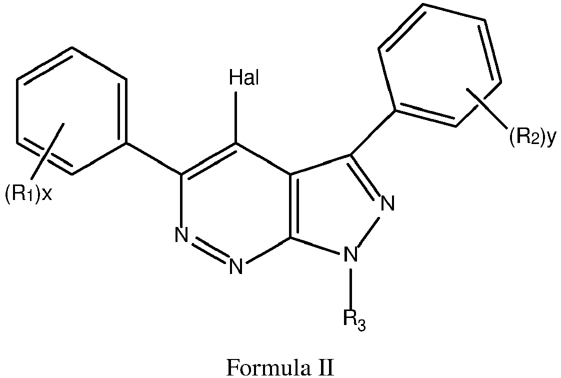 or a pharmaceutically acceptable salt thereof,
wherein Hal is -Cl, -F, -I, or -Br;
x is an integer ranging from 0 to 5;
each R₁ is independently -Cl, -F, -I, -Br, -C₁-C₃ alkyl, -O- C₁-C₃ alkyl, -CN, -CF₃, -C(O)NH(CH₃), or -C≡CCH₂OH;
y is an integer ranging from 0 to 5;
each R₂ is independently -Cl, -F, -Br, -C₁-C₃ alkyl, -O- C₁-C₃ alkyl, -CN, -CF₃, -C(O)NH(CH₃), or -C≡CCH₂OH;
R₃ is -H, -C₁-C₆ alkyl, -(C₁-C₆ alkylene)-OH, -(C₁-C₆ alkylene)-phenyl, -(C₁-C₆ alkylene)-O-(C₁-C₆ alkyl), -C₂-C₆ alkenyl, -(C₁-C₆ alkylene)-C(O)R₄,
-(C₁-C₆ alkylene)-R₅, 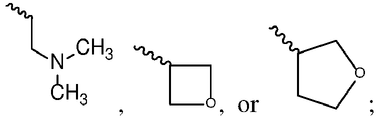
R₄ is -OH, -O-(C₁-C₆ alkyl), -NH₂, -NH(C₁-C₆ alkyl), -NH((C₁-C₆ alkylene)-OH), -NH((C₁-C₆ alkylene)N(C₁-C₆ alkyl)₂), -N(C₁-C₆ alkyl)((C₁-C₆ alkylene)-CN), -N(C₁-C₆ alkyl)((C₁-C₆ alkylene)N(C₁-C₆ alkyl)₂), -NH(C₁-C₆ alkylene)-O-(C₁-C₆ alkyl), 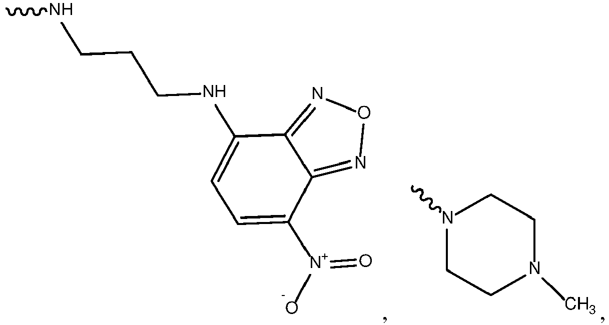 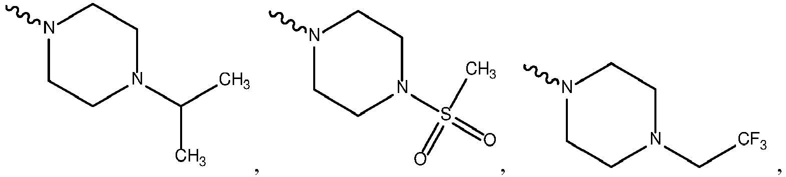 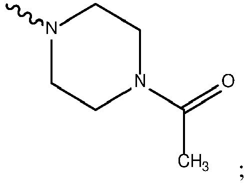 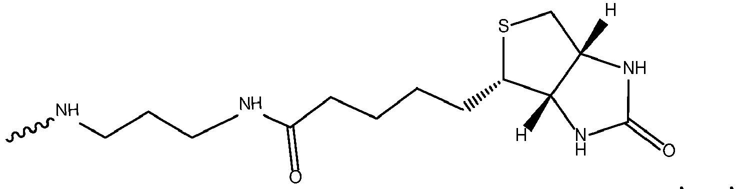 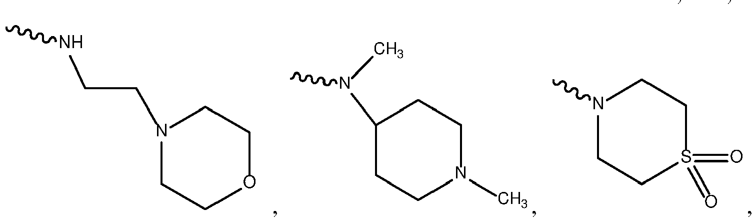 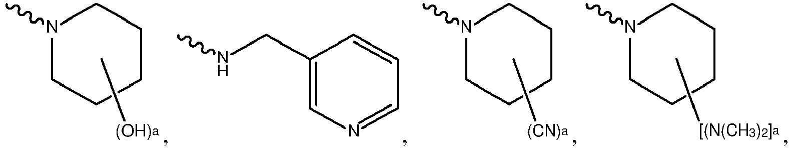 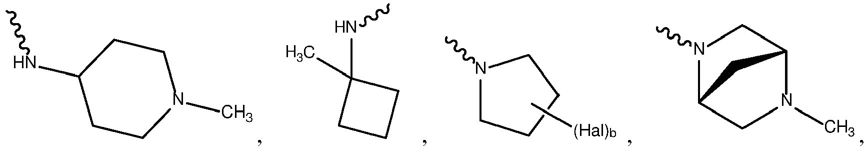 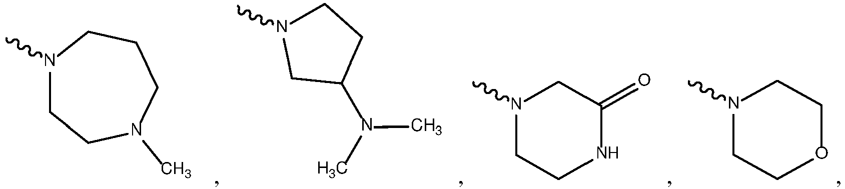 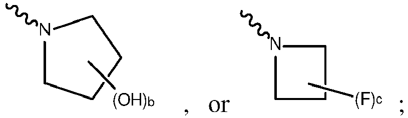
a is an integer ranging from 0 to 10 ;
b is an integer ranging from 0 to 8 ;
c is an integer ranging from 0 to 6; and
R₅ is 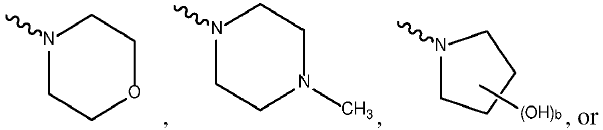 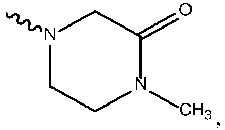
wherein the proteopathy is not retinitis pigmentosa, Leber's congenital amaurosis, a syndromic retinal degeneration, age-related macular degeneration, Usher syndrome or a retinal degenerative disease.

In certain embodiments, Hal is -Cl. In yet another embodiment, x and y are 0.

In certain embodiments, x and y are 0, x is 0 and y is 1, x is 1 and y is 2, x is 1 and y is 0, x is 1 and y is 1, x is 1 and y is 2, x is 2 and y is 0, x is 2 and y is 1, or x is 2 and y is 2.

In certain embodiments, Hal is -Cl and: x and y are 0, x is 0 and y is 1, x is 1 and y is 2, x is 1 and y is 0, x is 1 and y is 1, x is 1 and y is 2, x is 2 and y is 0, x is 2 and y is 1, or x is 2 and y is 2.

In particular embodiments, x is 1 and R₁ is in the ortho position relative to the pyrazolopyridazino ring system. In certain embodiments, x is 1 and R₁ is in the para position relative pyrazolopyridazino ring system. In further embodiments, x is 1 and R₁ is in the meta position relative pyrazolopyridazino ring system.

In particular embodiments, y is 1 and R2 is in the ortho position relative pyrazolopyridazino ring system. In certain embodiments, y is 1 and R₂ is in the para position relative pyrazolopyridazino ring system. In further embodiments, y is 1 and R₂ is in the meta position relative pyrazolopyridazino ring system.

In particular embodiments, x is 2 and R₁ is in the ortho and meta position relative pyrazolopyridazino ring system. In certain embodiments, x is 2 and R₁ is in the ortho and para position relative pyrazolopyridazino ring system. In further embodiments, x is 2 and R₁ is in the para and meta position relative pyrazolopyridazino ring system.

In particular embodiments, y is 2 and R₂ is in the ortho and meta position relative pyrazolopyridazino ring system. In certain embodiments, y is 2 and R₂ is in the ortho and para position relative pyrazolopyridazino ring system. In further embodiments, y is 2 and R₂ is in the para and meta position relative pyrazolopyridazino ring system.

In yet other embodiments, R₁ is chloro. In certain embodiments, R₁ is fluoro. In certain embodiments, R₁ is iodo. In other embodiments, R₁ is -Br. In further embodiments, R₁ is -OCH₃. In other embodiments, R₁ is -CH₃. In yet other embodiments, R₁ is -C(O)N(H)CH₃. In certain embodiments, R₁ is -CF₃. In further embodiments, R₁ is -CN. In additional embodiments, R₁ is -C≡CCH₂OH.

In yet other embodiments, x is 1 or 2, and R₁ is -Cl, -F, -I, -Br, -OCH₃, -CH3, -C(O)N(H)CH₃, -CF₃, -CN or -C≡CCH₂OH.

In yet other embodiments, Hal is -Cl, x is 1 or 2, and R₁ is -Cl, -F, -I, -Br, -OCH₃, -CH₃, -C(O)N(H)CH₃, -CF₃, -CN or -C≡CCH₂OH.

In yet other embodiments, R₂ is -Cl. In certain embodiments, R₂ is -F. In other embodiments, R₂ is -Br. In further embodiments, R₂ is -OCH₃. In other embodiments, R₂ is -CH₃. In yet other embodiments, R₂ is -C(O)N(H)CH₃. In certain embodiments, R₂ is -CF₃. In further embodiments, R₂ is -CN. In additional embodiments, R₂ is -C≡CCH₂OH.

In yet other embodiments, y is 1 or 2, and R₂ is -Cl, -F, -Br, -OCH₃, -CH₃, -C(O)N(H)CH₃, -CF₃, -CN or -C≡CCH₂OH.

In yet other embodiments, Hal is -Cl, y is 1 or 2, and R₂ is -Cl, -F, -Br, -OCH₃, -CH₃, -C(O)N(H)CH₃, -CF₃, -CN or -C≡CCH₂OH.

In particular embodiments, R₃ is -H. In certain embodiments, R₃ is -CH3. In further embodiments, R₃ is -CH₂CH₃. In still further embodiments, R₃ is -CHCH₂. In other embodiments, R₃ is -CH₂CH₂OH. In particular embodiments, R₃ is -(CH₂)₂C₆H₅. In other embodiments, R₃ is -CH₂C(O)OH. In yet other embodiments, R₃ is -CH₂C(O)N(H)CH₃. In certain embodiments, R₃ is -CH₂C(O)N(H)((CH₂)₂N(CH₃)₂).
In yet other embodiments, R₃ is -CH₂C(O)N(H)((CH₂)₃N(CH₃)₂). In other embodiments, R₃ is -CH₂C(O)N(CH₃)CH₂CN. In particular embodiments, R₃ is -CH₂C(O)NH₂. In certain embodiments, R₃ is -CH₂C(O)N(H)((CH₂)₂OH). In other embodiments, R₃ is -CH₂C(O)N(H)((CH₂)₂OCH₃). In still further embodiments, R₃ is -CH₂C(CH₃)₂OH. In yet other embodiments, R₃ is -CH₂C(O)OCH₃. In further embodiments, R₃ is -CH2CH(OH)CH3. In still further embodiments, R₃ is -CH₂CH₂OH. In particular embodiments,
R₃ is -CH(CH₃)CH₂OH.

In further embodiments, R₃ is -CH₂C(O)R₄ and R₄ is 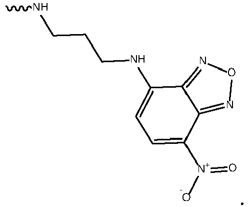 In other embodiments, R₃ is -CH₂C(O)R₄ and R₄ is 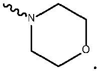 In particular embodiments, R₃ is -CH₂C(O)R₄ and R₄ is 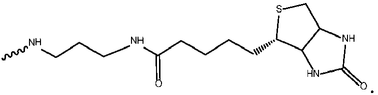 In yet other embodiments, R₃ is -CH₂C(O)R₄ and R₄ is 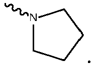 In certain embodiments, R₃ is -CH₂C(O)R₄ and R₄ is 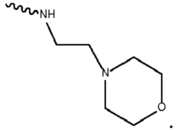 In other embodiments, R₃ is -CH₂C(O)R₄ and R₄ is 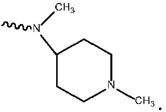 In particular embodiments, R₃ is -CH₂C(O)R₄ and R₄ is 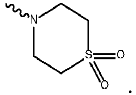 In yet other embodiments, R₃ is -CH₂C(O)R₄ and R₄ is 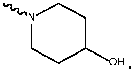 In certain embodiments, R₃ is -CH₂C(O)R₄ and R₄ is 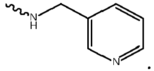 In other embodiments, R₃ is -CH₂C(O)R₄ and R₄ is 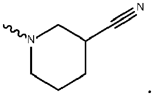 In particular embodiments, R₃ is -CH₂C(O)R₄ and R₄ is 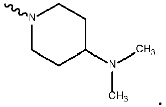 In yet other embodiments, R₃ is -CH₂C(O)R₄ and R₄ is 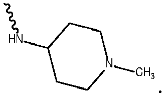 In certain embodiments, R₃ is -CH₂C(O)R₄ and R₄ is 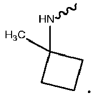 In other embodiments, R₃ is -CH₂C(O)R₄ and R₄ is 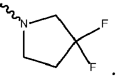 In particular embodiments, R₃ is -CH₂C(O)R₄ and R₄ is 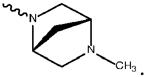 In yet other embodiments, R₃ is - CH₂C(O)R₄ and R₄ is 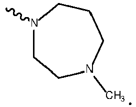 In certain embodiments, R₃ is -CH₂C(O)R₄ and R₄ is 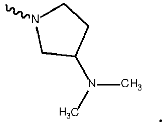 In other embodiments, R₃ is -CH₂C(O)R₄ and R₄ is 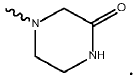 In particular embodiments, R₃ is -CH₂C(O)R₄ and R₄ is 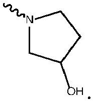 In yet other embodiments, R₃ is -CH₂C(O)R₄ and R₄ is 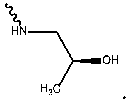 In certain embodiments, R₃ is-CH₂C(O)R₄ and R₄ is 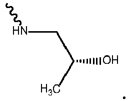 In other embodiments, R₃ is -CH₂C(O)R₄ and R₄ is 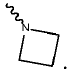 In particular embodiments, R₃ is -CH₂C(O)R₄ and R₄ is 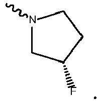 In yet other embodiments, R₃ is -CH₂C(O)R₄ and R₄ is 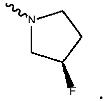 In certain embodiments, R₃ is -CH₂C(O)R₄ and R₄ is 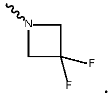 In other embodiments, R₃ is -CH₂C(O)R₄ and R₄ is 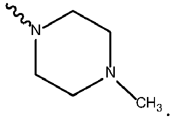 In further embodiments of the invention, R₃ is -CH₂C(O)R₄ and R₄ is 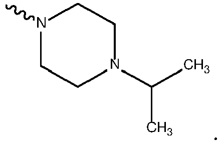 In certain embodiments of the invention, R₃ is -CH₂C(O)R₄ and R₄ is 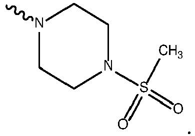 In other embodiments, R₃ is -CH₂C(O)R₄ and R₄ is 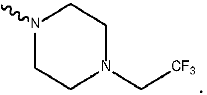 In further embodiments, R₃ is -CH₂C(O)R₄ and R₄ is 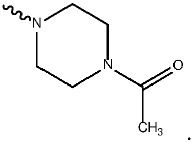 In further embodiments, R₃ is -CH₂C(O)R₄ and R₄ is

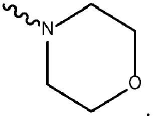

In particular embodiments, R₃ is -(CH₂)₂R₅ and R₅ is 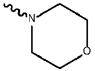 In yet other embodiments, R₃ is -(CH₂)₂R₅ and R₅ is 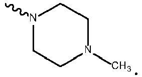 In certain embodiments, R₃ is - (CH₂)₂R₅ and R₅ is 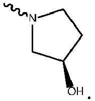 In other embodiments, invention, R₃ is -(CH₂)₂R₅ and R₅ is

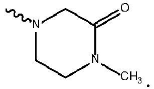

In some embodiments, a is an integer ranging from 0 to 5. In some embodiments, b is an integer ranging from 0 to 4. In some embodiments, c is an integer ranging from 0 to 6.

### Illustrative Pyrazolopyridazine Compounds of Formula II

In certain embodiments the Pyrazolopyridazine compound of Formula II has the structure: 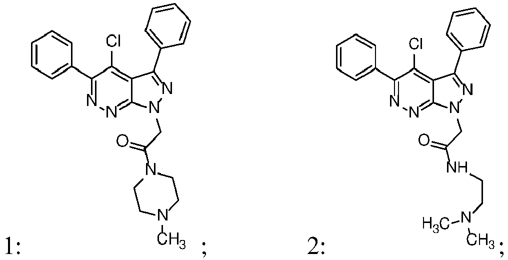 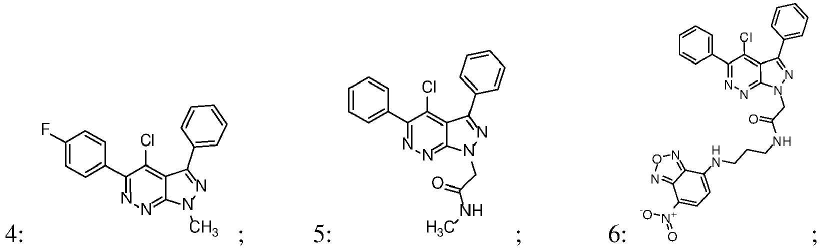 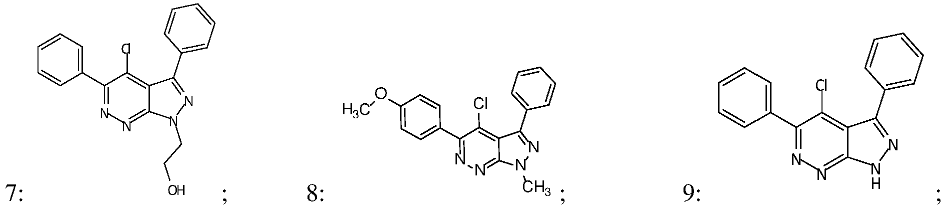 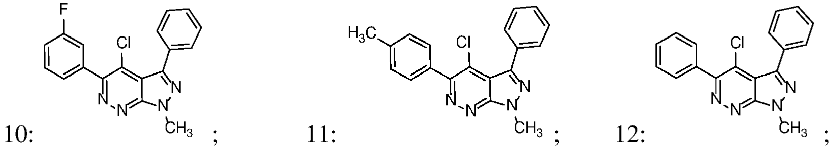 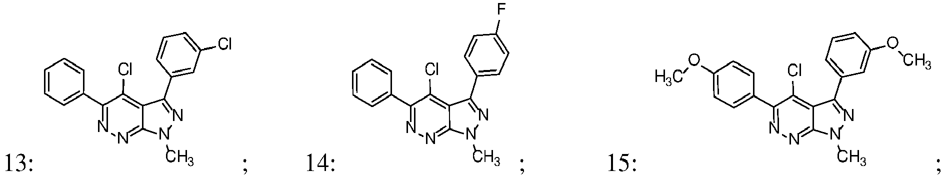 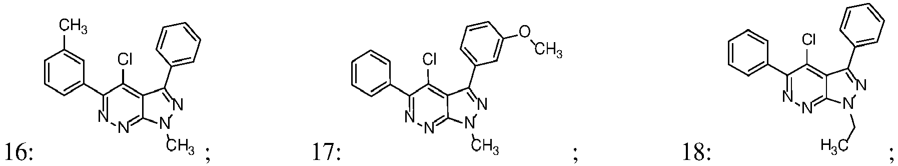 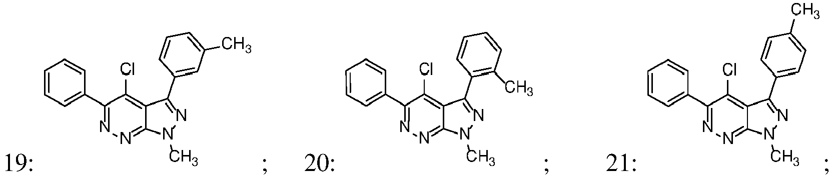 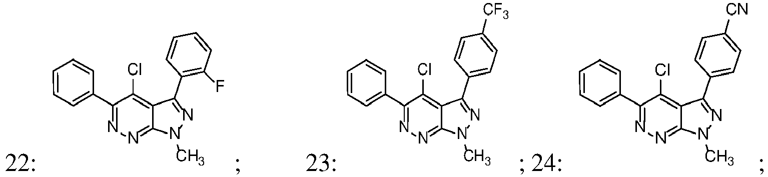 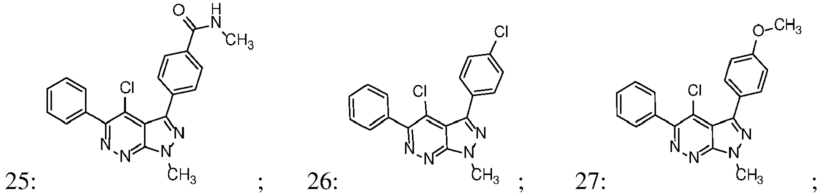 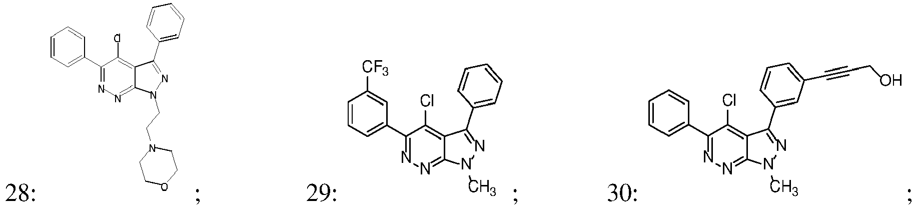 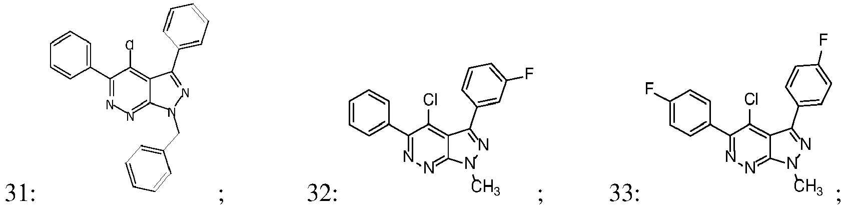 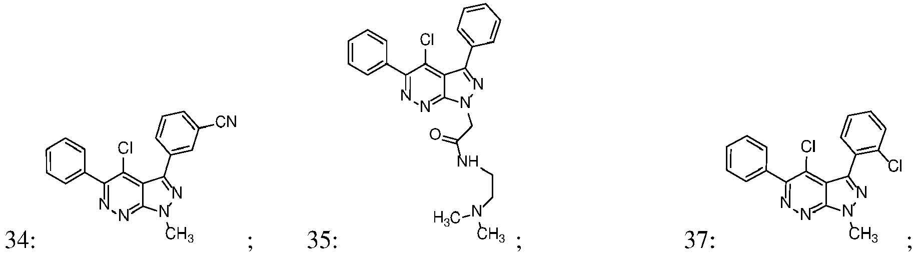 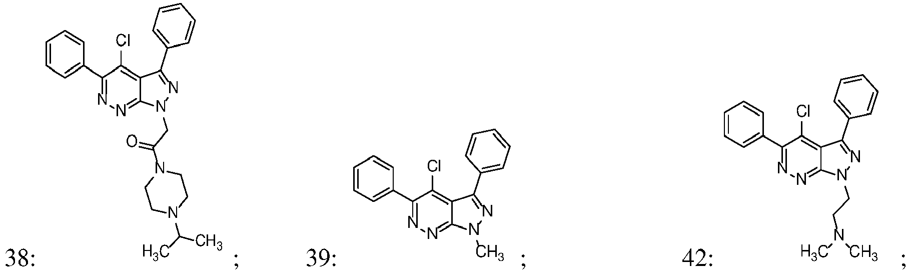 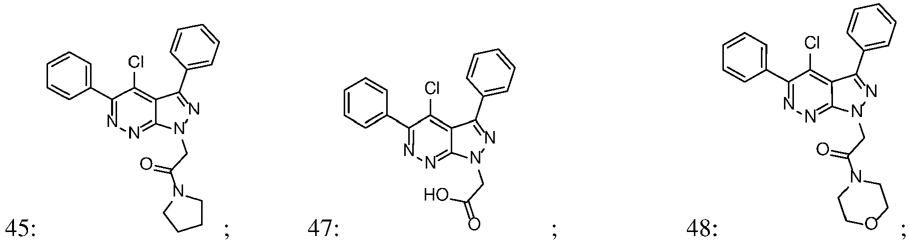 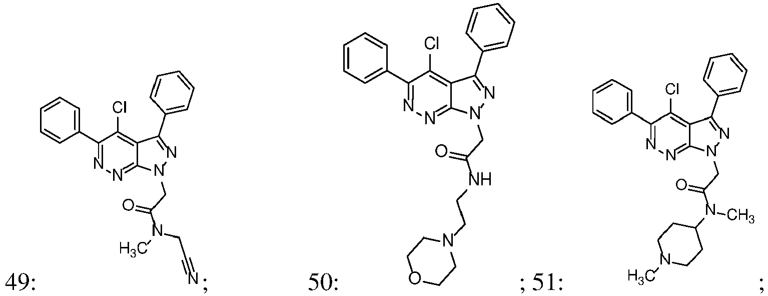 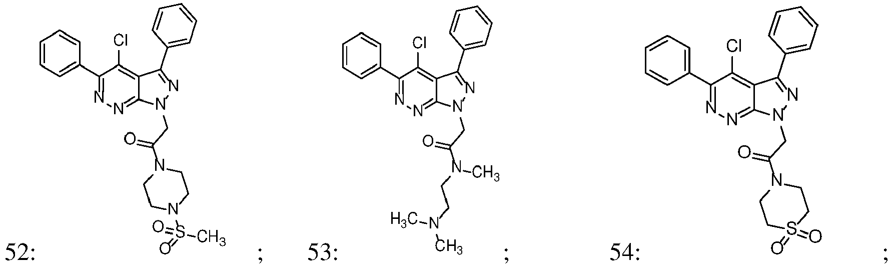 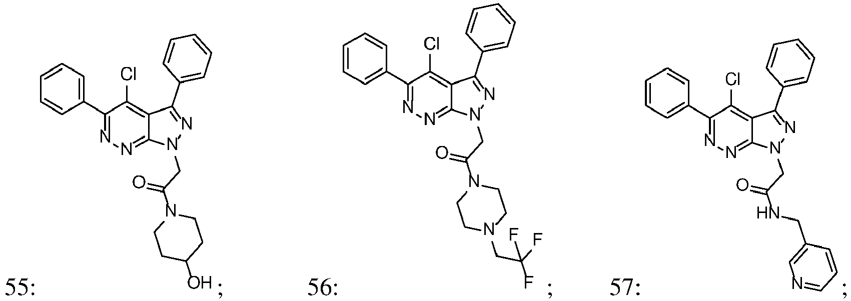 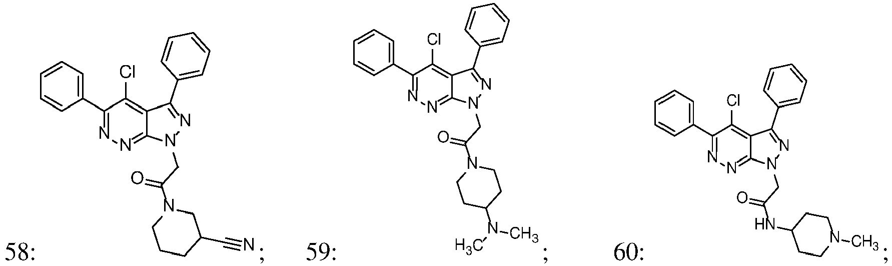 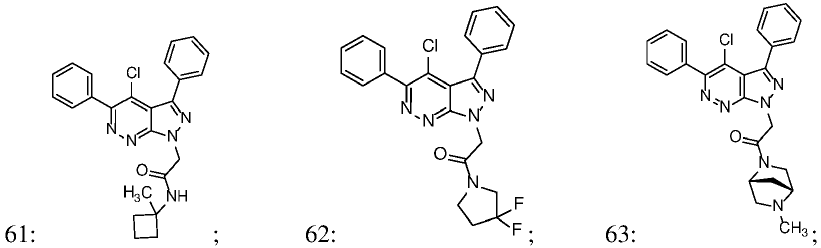 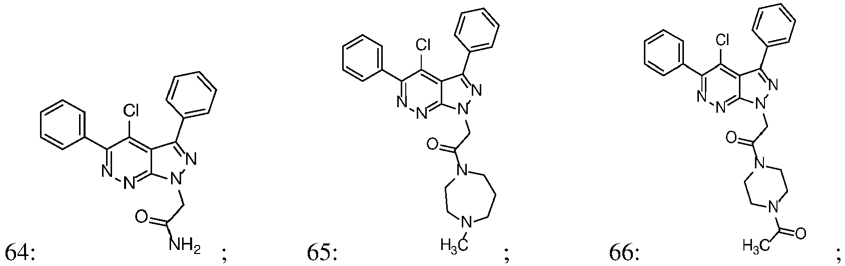 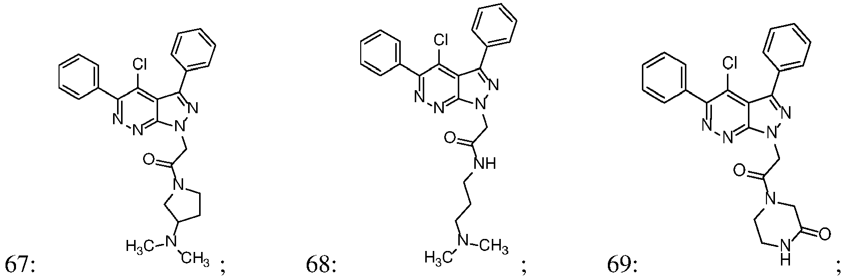 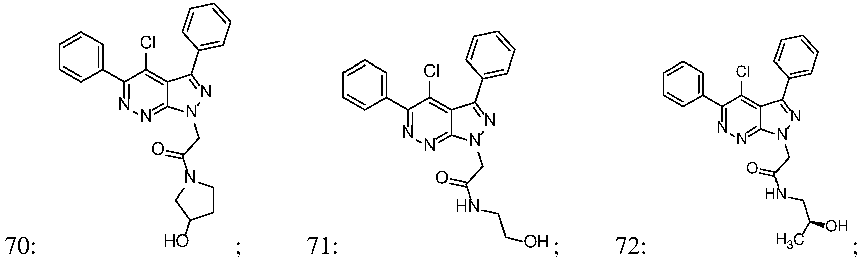 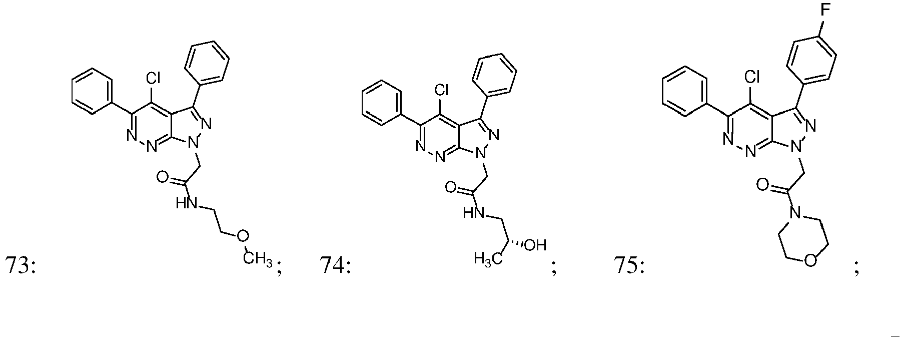 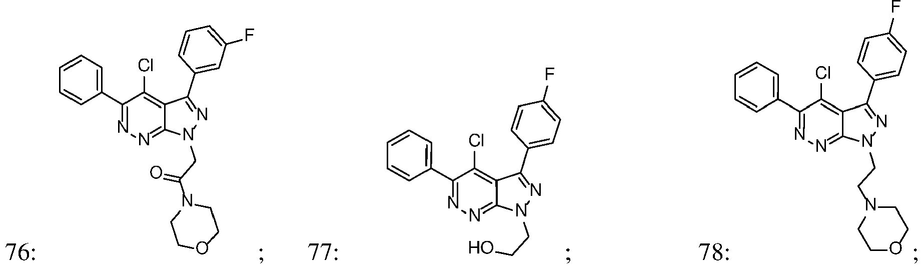 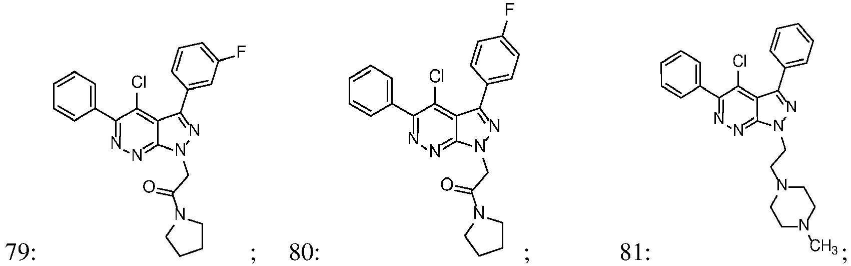 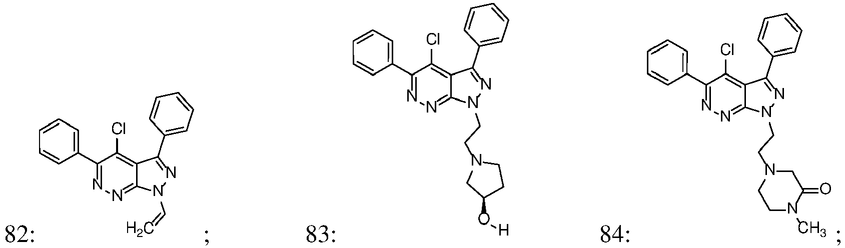 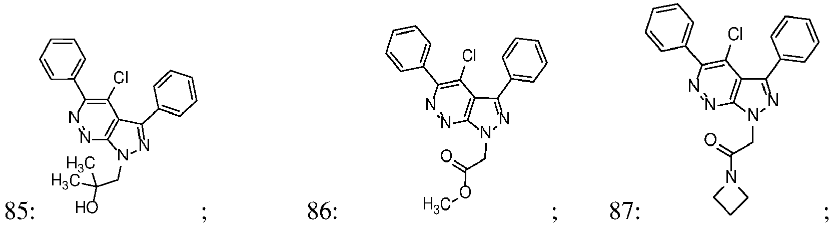 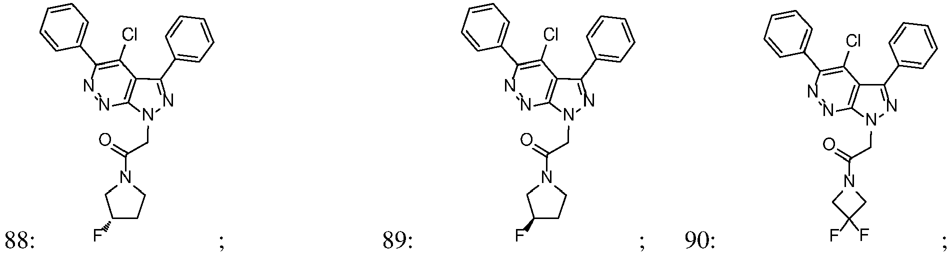 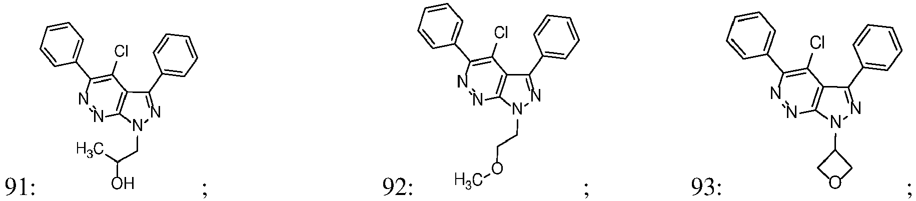 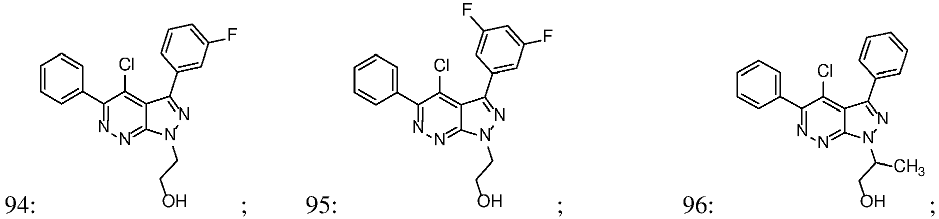 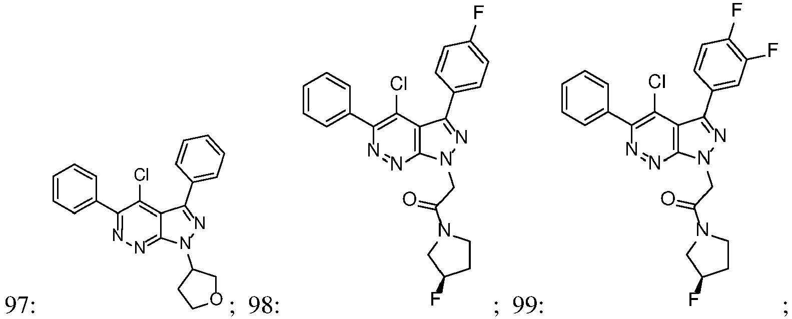 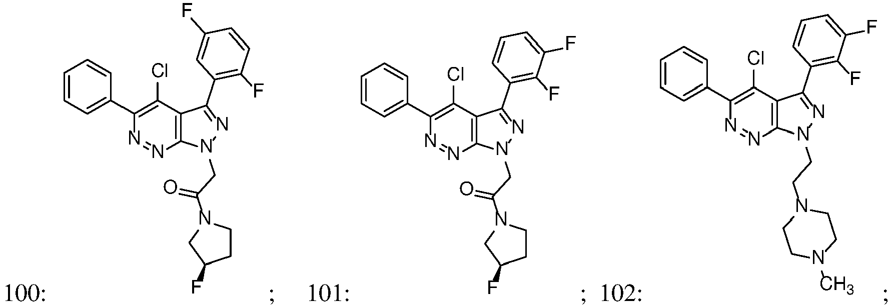 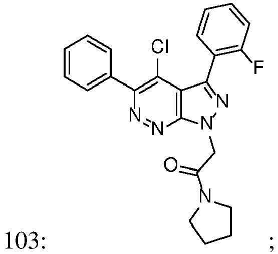 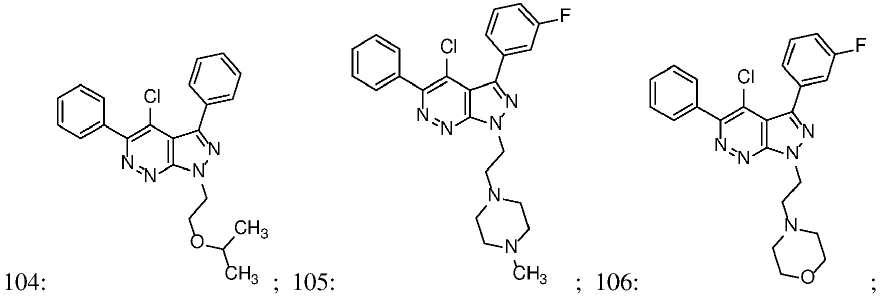 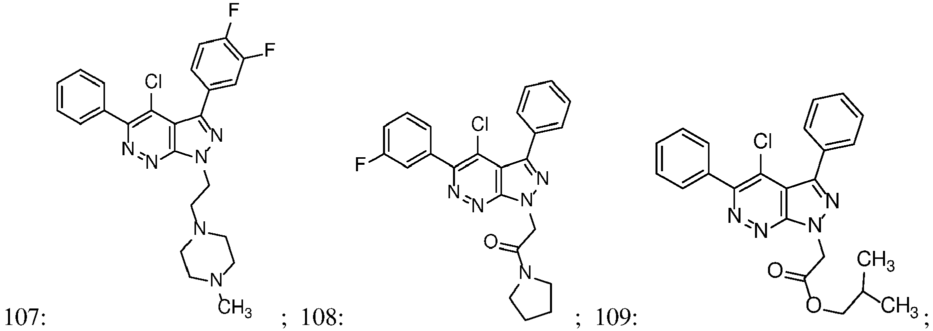 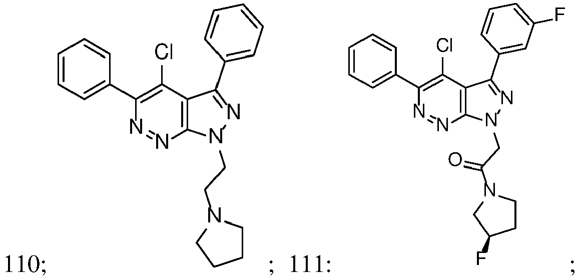 or a pharmaceutically acceptable salt thereof.

### Pyrazolopyridazine Compounds of Formula III (not in accordance with the invention)

An example (not in accordance with the invention) of a compound of Formula III is Compound 3, which has the structure: 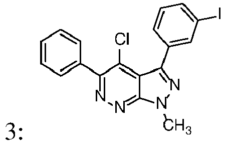 or a pharmaceutically acceptable salt thereof.

### Pyrazolopyridazine Compounds of Formula IV (not in accordance with the invention)

An example (not in accordance with the invention) of a compound of Formula IV is Compound 43, which has the structure: 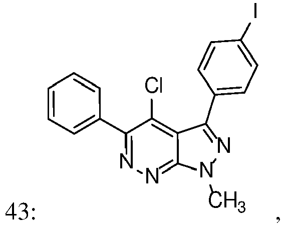 or a pharmaceutically acceptable salt thereof.

### Pyrazolopyridazine Compounds of Formula V (not in accordance with the invention)

Examples of compounds of Formula V (not in accordance with the present invention) are compounds having the structure: 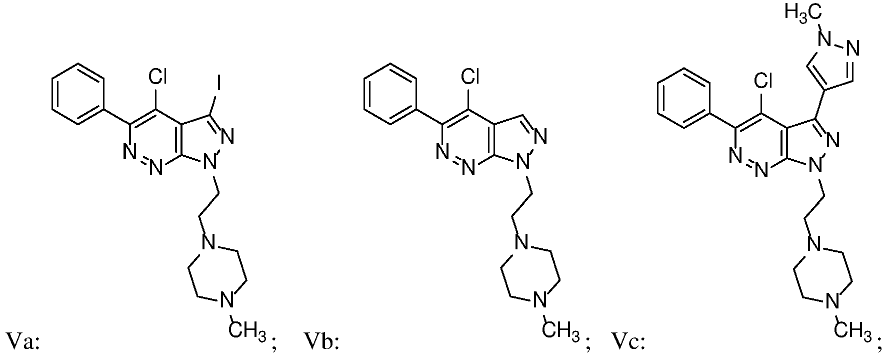 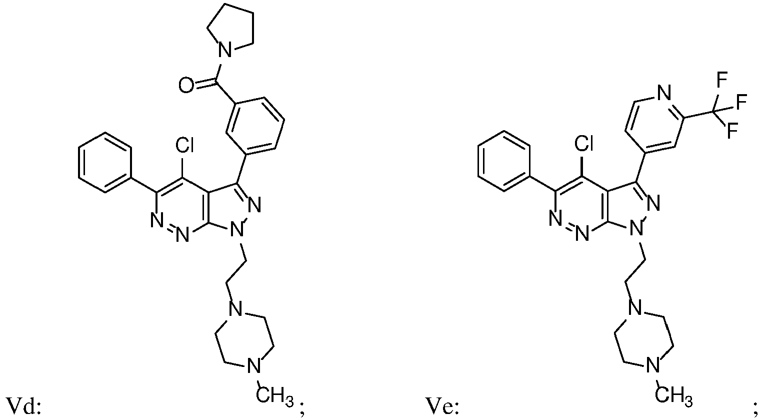 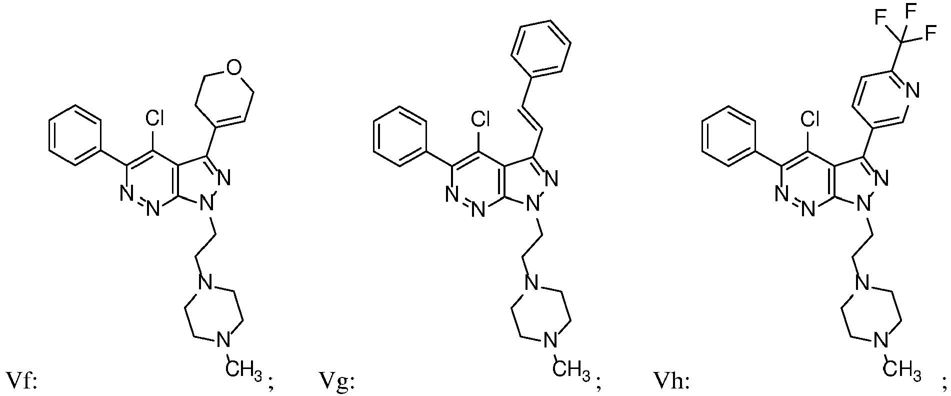 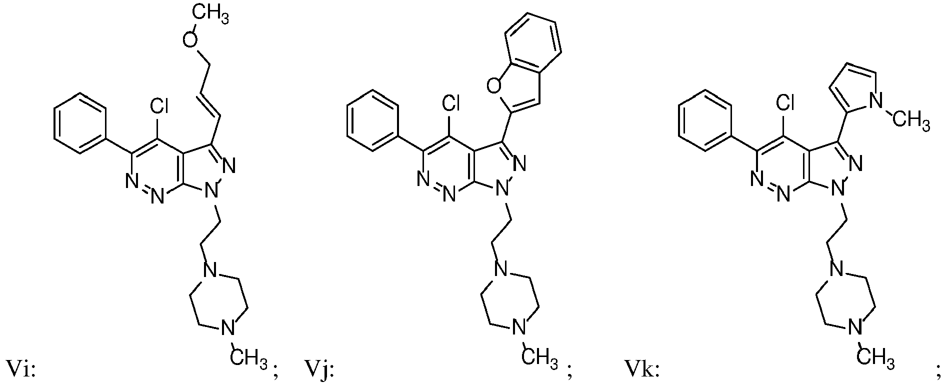 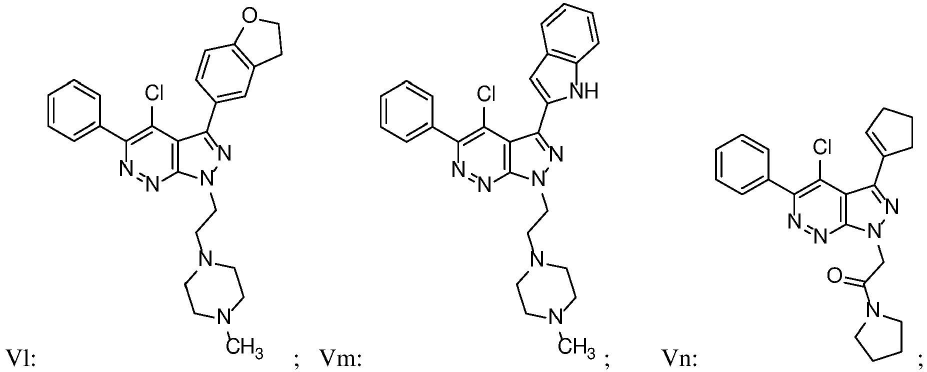 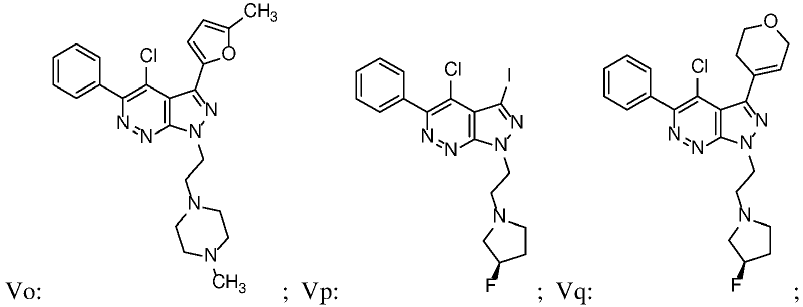 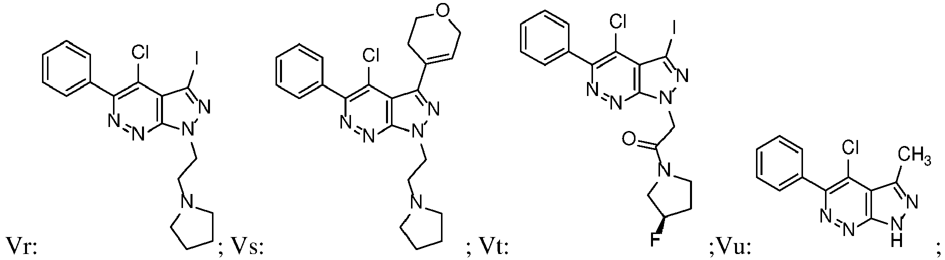 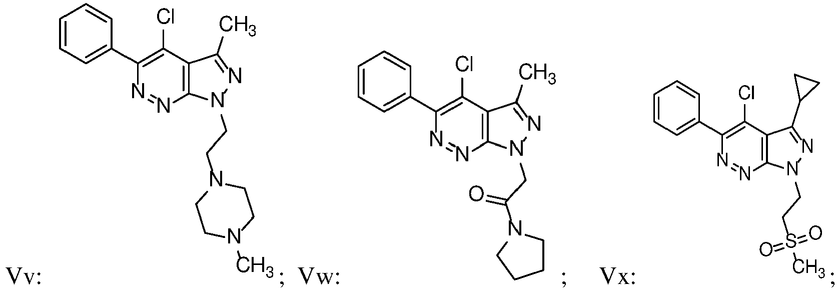 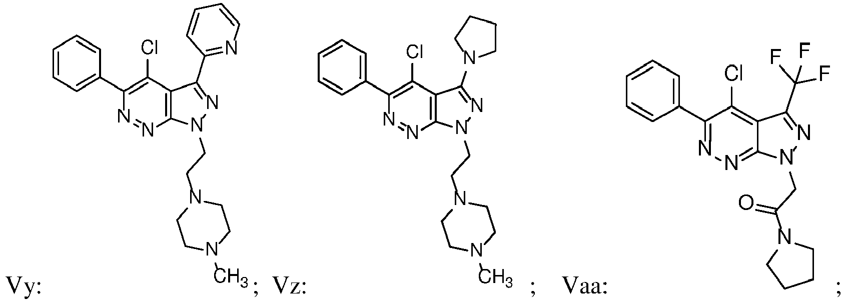 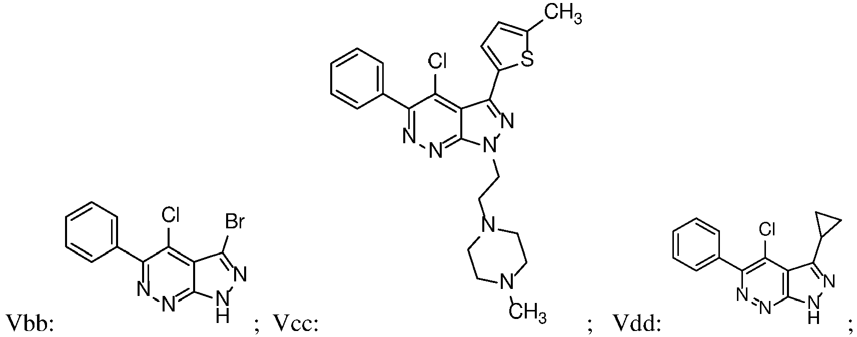 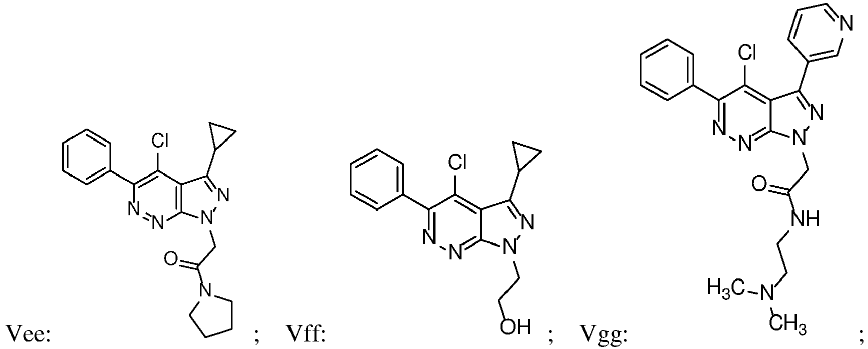 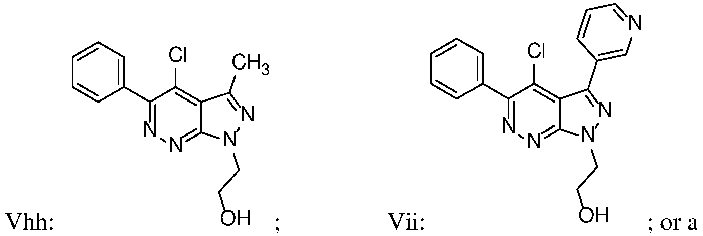 pharmaceutically acceptable salt thereof.

### Pyrazolopyridazine Compounds of Formula VI (not in accordance with the invention)

Examples of compounds of Formula VI (not in accordance with the present invention) are compounds having the structure: 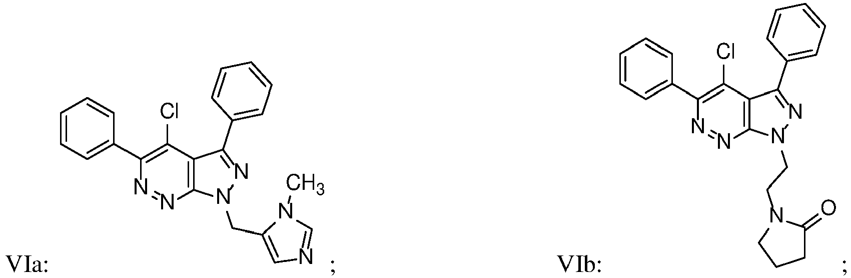 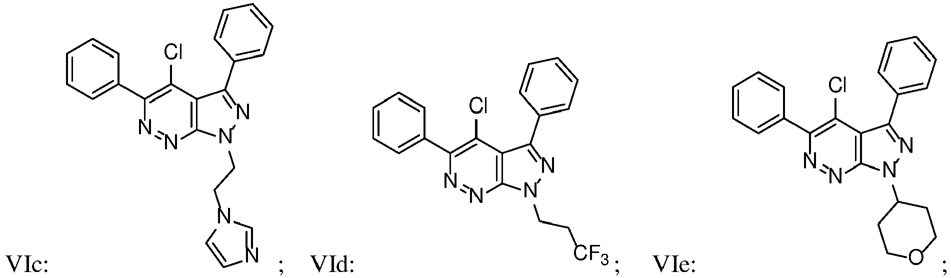 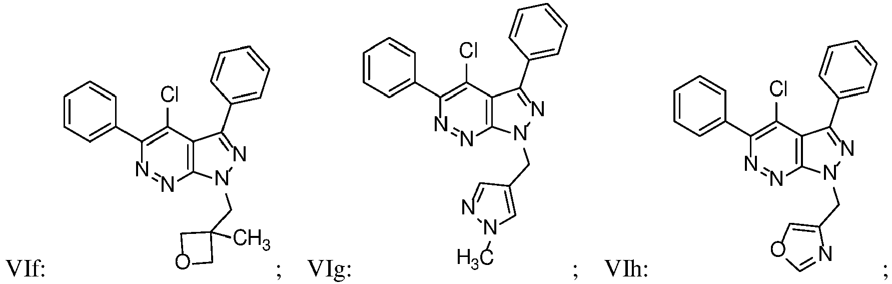 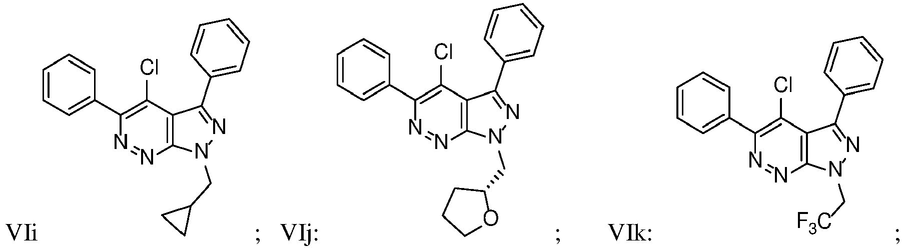 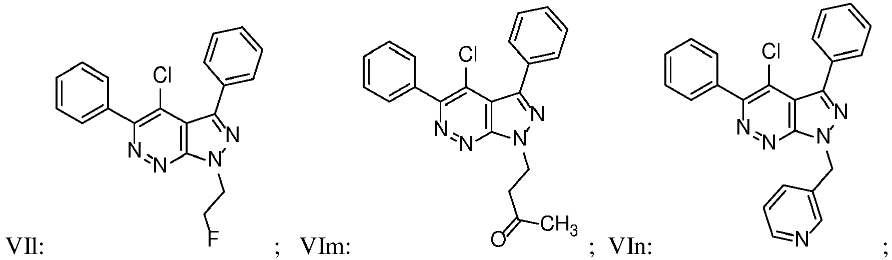 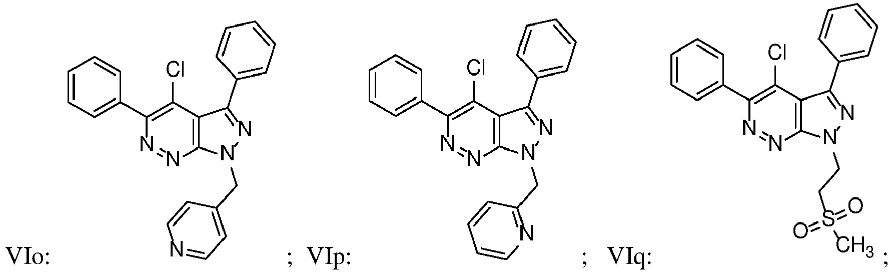 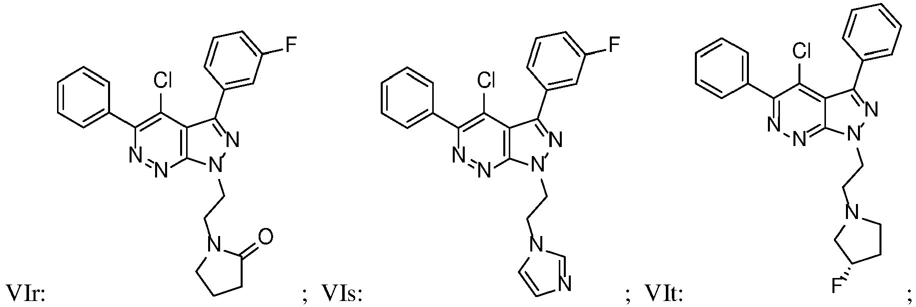 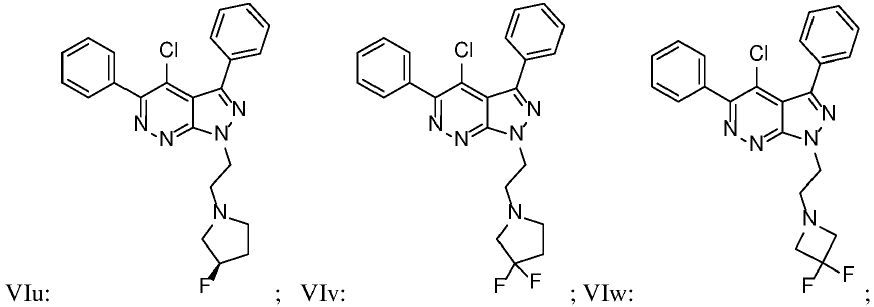 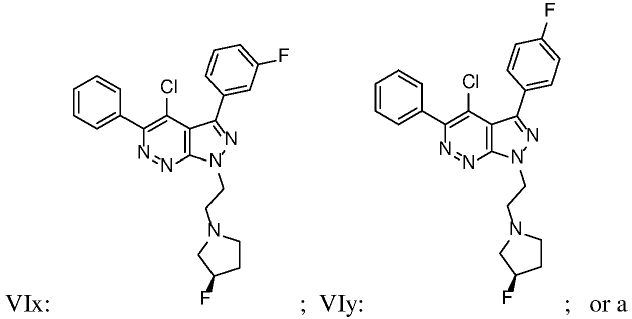 pharmaceutically acceptable salt thereof.

### Pyrazolopyridazine Compounds of Formula VII (not in accordance with the invention)

Examples of compounds of Formula VII (not in accordance with the present invention) are compounds having the structure: 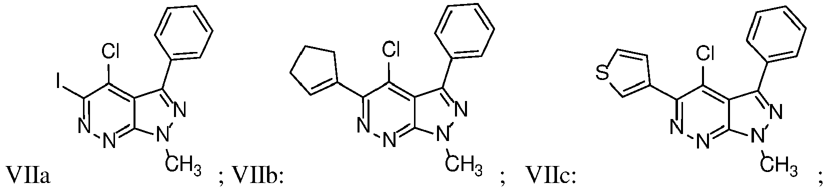 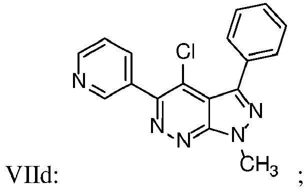 or a pharmaceutically acceptable salt thereof.

### Pyrazolopyridazine Compounds of Formula XIII (not in accordance with the invention)

Examples of compounds of Formula XIII (not in accordance with the present invention) are compounds having the structure: 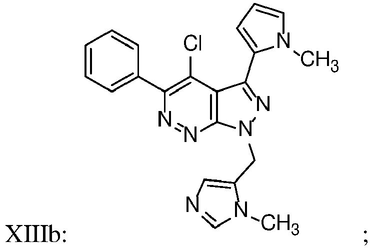 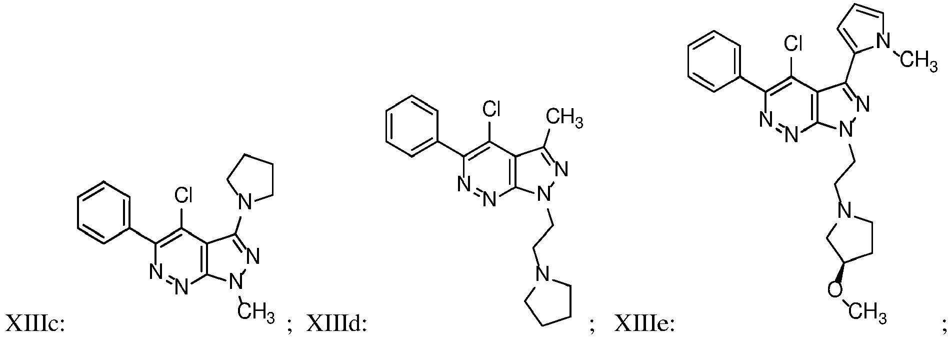 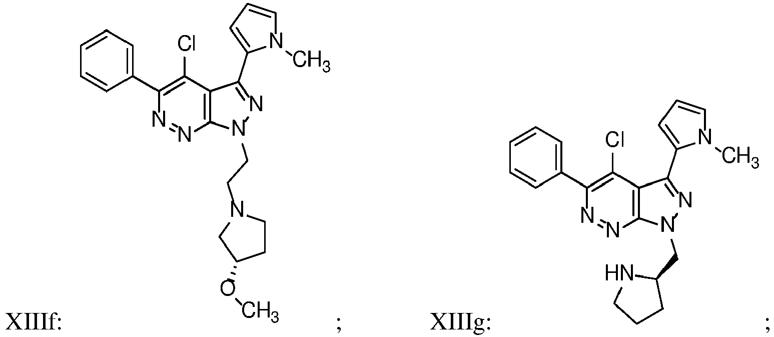 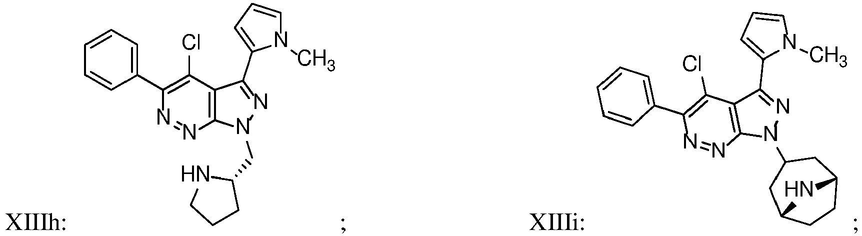 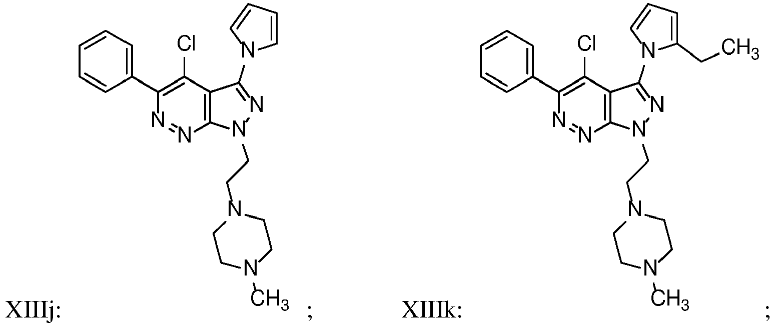 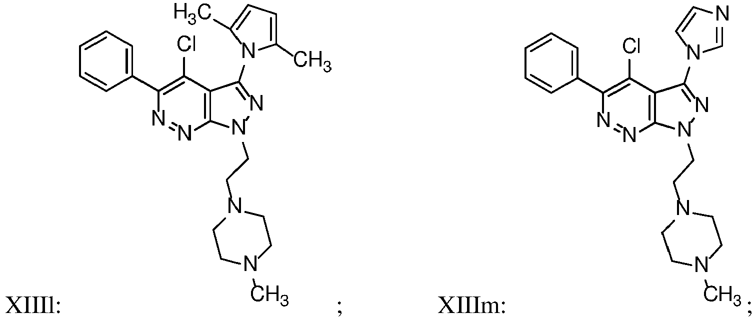 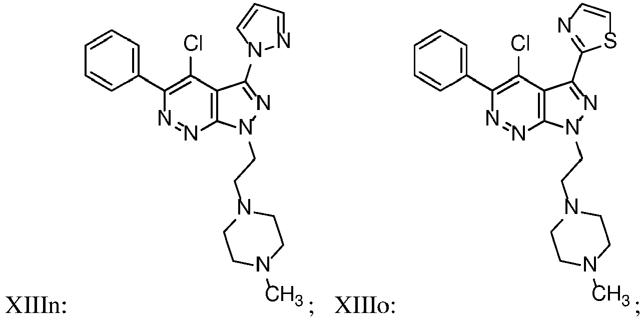 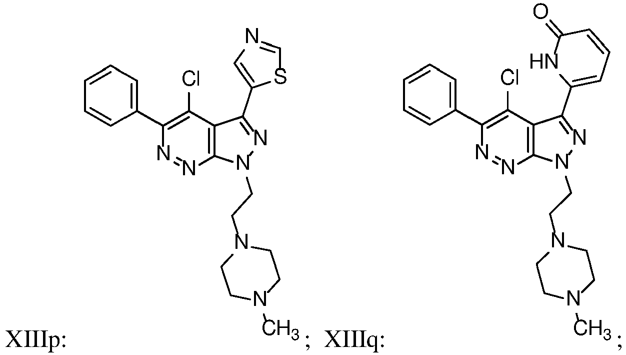 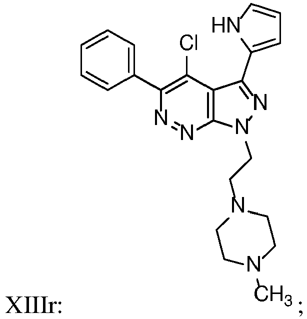 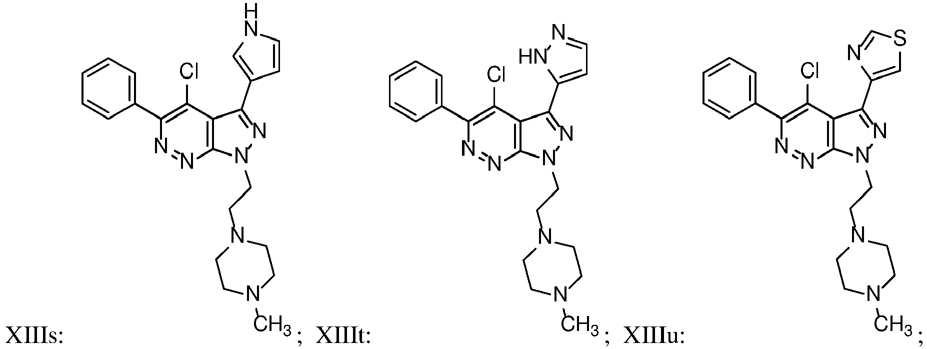 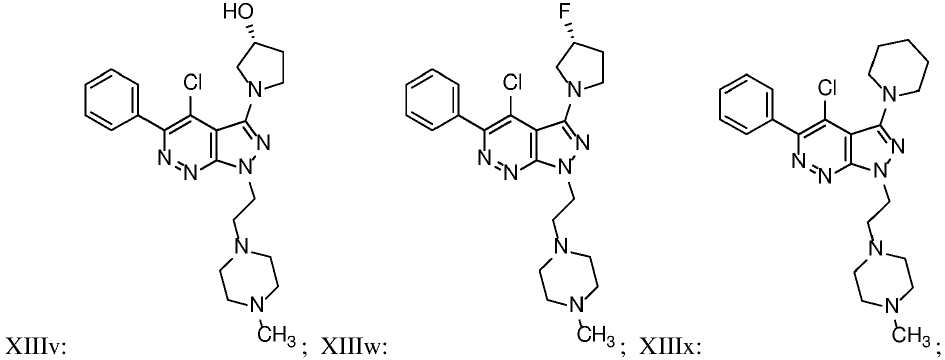 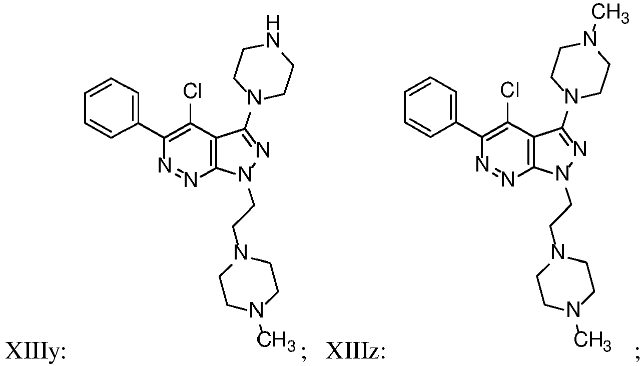 or a pharmaceutically acceptable salt thereof.

Another example (not in accordance with the invention) of a compound of Formula XIII is a pharmaceutically acceptable salt having the structure:

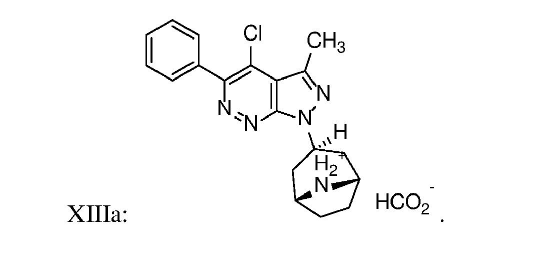

### Pyrazolopyridazine Compounds of Formula XIV (not in accordance with the invention)

An example of a compound of Formula XIV (not in accordance with the present invention) is the compounds having the structure: 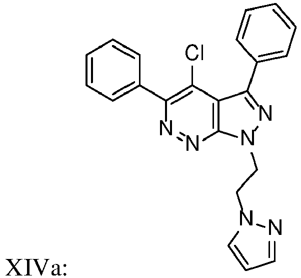 or a pharmaceutically acceptable salt thereof.

### Pyrazolopyridazine Compounds of Formula XV (not in accordance with the invention)

Examples of compounds of Formula XV (not in accordance with the present invention) are compounds having the structure: 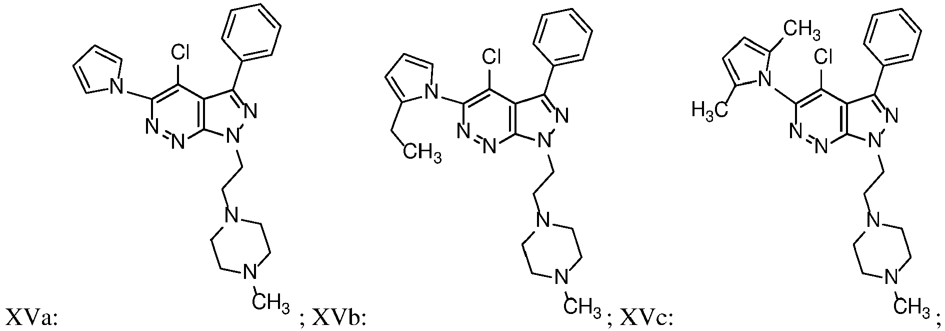 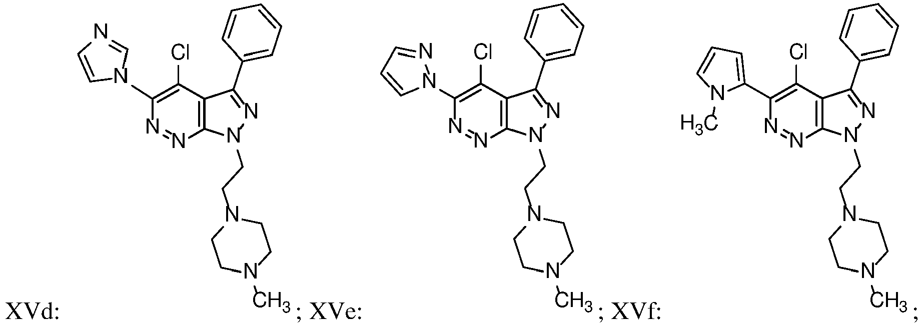 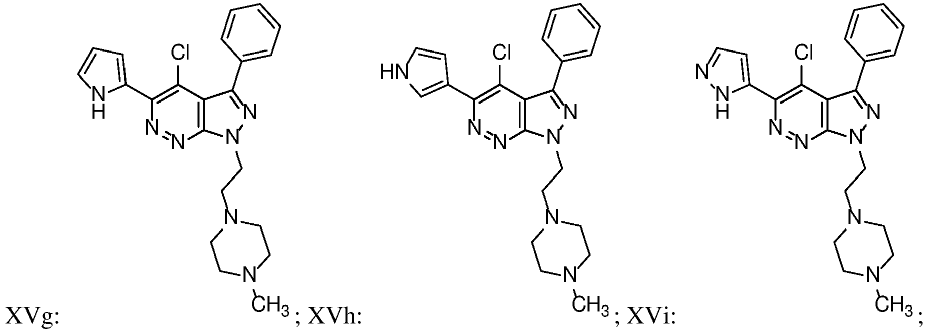 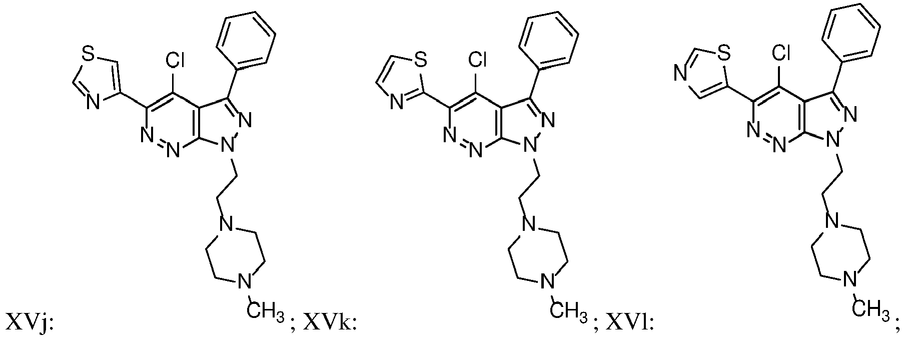 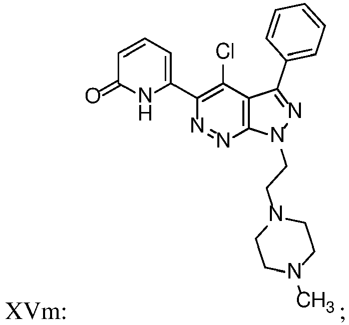 or a pharmaceutically acceptable salt thereof.

### Other Pyrazolopyridazine Compounds (not in accordance with the invention)

Also described herein (not in accordance with the invention) are methods for treating or preventing a proteopathy, comprising administering to a subject in need thereof an effective amount of a compound having the structure: 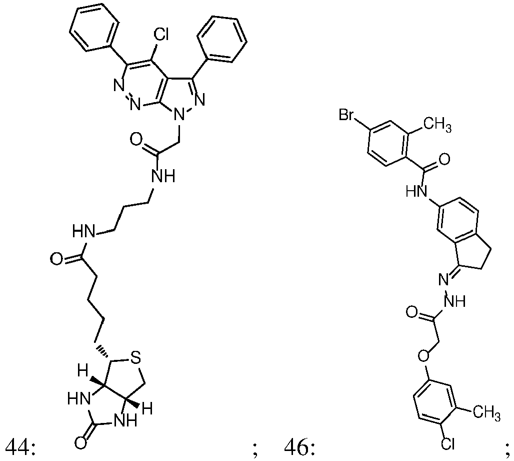 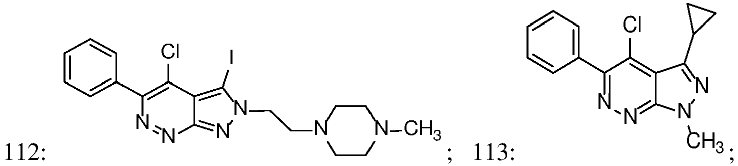 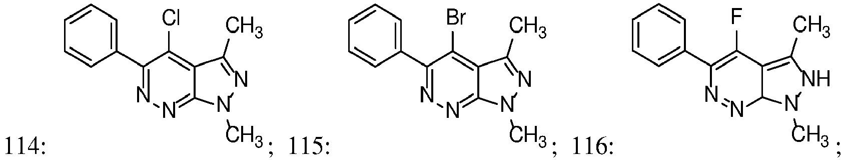 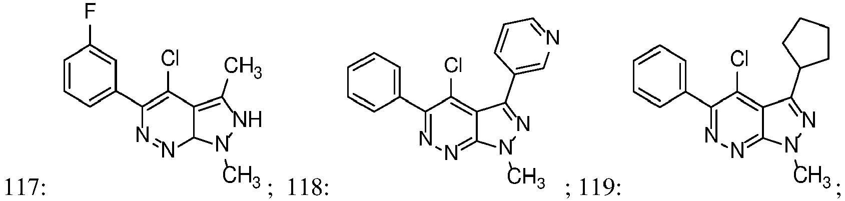 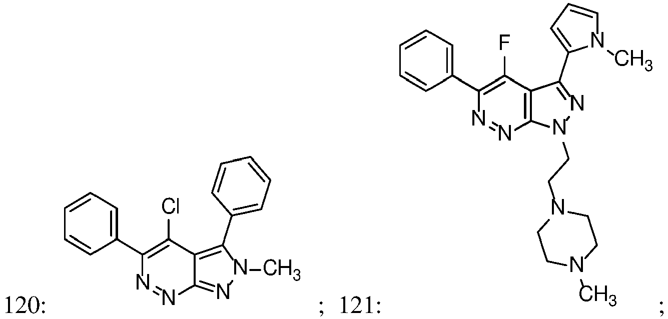 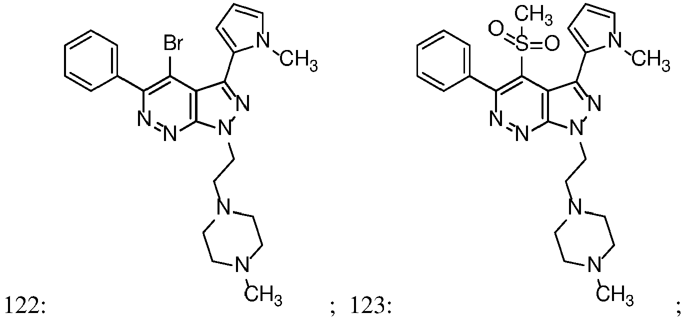 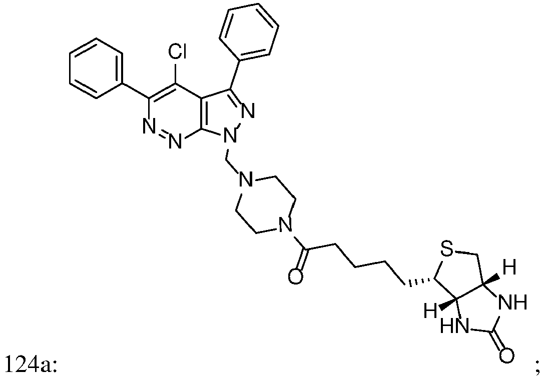 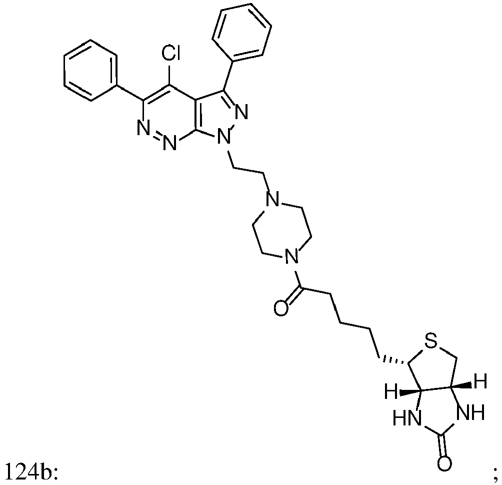 or a pharmaceutically acceptable salt thereof.

### Non-Pyrazolopyridazine Compounds (not in accordance with the invention)

A compound or pharmaceutically acceptable salt of the compound of Table 1 below is a non-Pyrazolopyridazine compound.

Also described herein (not in accordance with the invention) are methods for treating or preventing a proteopathy, comprising administering to a subject in need thereof an effective amount of a non-Pyrazolopyridazine compound.

**Table 1. Non-Pyrazolopyridazine compounds**

| # | Structure |
|---|---|
| 125 | 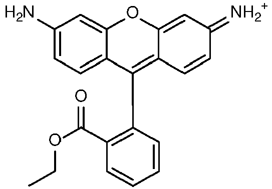 |
| 126 | 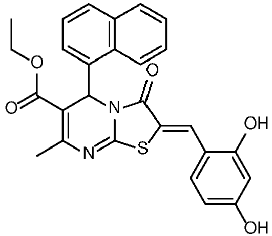 |
| 127 | 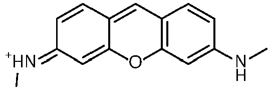 |
| 128 | 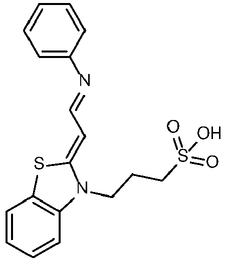 |
| 129 | 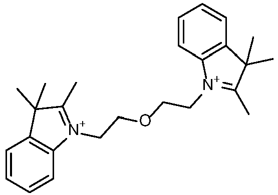 |
| 130 | 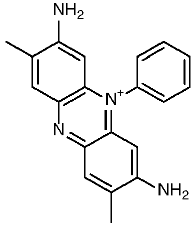 |
| 131 | 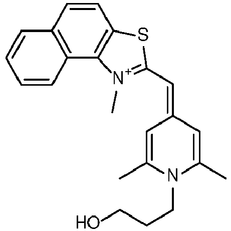 |
| 132 | 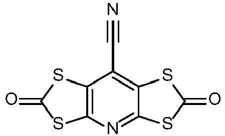 |
| 133 | 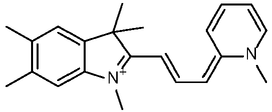 |
| 134 | 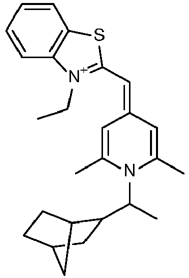 |
| 135 | 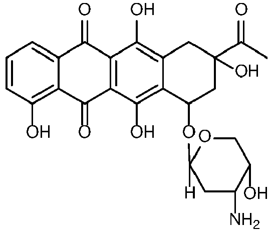 |
| 136 | 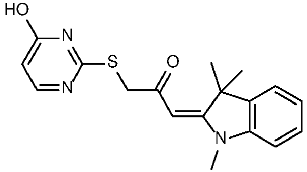 |
| 137 | 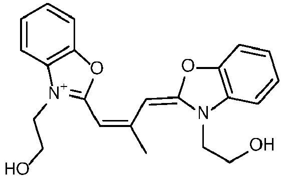 |
| 138 | 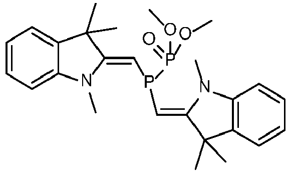 |
| 139 | 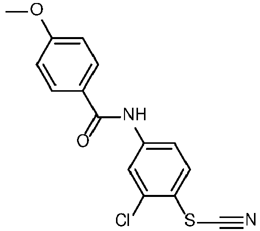 |
| 140 | 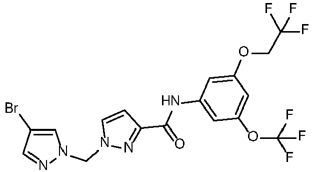 |
| 141 | 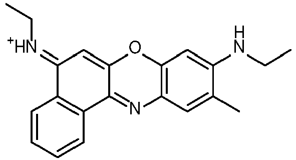 |
| 142 | 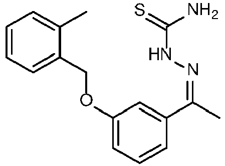 |
| 143 | 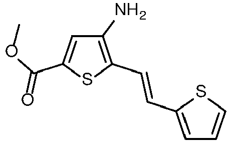 |
| 144 | 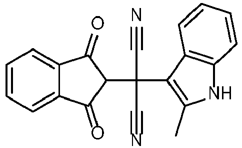 |
| 145 | 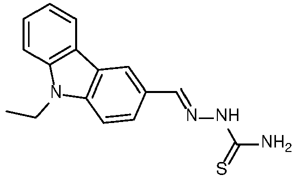 |
| 146 | 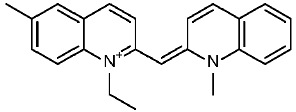 |
| 147 | 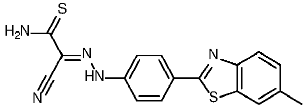 |
| 148 | 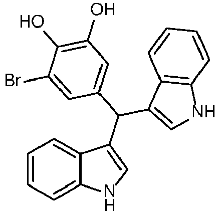 |
| 149 | 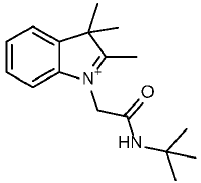 |
| 150 | 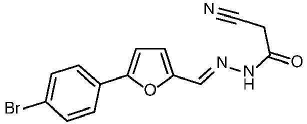 |
| 151 | 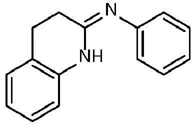 |
| 152 | 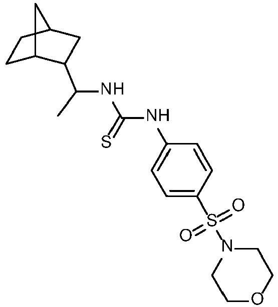 |
| 153 | 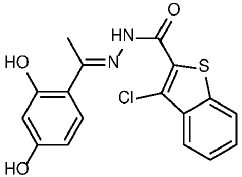 |
| 154 | 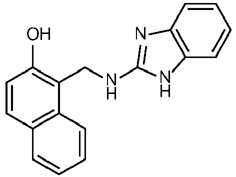 |
| 155 | 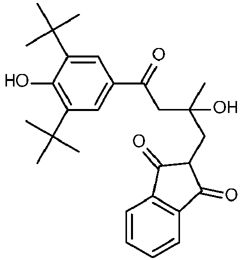 |
| 156 | 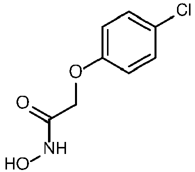 |
| 157 | 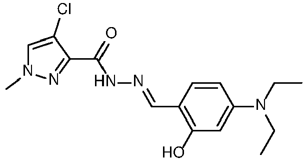 |
| 158 | 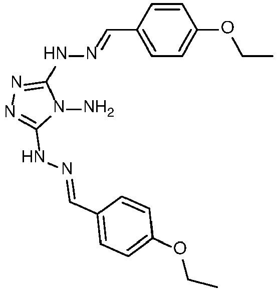 |
| 159 | 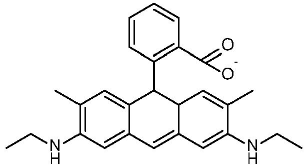 |
| 160 | 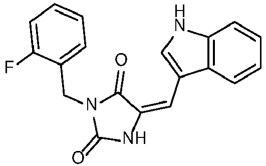 |
| 161 | 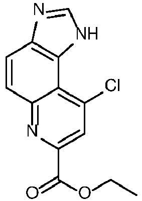 |
| 162 | 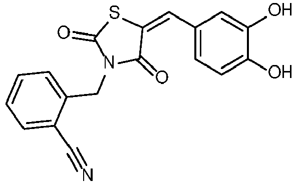 |
| 163 | 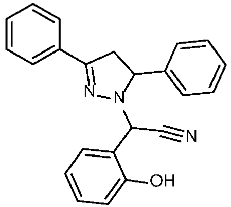 |
| 164 | 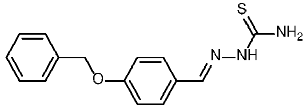 |
| 165 | 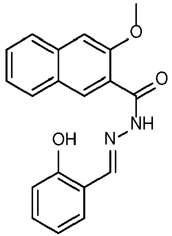 |
| 166 | 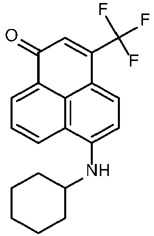 |
| 167 | 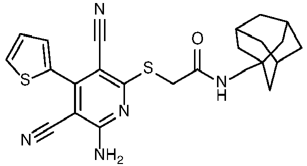 |
| 168 | 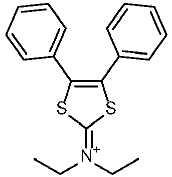 |
| 169 | 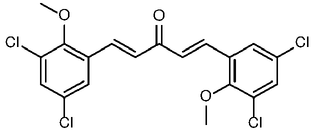 |
| 170 | 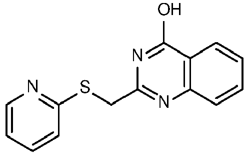 |
| 171 | 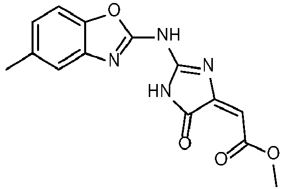 |
| 172 | 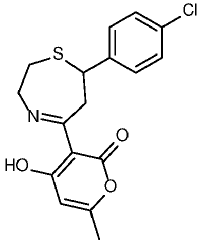 |
| 173 | 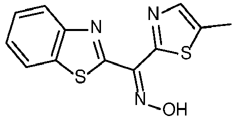 |
| 174 | 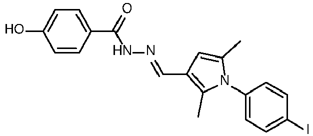 |
| 175 | 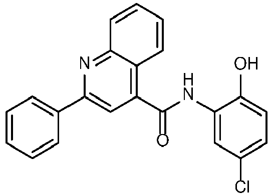 |
| 176 | 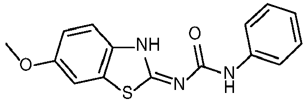 |
| 177 | 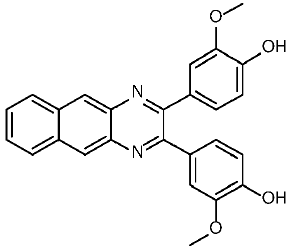 |
| 178 | 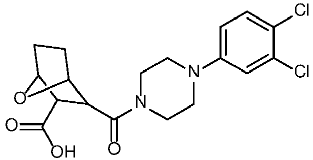 |
| 179 | 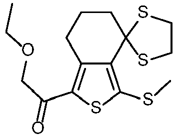 |
| 180 | 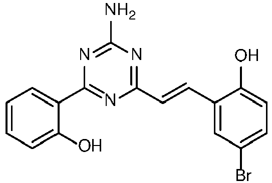 |
| 181 | 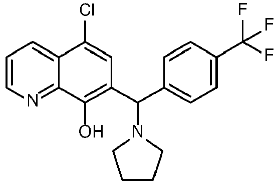 |
| 182 | 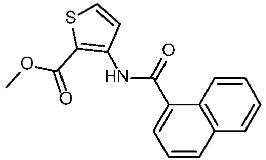 |
| 183 | 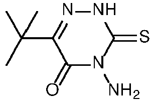 |
| 184 | 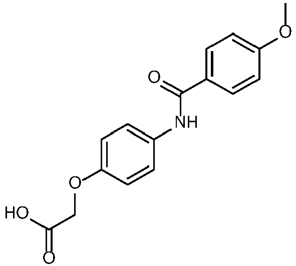 |
| 185 | 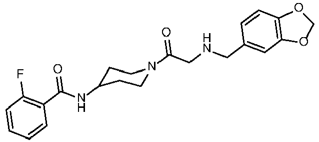 |
| 186 | 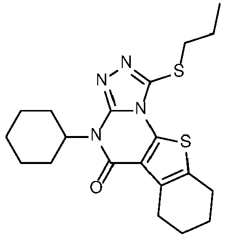 |
| 187 | 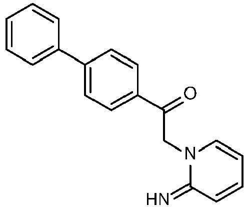 |
| 188 | 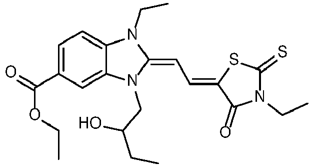 |
| 189 | 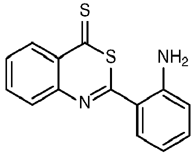 |
| 190 | 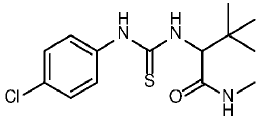 |
| 191 | 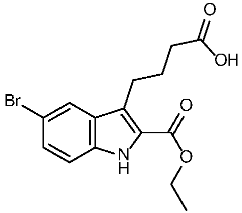 |
| 192 | 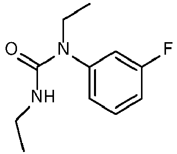 |
| 193 | 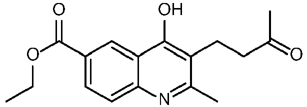 |
| 194 | 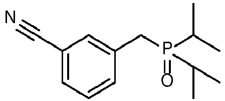 |
| 195 | 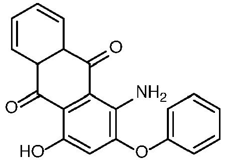 |
| 196 | 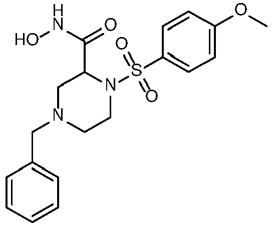 |
| 197 | 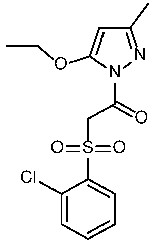 |
| 198 | 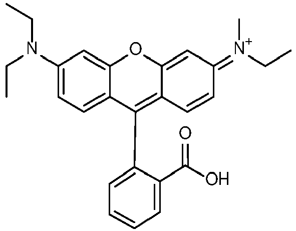 |
| 199 | 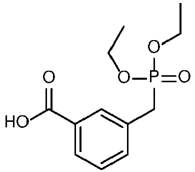 |
| 200 | 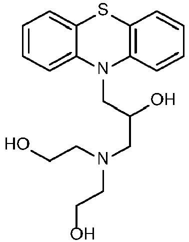 |
| 201 | 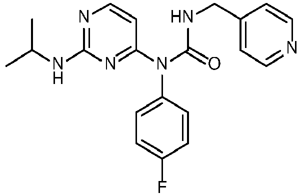 |
| 202 | 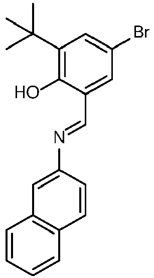 |
| 203 | 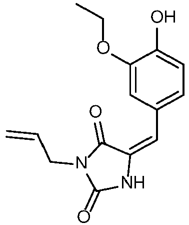 |
| 204 | 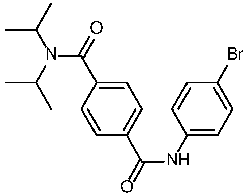 |
| 205 | 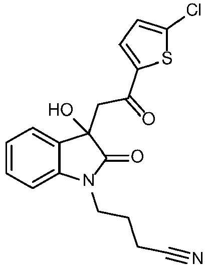 |
| 206 | 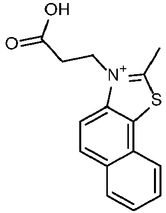 |
| 207 | 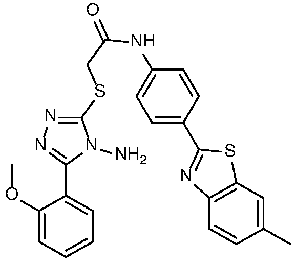 |
| 208 | 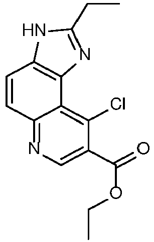 |
| 209 | 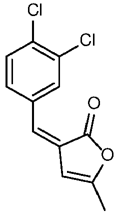 |
| 210 | 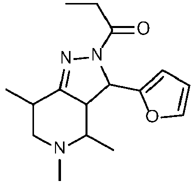 |
| 211 | 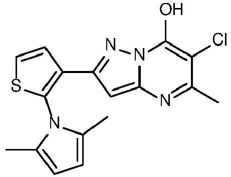 |
| 212 | 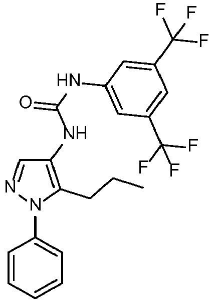 |
| 213 | 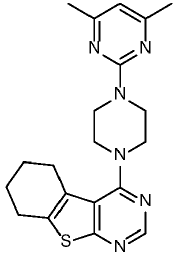 |

Some of the compounds disclosed herein, for example, Compounds 44, 63, 72, 74, 83, 88, 89, 98-101, 111, 124a, 124b, Vp, Vq, Vt, VIj, VIt, VIu, VIx, VIy, XIIIa, XIIIe, XIIIf, XIIIg, XIIIh, XIIIi, XIIIv, and XIIIw are depicted having a bold or hatched wedge, indicating absolute stereochemistry.

The Pyrazolopyridazine compounds as well as the non-Pyrazolopyridazine compounds can be in the form of a salt. In some embodiments, the salt of a Pyrazolopyridazine compound of Formula II is a pharmaceutically acceptable salt. Pharmaceutically acceptable salts include, for example, acid-addition salts and base-addition salts. The acid that forms an acid-addition salt can be an organic acid or an inorganic acid. A base that forms a base-addition salt can be an organic base or an inorganic base. In some embodiments, a pharmaceutically acceptable salt is a metal salt. In some embodiments, a pharmaceutically acceptable salt is an ammonium salt.

Acid-addition salts can arise from the addition of an acid to the free-base form of a compound. In some embodiments, the acid is organic. In some embodiments, the acid is inorganic. Non-limiting examples of suitable acids include hydrochloric acid, hydrobromic acid, hydroiodic acid, nitric acid, nitrous acid, sulfuric acid, sulfurous acid, a phosphoric acid, nicotinic acid, isonicotinic acid, lactic acid, salicylic acid, 4-aminosalicylic acid, tartaric acid, ascorbic acid, gentisinic acid, gluconic acid, glucaronic acid, saccaric acid, formic acid, benzoic acid, glutamic acid, pantothenic acid, acetic acid, propionic acid, butyric acid, fumaric acid, succinic acid, citric acid, oxalic acid, maleic acid, hydroxymaleic acid, methylmaleic acid, glycolic acid, malic acid, cinnamic acid, mandelic acid, 2-phenoxybenzoic acid, 2-acetoxybenzoic acid, embonic acid, phenylacetic acid, N-cyclohexylsulfamic acid, methanesulfonic acid, ethanesulfonic acid, benzenesulfonic acid, p-toluenesulfonic acid, 2-hydroxyethanesulfonic acid, ethane-1,2-disulfonic acid, 4-methylbenzenesulfonic acid, naphthalene-2-sulfonic acid, naphthalene-1,5-disulfonic acid, 2-phosphoglyceric acid, 3-phosphoglyceric acid, glucose-6-phosphoric acid, and an amino acid.

Non-limiting examples of suitable acid-addition salts include a hydrochloride salt, a hydrobromide salt, a hydroiodide salt, a nitrate salt, a nitrite salt, a sulfate salt, a sulfite salt, a phosphate salt, a hydrogen phosphate salt, a dihydrogen phosphate salt, a carbonate salt, a bicarbonate salt, a nicotinate salt, an isonicotinate salt, a lactate salt, a salicylate salt, a 4-aminosalicylate salt, a tartrate salt, an ascorbate salt, a gentisinate salt, a gluconate salt, a glucaronate salt, a saccarate salt, a formate salt, a benzoate salt, a glutamate salt, a pantothenate salt, an acetate salt, a propionate salt, a butyrate salt, a fumarate salt, a succinate salt, a citrate salt, an oxalate salt, a maleate salt, a hydroxymaleate salt, a methylmaleate salt, a glycolate salt, a malate salt, a cinnamate salt, a mandelate salt, a 2-phenoxybenzoate salt, a 2-acetoxybenzoate salt, an embonate salt, a phenylacetate salt, an N-cyclohexylsulfamate salt, a methanesulfonate salt, an ethanesulfonate salt, a benzenesulfonate salt, a p-toluenesulfonate salt, a 2-hydroxyethanesulfonate salt, an ethane-1,2-disulfonate salt, a 4-methylbenzenesulfonate salt, a naphthalene-2-sulfonate salt, a naphthalene-1,5-disulfonate salt, a 2-phosphoglycerate salt, a 3-phosphoglycerate salt, a glucose-6-phosphate salt, and an amino acid salt.

Metal salts can arise from the addition of an inorganic base to a Pyrazolopyridazine compound of Formula II having a carboxyl group. The inorganic base consists of a metal cation paired with a basic couterion, such as, for example, hydroxide, carbonate, bicarbonate, or phosphate. The metal can be an alkali metal, alkaline earth metal, transition metal, or main group metal. Non-limiting examples of suitable metals include lithium, sodium, potassium, cesium, cerium, magnesium, manganese, iron, calcium, strontium, cobalt, titanium, aluminum, copper, cadmium, and zinc.

Non-limiting examples of suitable metal salts include a lithium salt, a sodium salt, a potassium salt, a cesium salt, a cerium salt, a magnesium salt, a manganese salt, an iron salt, a calcium salt, a strontium salt, a cobalt salt, a titanium salt, a aluminum salt, a copper salt, a cadmium salt, and a zinc salt.

Ammonium salts can arise from the addition of ammonia or an organic amine to a Pyrazolopyridazine compound of Formula II having a carboxyl group. Non-limiting examples of suitable organic amines include triethyl amine, diisopropyl amine, ethanol amine, diethanol amine, triethanol amine, morpholine, N-methylmorpholine, piperidine, N-methylpiperidine, N-ethylpiperidine, dibenzyl amine, piperazine, pyridine, pyrrazole, imidazole, pyrazine, pipyrazine, ethylenediamine, N,N'-dibenzylethylene diamine, procaine, chloroprocaine, choline, dicyclohexyl amine, and N-methylglucamine.

Non-limiting examples of suitable ammonium salts include is a triethylammonium salt, a diisopropylammonium salt, an ethanolammonium salt, a diethanolammonium salt, a triethanolammonium salt, a morpholinium salt, an N-methylmorpholinium salt, a piperidinium salt, an N-methylpiperidinium salt, an N-ethylpiperidinium salt, a dibenzylammonium salt, a piperazinium salt, a pyridinium salt, a pyrrazolium salt, an imidazolium salt, a pyrazinium salt, an ethylenediammonium salt, an N,N'-dibenzylethylenediammonium salt, a procaine salt, a chloroprocaine salt, a choline salt, a dicyclohexylammonium salt, and a N-methylglucamine salt.

### Methods for Making the Pyrazolopyridazine Compounds

Methods for making the Pyrazolopyridazine compounds are disclosed in U.S. Patent No. 8,765,762, US 2013/0252936 and US 2014/0121197.

Compound 114 can be synthesized according to Vasilevsky, S.F. & Tretyakov, E.V. Cinnolines and pyrazolopyridazines. - Novel synthetic and mechanistic aspects of the Richter reaction. Liebigs Annalen 1995, 775-779 (1995).

Non-limiting examples of synthetic schema that are useful for synthesizing the Pyrazolopyridazine compounds include the following.

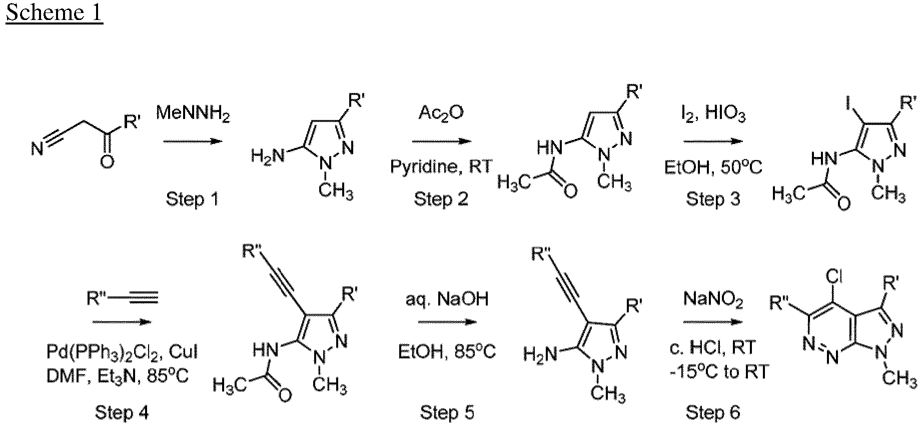

Scheme 1 generally describes the preparation of Pyrazolopyridazine compounds having a 1-N-methyl group and where R' and R" are independently an unsubstituted or a substituted phenyl group. For example, a 2-cyanocarbonyl compound in which R' is unsubstituted or substituted phenyl is condensed with N-methylhydrazine to provide a 3-substituted-1-methyl-1*H*-pyrazol-5-amine. The 5-amino group is acylated, for example, with acetic anhydride in the presence of a base, such as pyridine, to provide a 5-amido compound. The 5-amido compound is iodinated, for example, with a mixture of iodine and iodic acid in a solvent such as ethanol (EtOH) to provide an *N*-(3-substituted-4-iodo-1-methyl-1*H*-pyrazol-5-yl)acetamide. A palladium-mediated cross-coupling, such as a Sonagashira cross-coupling, of the acetamide with an R"-substituted terminal alkyne, catalyzed, for example, by a palladium complex such as palladium (II) bistriphenylphosphine dichloride in the presence of copper (I) iodide in a solvent such as dimethylformamide (DMF) with a base such as triethylamine provides a disubstituted alkyne in which R" is unsubstituted or substituted phenyl. Saponification of the alkyne acetamide with a base such as sodium hydroxide in a solvent such as ethanol provides the primary amine. Diazotization of the primary amine with sodium nitrite in concentrated hydrochloric acid provides a diazo intermediate, which cyclizes to provide a Pyrazolopyridazine compound having a 1-N-methyl group and where R' and R" are independently an unsubstituted or a substituted phenyl group.

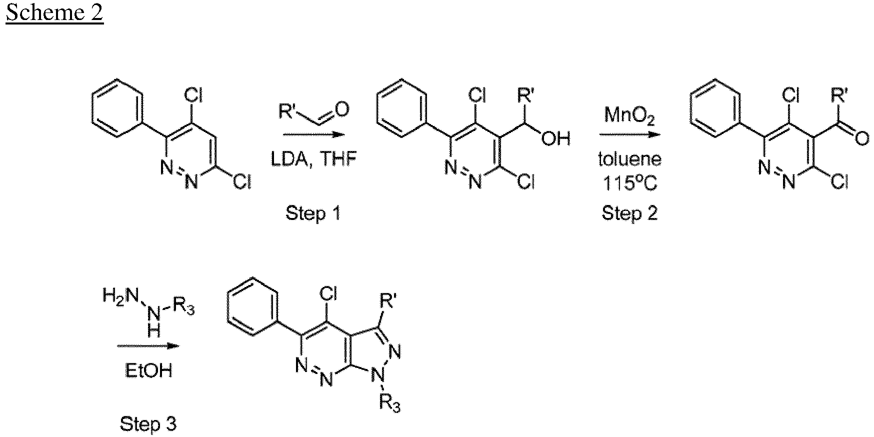

Scheme 2 generally describes the preparation of Pyrazolopyridazine compounds having an R₃ group and in which R' is an unsubstituted or a substituted phenyl group. R' and R3 can be the same or different. For example, 4,6-dichloro-3-phenylpyridazine is deprotonated with a base such as lithium diisopropyl amide (LDA) in a solvent such as tetrahydrofuran (THF), and the resultant 5-lithio species is condensed with an unsubstituted or a substituted benzaldehyde to provide a secondary alcohol. The alcohol is oxidized to a ketone with an oxidizing agent such as manganese dioxide in a solvent such as toluene. The ketone is condensed with an R3-substituted hydrazine in a solvent such as ethanol to provide an intermediate hydrazone, which cyclizes to provide a Pyrazolopyridazine compound having a 1-N- R₃ group, in which R₃ is defined as in Formulas II and III and in which R' is an unsubstituted or a substituted phenyl group.

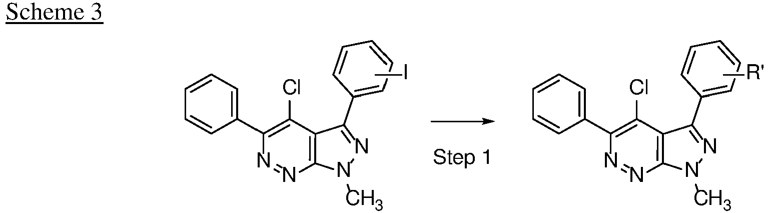

Scheme 3 generally describes the preparation of Pyrazolopyridazine compounds having a 1-N-methyl group and where R' is a cyano group, an alkyne, an alkene or an aryl group. For example, 1-methyl-3-iodophenyl-4-chloro-5-phenyl-1*H*-pyrazolo[3,4-c]pyridazine is coupled with a suitable coupling partner, such as a cyanide salt, a terminal alkyne, an alkenyl halide, or an aryl halide, optionally in the presence of a suitable catalyst such as a palladium complex, optionally in the presence of a non-palladium transition metal salt such as a zinc or copper salt, optionally in the presence of an additive such as triphenylphosphine or an organic amine base, to provide a Pyrazolopyridazine compound having a 1-N-methyl group and where R' is a cyano group, an alkyne, an alkene or an aryl group. The position of R' , i.e., *ortho, meta* or *para,* in the product is the same as the position of the iodo group in the starting material.

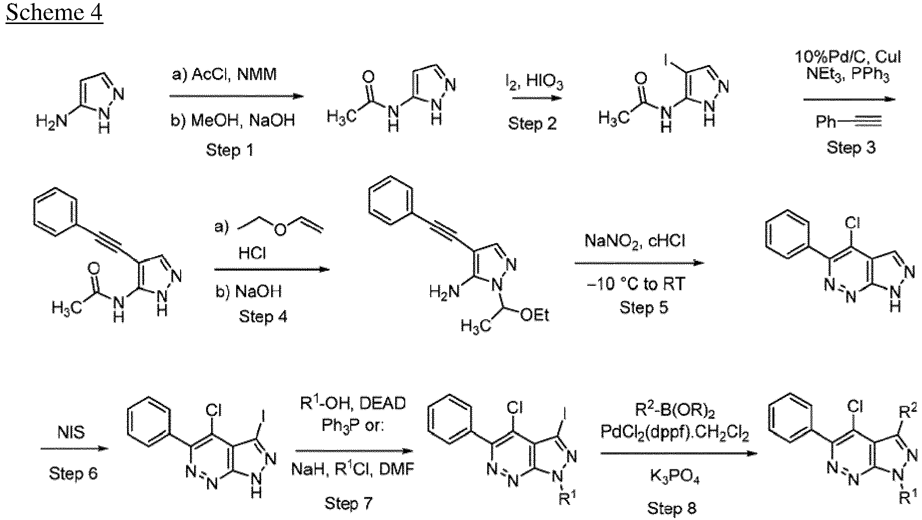

Scheme 4 generally describes the preparation of Pyrazolopyridazine compounds. 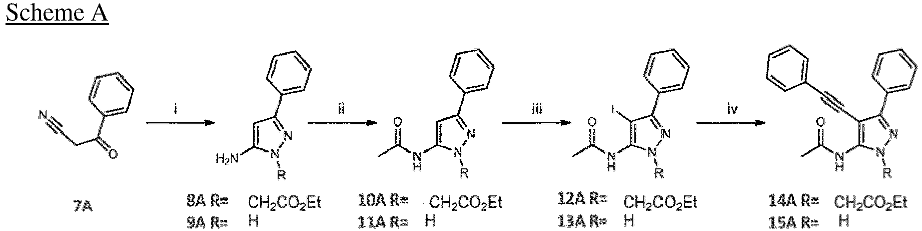 (i) ethyl hydrazinoacetate hydrochloride, EtOH, reflux, 2 h; (ii) Ac₂O, pyridine, 25°C, 16 h, or AcCl, N-methylmorpholine, CH₂Cl₂, 25°C, 3 h; (iii) I₂, HIO₃, EtOH, 50°C; (iv) Phenyl acetylene, Pd(PPh₃)₂Cl₂, CuI, Et3N, DMF, 90°C.

Scheme A generally describes the preparation of Ethyl 2-[5-acetamido-3-phenyl-4-(2-phenylethynyl)-1*H*-pyrazol-1-yl]acetate and N-[3-Phenyl-4-(2-phenylethynyl)-1*H-*pyrazol-5-yl]acetamide from benzoylacetonitrile.

### Compound 8A of Scheme A: Ethyl 2-(5-amino-3-(phenyl)-1H-pyrazol-1-yl)acetate

A mixture of benzoylacetonitrile (**7A,** 44 g, 304 mmol) and ethyl hydrazinoacetate hydrochloride (47 g, 304 mmol) in ethanol (400 mL) is heated to reflux for 2 h. The reaction mixture is concentrated *in vacuo.* The crude reaction mixture is partitioned between CH₂Cl₂ (400 mL) and saturated NaHCO₃ (aq). The aqueous phase is extracted with CH₂Cl₂ and the organic phases are combined, dried over MgSO₄, filtered and evaporated to give compound **8A** as a solid (70 g, 95% yield). ¹H NMR (400 MHz, CDCl₃) δ (ppm) 7.73 (m, 2H), 7.38 (m, 2H), 7.26 (m, 1H), 5.96 (s, 1H), 4.86 (s, 2H), 4.25 (m, 2H), 3.7 (s, 2H), 1.28 (s, 3H).

### Compound 10A of Scheme A: Ethyl 2-(5-acetamido-3-phenyl-1H-pyrazol-1-yl)acetate

To a solution of ethyl 2-(5-amino-3-(phenyl)-1*H*-pyrazol-1-yl)acetate (**8A**, 41.7 g, 0.17 mol) in pyridine (200 mL) is added acetic anhydride (17.4 g, 0.17 mol) dropwise at 0°C under an atmosphere of nitrogen. The reaction mixture is stirred at room temperature (RT) for 16 h. The reaction mixture is concentrated *in vacuo.* The residue is diluted with CH₂Cl₂ and water. The layers are separated and the organic layer is washed with water and brine, dried (MgSO₄) and concentrated *in vacuo.* CH₂Cl₂ is added to the residue and the solid is collected by filtration, yielding compound **10A** as a solid (22 g, 45% yield). The mother liquors are concentrated *in vacuo* and washed with cold CH₂Cl₂ to give a second batch of compound **10A** (15 g, 31% yield). ¹H NMR (400 MHz, DMSO-d₆) δ (ppm) 10.13 (s, 1H), 7.81 (d, J = 7.6 Hz, 2H), 7.45 (t, J = 7.6 Hz, 2H), 7.40-7.32 (m, 1H), 6.80 (s, 1H), 5.07 (s, 2H), 4.22 (q, J = 7.1 Hz, 2H), 2.14 (s, 3H), 1.28 (t, J = 7.1 Hz, 3H).

### Compound 11A of Scheme A: N-(3-Phenyl-1H-pyrazol-5-yl)acetamide

To a solution of 3-phenyl-1H-pyrazol-5-amine (**9**, 18.6 g, 0.117 mol) and N-methylmorpholine (30.8 mL, 0.281 mol) in CH₂Cl₂ (250 mL) is added acetyl chloride (20 mL, 0.281 mol) dropwise at 0°C under an atmosphere of nitrogen. The reaction mixture is stirred at RT for 3 h. The reaction mixture is diluted with CH₂Cl₂ and water. The layers are separated and the organic layer is washed with water and brine, dried (phase separator cartridge) and concentrated *in vacuo.* Diethyl ether is added to the residue and the solid is collected by filtration, yielding compound **11A** as a solid (25.1 g, 88% yield). ¹H NMR (400 MHz, DMSO-d₆) δ (ppm) 12.79 (s, 1H), 10.40 (s, 1H), 7.71 (d, J = 7.5 Hz, 2H), 7.44 (dd, J = 7.6, 7.6 Hz, 2H), 7.34 (dd, J = 7.2, 7.2 Hz, 1H), 6.88 (s, 1H), 2.02 (s, 3H).

### Compound 12A of Scheme A: Ethyl 2-(5-acetamido-4-iodo-3-phenyl-1H-pyrazol-1-yl)acetate

A suspension of compound **10A** (37 g, 129 mmol), iodic acid (5.6 g, 32 mmol) and iodine (19.7 g, 77 mmol) in ethanol (400 mL) is heated at 50°C for 2 h and cooled to RT. The reaction mixture is concentrated *in vacuo* and the residue is eluted through a pad of silica gel with CH₂Cl₂ / diethyl ether (1:0 to 97:3). The residue is partitioned between CH₂Cl₂ and 2 M Na₂S₂O₃ solution (aq). The layers are separated and the organic washed is dried (MgSO₄), and concentrated *in vacuo* to give a residue that is partially purified by chromatography (silica gel, CH₂Cl₂/isohexane 1:1 to 1:0, then CH₂Cl₂/diethyl ether 9:1 to 8:2), then triturated with diethyl ether yielding compound **12A** as an off-white solid (43 g, 81% yield). ¹H NMR (400 MHz, CDCl₃) as a 3:1 mixture of rotamers δ (ppm) 7.81 (d, J = 7.6 Hz, 2H), 7.45-7.35 (m, 3H), 7.15 (br s, 0.75H), 6.85 (br s, 0.25H), 4.97 (s, 2H), 4.25 (q, J = 7.1 Hz, 2H), 2.24 (s, 2.25H), 2.04 (s, 0.75H), 1.30 (t, J = 7.1 Hz, 3H).

### Compound 13A of Scheme A: N-(4-Iodo-3-phenyl-1H-pyrazol-5-yl)acetamide

A suspension of compound **11A** (25.1 g, 0.103 mol), iodic acid (4.5 g, 0.026 mol) and iodine (15.7 g, 0.062 mol) in ethanol (250 mL) is heated at 50°C for 3 h and cooled to RT. The reaction mixture i concentrated *in vacuo* and partitioned between CH₂Cl₂ and 2 M Na₂S₂O₃ solution (aq). The layers are separated and the organic washed with brine, dried (phase separator cartridge), and concentrated *in vacuo* to give a mixture of compound **13A** and starting material **11A** (2.2:1, 30.3 g). The mixture is put in reaction again using iodic acid (1.6 g, 9.6 mmol) and iodine (9.7 g, 38 mmol) in ethanol (250 mL) under the same conditions, to give compound **13A** as a solid (31.9 g, 84% yield). ¹H NMR (400 MHz, CDCl₃) δ (ppm) 11.74 - 11.74 (m, 1H), 7.81 (d, J = 7.2 Hz, 2H), 7.59 (s, 1H), 7.49 - 7.38 (m, 3H), 2.31 (s, 3H).

### Compound 14A of Scheme A: Ethyl 2-[5-acetamido-3-phenyl-4-(2-phenylethynyl)-1H-pyrazol-1-yl]acetate

Nitrogen is bubbled through a mixture of compound **12A** (18.6 g, 45 mmol), phenyl acetylene (9.2 g, 90 mmol), copper iodide (860 mg, 4.5 mmol), triethylamine (200 mL) and DMF (75 mL) for 15 min. Bis(triphenylphosphine)palladium(II) dichloride (1.6 g, 2.25 mmol) is added and the reaction mixture is stirred at 90°C under nitrogen for 4.5 h. The reaction mixture is cooled to RT, diluted with ethyl acetate and water. The organic phase is washed with water and brine, dried (MgSO₄), filtered and concentrated *in vacuo.* The residue is partially purified by column chromatography (silica gel, CH₂Cl₂, then isohexane/ethyl acetate 1:1 followed by CH₂Cl₂/ethyl acetate 9:1 to 8:2), then triturated with diethyl ether yielding compound **14A** as a solid (13 g, 75% yield). ¹H NMR (400 MHz, DMSO-d₆) δ (ppm) 10.38 (s, 1H), 8.13-8.09 (m, 2H), 7.60-7.44 (m, 8H), 5.03 (s, 2H), 4.23 (q, J = 7.1 Hz, 2H), 2.17 (s, 3H), 1.28 (t, J = 7.1 Hz, 3H).

### Compound 15A of Scheme A: N-[3-Phenyl-4-(2-phenylethynyl)-1H-pyrazol-5-yl]acetamide

By a similar procedure to that described for the synthesis of compound **14A,** compound **15A** (12.5 g, 48% yield) is obtained from compound **13A** (31.87 g, 86 mmol). ¹H NMR (400 MHz, CDCl₃) δ (ppm) 11.57 - 11.57 (m, 1H), 8.11 (d, J = 7.4 Hz, 2H), 7.91 (s, 1H), 7.55 - 7.49 (m, 2H), 7.44 (dd, J = 7.5, 7.5 Hz, 2H), 7.37 (dd, J = 1.9, 5.0 Hz, 4H), 2.32 (s, 3H). 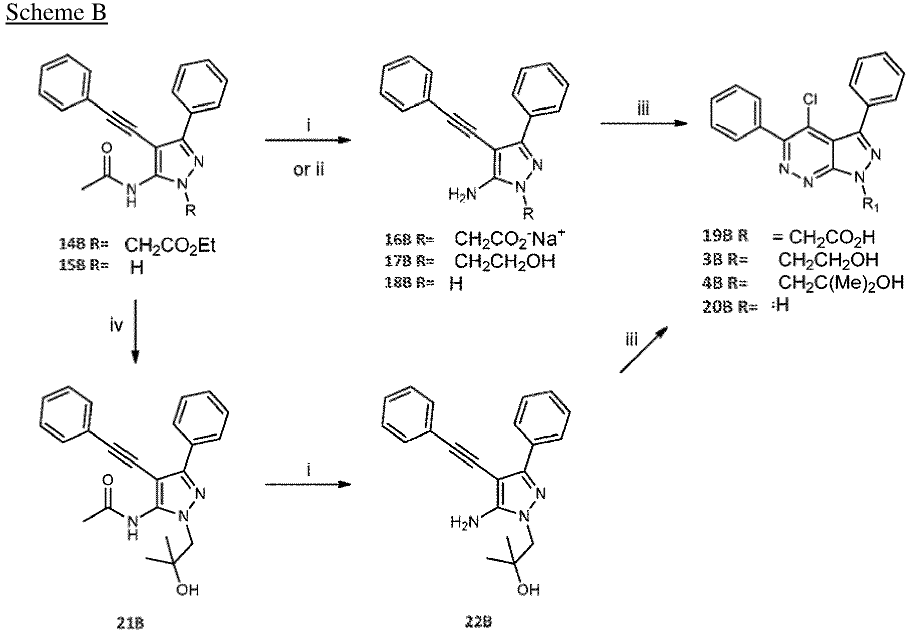 (i) NaOH, EtOH, 80°C; (ii) (a) NaBH₄, EtOH, 25°C; (b) NaOH, EtOH, 80°C; (iii) NaNO₂, c.HCl, -10°C to 25°C; (iv) MeMgCl, THF, 0°C to 25°C.

Scheme B generally describes the preparation of compound **3B** and compound **4B.**

### Compound 16B of Scheme B: Sodium 2-[5-amino-3-phenyl-4-(2-phenylethynyl)-1H-pyrazol-1-yl]acetate

A mixture of compound **14B** (13 g, 34 mmol), ethanol (150 mL) and 25% NaOH solution (aq) (150 mL) is stirred and heated to 80°C for 8 h and cooled to RT. Upon cooling, a precipitate is formed. The precipitate is filtered and washed with a cooled mixture of ethyl acetate/water (1:1). The solid is further triturated with diethyl ether, filtered and dried (MgSO₄), yielding compound **16B** as a solid (9.8 g, 85% yield). ¹H NMR (400 MHz, DMSO-d₆) δ (ppm) 8.06 (d, J = 7.8 Hz, 2H), 7.56 (d, J = 7.6 Hz, 2H), 7.50-7.39 (m, 4H), 7.39-7.31 (m, 2H), 4.31 (s, 2H).

### Compound 17B of Scheme B: 2-[5-Amino-3-phenyl-4-(2-phenylethynyl-1H-)pyrazol-1-yl]ethan1-ol

To a suspension of compound **14B** (22.4 g, 58 mmol) in ethanol (290 mL) is added sodium borohydride (11 g, 289 mmol) and the reaction mixture is stirred at RT for 16 h. The reaction mixture is partially concentrated to a final volume of 250 mL. A 25% NaOH solution (aq) (250 mL) is added and the reaction mixture is stirred at 80°C for 4 h. The reaction mixture is cooled down to room temperature and phases are separated. The aqueous phase is extracted with ethyl acetate three times and the organic phases combined, dried (MgSO₄), filtered and concentrated *in vacuo.* The residue is triturated from diethyl ether (20 mL) and the product is filtered and dried *in vacuo* yielding compound **17B** as an off-white solid (9.96 g, 57% yield). The mother liquor is concentrated *in vacuo* and purified by column chromatography (silica gel, gradient 0 to 100% ethyl acetate/isohexane) yielding a further crop (1.79 g, 10% yield). ¹H NMR (400 MHz, CDCl₃) δ (ppm) 7.78-7.74 (m, 4H), 7.55-7.48 (m, 6H), 4.98 (t, J = 4.8 Hz, 2H), 4.29 (m, 2H), 3.03 (t, J = 6.4 Hz, 1H).

### Compound 18B of Scheme B: 3-Phenyl-4-(2-phenylethynyl)-1H-pyrazol-5-amine

By a similar procedure to that described for the synthesis of compound **16B,** compound **18B** (5.4 g, 50% yield) is obtained from compound **15B** (12.5 g, 41 mmol). ¹H NMR (400 MHz, CDCl₃) δ (ppm) 7.87 (d, J = 7.2 Hz, 2H), 7.51 - 7.43 (m, 4H), 7.42 - 7.32 (m, 4H), 4.09 (s, 2H).

### Compound 19B of Scheme B: 2-(4-Chloro-3,5-diphenyl-1H-pyrazolo[3,4-c]pyridazin-1-yl)acetic acid

Sodium nitrite (1.86 g, 26.9 mmol) is added portionwise to cHCl (30 mL) at 0°C and stirred for 15 min and then compound **16B** (3 g, 8.85 mmol) is added as a solid to the reaction mixture, portionwise. The suspension is then stirred at RT for 16 h. The reaction mixture is diluted with CH₂Cl₂ and washed with water and brine. The organic layer is dried (MgSO₄) and concentrated *in vacuo.* The residue is purified by column chromatography (silica gel, diethyl ether/ CH₂Cl₂ 1:9) yielding compound **19B** as a solid (1.7 g, 53% yield). ¹H NMR (400 MHz, CDCl₃) δ (ppm) 7.80-7.72 (m, 4H), 7.56-7.47 (m, 6H), 5.64 (s, 2H), 2.10 (s, 1H).

### Compound 3B of Scheme B: 2-(4-Chloro-3,5-diphenyl-1H-pyrazolo[3,4-c]pyridazin-1-yl)ethan-1-ol

Sodium nitrite (4.58 g, 66.3 mmol) is added portionwise to cHCl (220 mL) at -10°C and stirred for 10 min. Compound **17B** (6.7 g, 22.1 mmol) is added as a solid. The reaction mixture is allowed to warm up, is sonicated for 5 min then stirred at RT for 2 h. The reaction mixture is diluted with CH₂Cl₂ and water and the aqueous phase is extracted with CH₂Cl₂. The organic phases are combined, dried (MgSO₄),filtered and concentrated *in vacuo.* The residue is partially purified by column chromatography (silica gel, gradient 0 to 100% ethyl acetate/isohexane). The resulting residue is triturated from diethyl ether then from ethyl acetate, yielding compound **3B** as a solid (900 mg, 12% yield). ¹H NMR (400 MHz, CDCl₃) δ (ppm) 7.79-7.75 (m, 4H), 7.55-7.46 (m, 6H), 4.98 (m, 2H), 4.32-4.25 (m, 2H), 3.04 (t, J = 6.4 Hz, 1H). ¹³C NMR (100 MHz, CDCl₃) δ (ppm) 153.09, 151.98, 143.65, 134.48, 130.11, 129.35, 129.33, 129.06, 128.29, 128.17, 127.39, 127.33, 113.70, 60.64, 50.63. LC-MS (analytical method 1: HPLC (Phenomenex Luna 5 µm C18, 100 x 4.6 mm) with gradient of 5-95% acetonitrile in water (with 0.1% formic acid in each mobile phase)) Rt 4.14 min; m/z 351 [M+H] 99.04% purity.

### Compound 21B of Scheme B: N-(1-(2-Hydroxy-2-methylpropyl)-3-phenyl-4-(phenylethynyl)-1H-pyrazol-5-yl)acetamide

To a solution of compound **14B** (1.0 g, 2.58 mmol) in THF (26 mL) is added methyl magnesium chloride (3 M solution in THF, 3 mL, 9 mmol) at 0°C. The solution obtained is stirred at RT for 3.5 h then successively diluted with ethyl acetate and quenched by addition of 1 M HCl (aq). The aqueous phase is extracted with ethyl acetate, and the combined organic layers are dried (MgSO₄) and concentrated *in vacuo.* The resultant residue is purified using chromatography (silica gel, gradient 0 to 75% ethyl acetate/isohexane) yielding compound **21B** as a solid (529 mg, 55% yield). ¹H NMR (400 MHz, CDCl₃) as a 1.5:1 mixture of compound **21B** δ (ppm) 8.11 (dd, J = 7.5, 12.3 Hz, 2H), 7.51 - 7.40 (m, 4H), 7.37 - 7.31 (m, 4H), 4.15 - 4.07 (m, 2H), 2.24 - 2.23 (m, 3H), 1.28 (s, 6H) and N-[2-acetonyl-5-phenyl-4-(2-phenylethynyl)pyrazol-3-yl]acetamide δ (ppm) 8.11 (dd, J = 7.5, 12.3 Hz, 2H), 7.51 - 7.40 (m, 4H), 7.37 - 7.31 (m, 4H), 4.95 (s, 2H), 2.24 - 2.23 (m, 3H), 1.28 (s, 6H).

### Compound 22B of Scheme B: 1-(5-Amino-3-phenyl-4-(phenylethynyl)-1H-pyrazol-1-yl)-2-methylpropan-2-ol

Compound **22B** (310 mg, yield 59%) is synthesized from compound **21B** (597 mg, 1.6 mmol) following similar procedures outlined in the synthesis of compound **16B.** ¹H NMR (400 MHz, CDCl₃) δ (ppm) 8.11 - 8.08 (m, 2H), 7.50 - 7.47 (m, 2H), 7.41 (dd, J = 7.5, 7.5 Hz, 2H), 7.37 - 7.29 (m, 4H), 4.48 (s, 2H), 4.01 (s, 2H), 2.70 (s, 1H), 1.32 - 1.31 (m, 6H).

### Compound 4B of Scheme B: 1-(4-Chloro-3,5-diphenyl-1H-pyrazolo[3,4-c]pyridazin-1-yl)-2-methylpropan-2-ol

To cooled (cooling bath -15°C) cHCl (9 mL) is added sodium nitrite in one portion (121 mg, 1.75 mmol) and the suspension is left to stir for 10 min after which compound **22B** (290 mg, 0.88 mmol) is added. After 5 min, the cooling bath is removed and the reaction mixture is stirred at RT for 3 h. The reaction is cooled again (0°C) and CH₂Cl₂ is added followed by water. The aqueous phase is extracted with CH₂Cl₂ and the organic phases are combined, dried (MgSO₄), filtered and concentrated *in vacuo.* Crude material is purified by column chromatography (silica gel, gradient 0 to 50% ethyl acetate/isohexane) yielding compound **4B** as orange oil (56 mg). The material obtained is further purified by preparative HPLC, yielding compound **4B** as a solid (34 mg, 10% yield). ¹H NMR (400 MHz, CDCl₃) δ (ppm) 7.81-7.75 (m, 4H), 7.55-7.50 (m, 6H), 4.85 (s, 2H), 3.50 (s, 1H), 1.36 (s, 6H). ¹³C NMR (100 MHz, CDCl₃) δ (ppm) 154.36, 152.91, 144.60, 135.42, 130.96, 130.29, 130.28, 129.93, 129.23, 129.07, 128.29, 128.24, 114.16, 71.52, 58.60, 27.26. LCMS (analytical method 1: HPLC (Phenomenex Luna 5 µm C18, 100 x 4.6 mm) with gradient of 5-95% acetonitrile in water (with 0.1% formic acid in each mobile phase)) Rt 4.49 min; m/z 379 [M+H] 99.71% purity.

### Compound 20B of Scheme B: 4-Chloro-3,5-diphenyl-1H-pyrazolo[3,4-c]pyridazine

Sodium nitrite (2.88 g, 42 mmol) is added portionwise to cHCl (314 mL) at -15°C and stirred for 15 min. Compound **18B** (5.4 g, 21 mmol) is added as a solid, followed by the addition of CH₂Cl₂ (10 mL). The reaction mixture is allowed to warm up and stirred at RT for 1 h. The reaction mixture is diluted with CH₂Cl₂ (44 mL) and NaCl (2.7 g) is added. The reaction mixture is heated to 50°C for 1 d. The layers are separated and the organic layer is washed with water, dried (phase separator cartridge) and concentrated *in vacuo.* The residue is purified by column chromatography (silica gel, isohexane/ethyl acetate 4:1, then CH₂Cl₂/ethyl acetate 1:0 to 4:1) yielding compound **20B** as a solid (3.0 g, 47% yield). ¹H NMR (400 MHz, DMSO-d₆) δ (ppm) 15.08 (s, 1H), 7.81 - 7.73 (m, 4H), 7.58 - 7.51 (m, 6H). 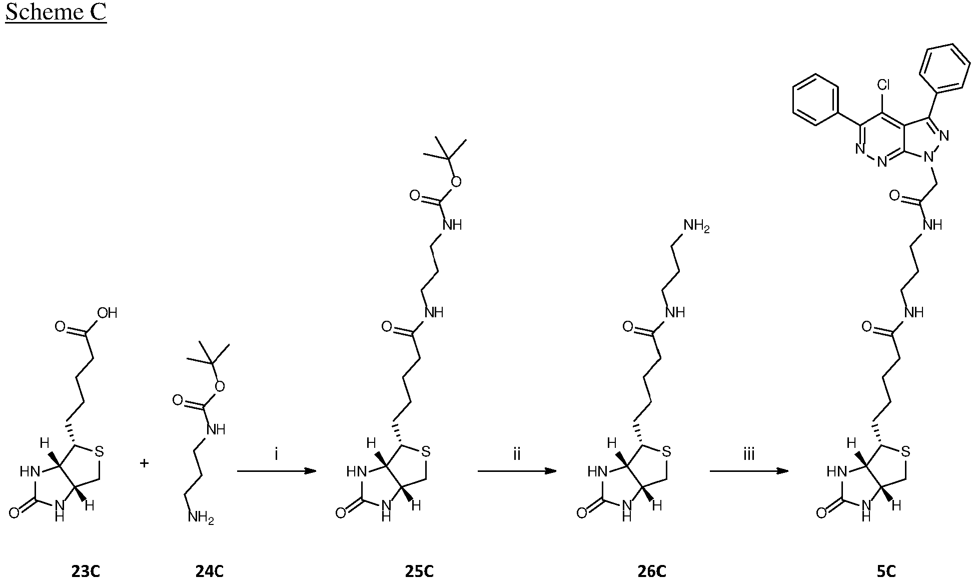 (i) HATU, DIPEA, DMF, 25°C; (ii) TFA, DCM, 25°C; (iii) compound **19B,** HATU, DIPEA, DMF, 25°C.

Scheme C generally describes the preparation of compound **5C.**

### Compound 25C of Scheme C: tert-Butyl (3-(5-((3aS,4S,6aR)-2-oxohexahydro-1H-thieno[3,4-d]imidazol-4-yl)pentanamido)propyl)carbamate

To a solution of biotin (**23C,** 350 mg, 1.43 mmol) in DMF (7.2 mL) are added tert-butyl N-(3-aminopropyl)carbamate (**24C,** 250 mg, 1.43 mmol), DIPEA (0.375 mL, 2.15 mmol) and HATU (816 mg, 2.15 mmol). The reaction mixture is stirred at RT for 20 h, and then diluted with ethyl acetate and 4% LiCl aqueous solution. The aqueous phase is extracted with ethyl acetate twice, and the combined organic layers are dried (MgSO₄) and concentrated *in vacuo.* The resultant residue is purified using chromatography (silica gel, gradient 0 to 12% 7 M NH₃ in MeOH/ CH₂Cl₂) yielding compound **25C** as a solid (180 mg, 31% yield). ¹H NMR (400 MHz, DMSO-d₆) δ (ppm) 7.75 (t, J = 5.2 Hz, 1H), 6.77 (s, 1H), 6.43 (s, 1H), 6.37 (s, 1H), 4.38-4.34 (m, 1H), 4.21-4.16 (m, 1H), 3.23 (d, J = 5.3 Hz, 1H), 3.16 (dq, J = 6.2, 4.3 Hz, 1H), 3.07 (dd, J = 6.8, 12.9 Hz, 2H), 2.96 (dd, J = 6.6, 13.0 Hz, 2H), 2.88 (dd, J = 5.2, 12.4 Hz, 1H), 2.11 (t, J = 7.5 Hz, 2H), 1.73-1.62 (m, 1H), 1.61-1.48 (m, 5H), 1.43 (s, 9H), 1.40-1.29 (m, 2H).

### Compound 26C of Scheme C: N-(3-Aminopropyl)-5-((3aS,4S,6aR)-2-oxohexahydro-1H-thieno[3,4-d]imidazol-4-yl)pentanamide

To a solution of compound **25C** (166 mg, 0.415 mmol) in CH₂Cl₂ (1 mL) is added TFA (1 mL). The reaction mixture is stirred at RT for 2 h, then concentrated *in vacuo.* The resultant residue is dissolved in CH₂Cl₂ (2 mL) and Biotage MP-carbonate resin (550 mg, 1.66 mmol) is added. The reaction mixture is stirred at RT for 30 min. Beads are filtered off and washed with CH₂Cl₂/MeOH (1:1, 2 mL) and the filtrate is concentrated *in vacuo* to afford compound **26C** as a colorless oil (124 mg, 100% yield), which is used as such in the next step.

### Compound 5C of Scheme C: N-(3-(2-(4-Chloro-3,5-diphenyl-1H-pyrazolo[3,4-c]pyridazin-1-yl)acetamido)propyl)-5-((3aS,4S,6aR)-2-oxohexahydro-1H-thieno[3,4-d]imidazol-4-yl)pentanamide

To a solution of compound **26C** (124 mg, 0.415 mmol) in DMF (2 mL) are added compound **19B** (151 mg, 0.415 mmol), DIPEA (0.11 mL, 0.62 mmol) and HATU (236 mg, 0.62 mmol). The reaction mixture is stirred at RT for 1.5 h and then diluted with CH₂Cl₂ and 4% LiCl aqueous solution. The aqueous phase is extracted with CH₂Cl₂ twice, and the combined organic layers are dried (phase separation) and concentrated *in vacuo.* The resultant residue is first purified by prep HPLC yielding 70 mg, which is further purified by silica gel chromatography, yielding the desired compound **5C** as a solid (44 mg, 16% yield). ¹H NMR (400 MHz, DMSO-d₆) δ (ppm) 8.39 (dd, J = 5.7, 5.7 Hz, 1H), 7.87 - 7.78 (m, 5H), 7.64 - 7.57 (m, 6H), 6.45 (s, 1H), 6.39 (s, 1H), 5.51 (s, 2H), 4.33 (dd, J = 5.3, 7.6 Hz, 1H), 4.18 - 4.13 (m, 1H), 3.22 - 3.08 (m, 5H), 2.85 (dd, J = 5.2, 12.5 Hz, 1H), 2.61 (d, J = 12.4 Hz, 1H), 2.10 (dd, J = 7.5, 7.5 Hz, 2H), 1.66 - 1.48 (m, 6H), 1.39 - 1.28 (m, 2H). ¹³C NMR (100 MHz, CDCl₃) δ (ppm) 172.51, 166.25, 163.16, 154.31, 152.75, 144.06, 135.95, 131.35, 130.58, 130.52, 129.58, 129.44, 129.42, 128.75, 128.70, 114.18, 61.47, 59.64, 55.85, 50.91, 37.21, 36.66, 35.66, 29.64, 28.66, 28.48, 25.75. LCMS (analytical method 1: HPLC (Phenomenex Luna 5 µm C18, 100 x 4.6 mm) with gradient of 5-95% acetonitrile in water (with 0.1% formic acid in each mobile phase)) Rt 3.52 min; m/z 647 [M+H] 98.38% purity. 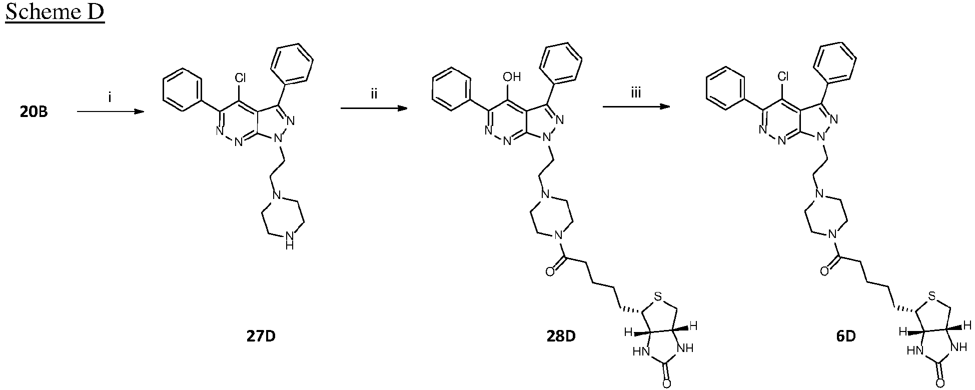 (i) (a) 1-*tert*-Butoxycarbonyl-4-(2-hydroxyethyl)piperazine, DEAD, PPh₃, 1,4-dioxane, 120°C, 1 h; (b) HCl in 1,4-dioxane, 25°C; (ii) (a) HATU, DIPEA, DMF, 25°C; (b) NaOH, 40°C, 1 h; (iii) POCl₃, 25°C.

Scheme D generally describes the preparation of compound **6D** (not in accordance with the invention).

### Compound 27D of Scheme D: 4-Chloro-3,5-diphenyl-1-(2-(piperazin-1-yl)ethyl)-1H-pyrazolo[3,4-c]pyridazine

To a mixture of compound **20B** (345 mg, 1.13 mmol), 1-*tert*-butoxycarbonyl-4-(2-hydroxyethyl)piperazine (520 mg, 2.26 mmol) and triphenylphosphine (888 mg, 2.26 mmol) in 1,4-dioxane (8.4 mL) is slowly added to diethyl azodicarboxylate (0.355 mL, 2.26 mmol) at RT. The reaction mixture is then heated using microwave irradiation to 120°C for 1 h. The reaction mixture is cooled down to RT and 4 M HCl in 1,4-dioxane (4 mL) is added. The reaction mixture is stirred at RT for 4 h, diluted with CH₂Cl₂ (10 mL) and the solution is loaded onto a Biotage SCX-2 cartridge (20 g), eluted with methanol, then 7 M NH₃ in methanol. Fractions were concentrated *in vacuo* to give compound **27D** in a 1:1 ratio with 2-hydroxyethyl)piperazine (950 mg, 100% yield) and is used as such in the next step.

### Compound 28D of Scheme D: (3aS,4S,6aR)-4-(5-(4-(2-(4-Hydroxy-3,5-diphenyl-1H-pyrazolo[3,4-c]pyridazin-1-yl)ethyl)piperazin-1-yl)-5-oxopentyl)tetrahydro-1H-thieno[3,4-d]imidazol-2(3H)-one

To a solution of compound **27D** (950 mg crude, 1.13 mmol) in DMF (5.7 mL), DIPEA (0.59 mL, 3.39 mmol), biotin (678 mg, 2.78 mmol) and HATU (1.29 g, 3.39 mmol) are added. The reaction mixture is stirred at RT for 24 h and then diluted with DMSO (5 mL). NaOH (2 M, 5 mL) is added and the reaction mixture is heated to 40°C for 1 h, then stirred at RT for 2 days. The reaction mixture is purified by preparative HPLC to yield compound **28D** (100 mg, 14% yield). ¹H NMR

### Compound 6D of Scheme D: (3aS,4S,6aR)-4-(5-(4-(2-(4-Chloro-3,5-diphenyl-1H-pyrazolo[3,4-c]pyridazin-1-yl)ethyl)piperazin-1-yl)-5-oxopentyl)tetrahydro-1H-thieno[3,4-d]imidazol-2(3H)-one

A solution of compound **28D** (97 mg, 0.155 mmol) in phosphorous oxychloride (6 mL) is stirred at RT for 2 days. The reaction mixture is diluted with CH₂Cl₂ and 2 M Na₂CO₃ solution. The aqueous phase is extracted twice with CH₂Cl₂. Combined organic layers are dried (MgSO₄) and concentrated *in vacuo.* The resultant residue is purified using chromatography (silica gel, gradient 0 to 12% 7 M NH₃ in MeOH/ CH₂Cl₂) yielding compound **6D** as a solid (42 mg, 42% yield). ¹H NMR (400 MHz, DMSO-d₆) δ (ppm) 7.86-7.78 (m, 4H), 7.66-7.56 (m, 6H), 6.47 (s, 1H), 6.39 (s, 1H), 5.01-4.94 (m, 2H), 4.38-4.32 (m, 1H), 4.20-4.15 (m, 1H), 3.17-3.10 (m, 1H), 3.08-3.01 (m, 2H), 2.87 (dd, J = 12.4, 5.1 Hz, 1H), 2.63 (d, J = 12.4 Hz, 1H), 2.58 (m, 1H), 2.57-2.54 (m, 2H), 2.51 (m, 2H), 2.36-2.28 (m, 2H), 1.71-1.61 (m, 1H), 1.56-1.45 (m, 3H), 1.38 (m, 2H), 1.27-1.17 (m, 1H). ¹³C NMR (100 MHz, CDCl₃) δ (ppm) 170.51, 162.70, 153.69, 151.95, 143.18, 135.52, 131.06, 130.15, 130.11, 129.03, 128.94, 128.84, 128.26, 128.20, 113.42, 61.04, 59.76, 59.19, 56.16, 55.48, 52.71, 52.28, 45.27, 32.05, 28.29, 28.11, 24.85. LCMS (analytical method 2: HPLC (Hichrom ACE 3 C18-AR mixed mode column 100 x 4.6 mm) with gradient of 2-100% acetonitrile in water (with 0.1% formic acid in each mobile phase)) Rt 9.77 min; m/z 645 [M+H] 93.27% purity.

### Molecular Chaperones

Though there are numerous configurations for a particular protein to adopt as it folds, a protein that is ultimately biologically functional folds to a stable state, referred to as its "native state", in which the protein's tendency to aggregate is at a minimum. The folding process of proteins is thermodynamically driven by the so-called "hydrophobic effect", which is the tendency of a protein's hydrophobic amino acid residues to interact with one another and form a hydrophobic core, while the protein's hydrophilic amino acid residues remain at the protein's surface. Nascent or partially folded proteins are "sticky" because their hydrophobic amino acids are not completely buried in the protein's core. As a result, sticky proteins can clump together into intractable aggregates, especially in a cellular environment that is crowded with other protein molecules.

A quality-control system exists in a cell's cytoplasm and nucleus to ensure that protein folding occurs efficiently. This system includes molecular chaperones and the ubiquitin proteasome system (UPS). The UPS allows for tagging of proteins with ubiquitin to target them for degradation in a proteasome, a complex of protein molecules that degrade ubiquitinated polypeptides and recycle the ubiquitin tags.

Molecular chaperones are proteins that help other proteins fold efficiently by shielding the sticky hydrophobic surfaces of unfolded or misfolded proteins, thereby minimizing the proteins' tendency to aggregate. Molecular chaperones can be found in almost all organisms and are present in many cellular compartments. Some molecular chaperones are expressed constitutively and not induced by stress, others are expressed constitutively and induced by stress, and some are induced by stress. Molecular chaperones, such as the heat shock proteins (Hsps), are classified according to their molecular weight and include the small Hsps, Hsp40, Hsp60, Hsp70, Hsp90 and Hsp100 families (Table 2).

**Table 2. Illustrative Families of Heat Shock Proteins**

| **Approximate molecular weight (kD)** | **Heat shock protein** |
|---|---|
| 10 kD | HSP10 |
| 20-30 kD | HSPB group, includes HSP27 |
| 40 kD | HSP40 |
| 60 kD | HSP60 |
| 70 kD | HSPA group, includes HSP71, HSP70, HSP72, Grp78 (BiP), Hsx70 in primates |
| 70 kD | Ribosome-associated complex (RAC) |
| 90 kD | HSPC group, includes HSP90, Grp94 |
| 100 kD | HSPH group, includes HSP104, HSP110 |

A Pyrazolopyridazine compound, non-Pyrazolopyridazine compound or a metabolite thereof may bind to a molecular chaperone. A Pyrazolopyridazine compound, non-Pyrazolopyridazine compound or a metabolite thereof may covalently bind to a molecular chaperone.

The binding of a Pyrazolopyridazine compound, non-Pyrazolopyridazine compound or a metabolite thereof to a molecular chaperone may result in the treatment or prevention of a proteopathy in a subject in need thereof.

For example, the molecular chaperone may be a member of the Hsp10 family, Hsp40 family, Hsp60 family, Hsp70 family, Hsp90 family, or Hsp100 family.

### Proteopathies

The Pyrazolopyridazine compounds of Formula II are useful for treating or preventing a proteopathy.

In some embodiments, the proteopathy is a neurodegenerative disease. Illustrative neurodegenerative diseases include Alzheimer's disease, progressive supranuclear palsy, dementia pugilistica, frontotemporal dementia and parkinsonism linked to chromosome 17 (FTDP-17), Lytico-Bodig disease, tangle-predominant dementia, ganglioma, gangliocytoma, meningioangiomatosis, subacute sclerosing panencephalitis, lead encephalopathy, tuberous sclerosis, Hallervorden-Spatz disease, lipofuscinosis, Pick's disease, corticobasal degeneration, argyrophilic grain disease, Huntington's disease, Parkinson's disease, dementia accompanied by Lewy bodies, multiple system atrophy, neuroaxonal dystrophies, dentatorubralpallidoluysian atrophy (DRPLA), spinal-bulbar muscular atrophy (SBMA), spinocerebellar ataxia 1 (SCA 1), SCA 2, SCA 3, SCA 6, SCA 7, SCA 17, prion disease, amyotrophic lateral sclerosis, frontotemporal lobar degeneration (FTLD) and familial encephalopathy accompanied by neuroserpin inclusion bodies (FENIB).

In some embodiments, the proteopathy is an amyloidosis. Illustrative amyloidoses include familial British dementia (ABri), familial Danish dementia (ADan), hereditary cerebral haemorrhage with amyloidosis-Icelandic (HCHWA-I), familial amyloidotic neuropathy (ATTR), AL (light chain) primary systemic amyloidosis, AH (heavy chain) amyloidosis, AA secondary amyloidosis, Aβ amyloidosis, aortic medial amyloidosis, LECT2 amyloidosis, AIAPP amyloidosis, apolipoprotein AI amyloidosis (AApoAI), apolipoprotein All amyloidosis (AApoAII), apolipoprotein AIV amyloidosis (AApoAIV), familial amyloidosis of the Finnish type (FAF), fibrinogen amyloidosis (AFib), lysozyme amyloidosis (ALys), dialysis amyloidosis (Aβ₂M), medullary thyroid carcinoma (ACal), cardiac atrial amyloidosis (AANF), pituitary prolactinoma (APro), hereditary lattice corneal dystrophy, cutaneous lichen amyloidosis (AKer), Mallory bodies, primary cutaneous amyloidosis, corneal lactoferrin amyloidosis, odontogenic (Pindborg) tumor amyloid or seminal vesicle amyloid.

In some embodiments, the proteopathy is a lysosomal storage disease. Illustrative lysosomal storage diseases include activator deficiency/GM2 gangliosidosis, alpha-mannosidosis, aspartylglucosaminuria, cholesteryl ester storage disease,chronic Hexosaminidase A Deficiency, cystinosis, Danon disease, Fabry disease, Farber disease, fucosidosis, galactosialidosis, Gaucher Disease Type I, Gaucher Disease Type II, Gaucher Disease Type III, GM1 gangliosidosis infantile, GM1 gangliosidosis late infantile/juvenile, GM1 gangliosidosis adult/chronic, I-Cell disease/Mucolipidosis II, Infantile Free Sialic Acid Storage Disease/ISSD, Juvenile Hexosaminidase A Deficiency, Krabbe disease infantile onset, Krabbe disease late onset, lysosomal acid lipase deficiency early onset, lysosomal acid lipase deficiency Late onset, Metachromatic Leukodystrophy, Pseudo-Hurler polydystrophy/Mucolipidosis IIIA, MPS I Hurler Syndrome, MPS I Scheie Syndrome, MPS I Hurler-Scheie Syndrome, MPS II Hunter syndrome, Sanfilippo syndrome Type A/MPS III A, Sanfilippo syndrome Type B/MPS III B, Sanfilippo syndrome Type C/MPS III C, Sanfilippo syndrome Type D/MPS III D, Morquio Type A/MPS IVA, Morquio Type B/MPS IVB, MPS IX Hyaluronidase Deficiency, MPS VI Maroteaux-Lamy, MPS VII Sly Syndrome, Mucolipidosis I/Sialidosis, Mucolipidosis IIIC, Mucolipidosis type IV, Multiple sulfatase deficiency, Niemann-Pick Disease Type A, Niemann-Pick Disease Type B, Niemann-Pick Disease Type C, CLN6 disease-atypical late infantile, CLN6 disease-late onset variant, CLN6 disease-early juvenile, Batten-Spielmeyer-Vogt/Juvenile NCL/CLN3 disease, Finnish Variant Late Infantile CLN5, Jansky-Bielschowsky disease/Late infantile CLN2/TPP1 Disease, Kufs/Adult-onset NCL/CLN4 disease, Northern Epilepsy/variant late infantile CLN8, Santavuori-Haltia/Infantile CLN1/PPT disease, Beta-mannosidosis, Pompe disease/glycogen storage disease type II, Pycnodysostosis, Sandhoff disease/Adult Onset/GM2 Gangliosidosis, Sandhoff disease/GM2 gangliosidosis - Infantile, Sandhoff disease/GM2 gangliosidosis - Juvenile, Schindler disease, Salla disease/Sialic Acid Storage Disease, Tay-Sachs/GM2 gangliosidosis or Wolman disease.

In some embodiments, the lysosomal storage disease is a mucopolysaccharidosis disorder. Illustrative mucopolysaccharidosis disorders include Pseudo-Hurler polydystrophy/Mucolipidosis IIIA, MPS I Hurler Syndrome, MPS I Scheie Syndrome, MPS I Hurler-Scheie Syndrome, MPS II Hunter syndrome, Sanfilippo syndrome Type A/MPS III A, Sanfilippo syndrome Type B/MPS III B, Sanfilippo syndrome Type C/MPS III C, Sanfilippo syndrome Type D/MPS III D, Morquio Type A/MPS IVA, Morquio Type B/MPS IVB, MPS IX Hyaluronidase Deficiency, MPS VI Maroteaux-Lamy, MPS VII Sly Syndrome, Mucolipidosis I/Sialidosis, Mucolipidosis IIIC and Mucolipidosis type IV.

In other embodiments, the lysosomal storage disease is Pompe disease/glycogen storage disease type II.

In accordance with the present invention, the proteopathy to be treated is not a retinal degenerative disease. Non-limiting examples of retinal degenerative diseases include: retinitis pigmentosa, Leber's congenital Amaurosis, Syndromic retinal degenerations, age-related macular degeneration including wet and dry age-related macular degeneration, and Usher Syndrome (including subtypes thereof, for example Usher I, Usher II, and Usher III).

Additional illustrative proteopathies include those disclosed in Table 3.

**Table 3. Illustrative Proteopathies**

| **Proteopathy** |
|---|
| Alzheimer's disease |
| progressive supranuclear palsy |
| dementia pugilistica |
| frontotemporal dementia and parkinsonism linked to chromosome 17 (FTDP-17) |
| Lytico-Bodig disease |
| tangle-predominant dementia |
| ganglioma |
| gangliocytoma |
| meningioangiomatosis |
| subacute sclerosing panencephalitis |
| lead encephalopathy |
| tuberous sclerosis |
| Hallervorden-Spatz disease |
| Lipofuscinosis |
| Pick's disease |
| corticobasal degeneration |
| argyrophilic grain disease |
| Parkinson's disease |
| dementia with Lewy bodies |
| multiple system atrophy |
| neuroaxonal dystrophies |
| Huntington's disease |
| dentatorubralpallidoluysian atrophy (DRPLA) |
| spinal-bulbar muscular atrophy (SBMA) |
| spinocerebellar ataxia 1 (SCA 1) |
| SCA 2 |
| SCA 3/Machado-Joseph disease |
| SCA 6 |
| SCA 7 |
| SCA 17 |
| Prion diseases |
| Amyotrophic lateral sclerosis (ALS) |
| Frontotemporal lobar degeneration (FTLD) (Ubi+, Tau-) |
| FTLD-FUS |
| α1-antitrypsin deficiency |
| thrombosis |
| emphysema |
| types 1 and 2 hereditary angioedema (HAE) |
| familial encephalopathy |
| Familial British dementia, ABri (cerebral amyloid angiopathy) |
| Familial Danish dementia, Adan (cerebral amyloid angiopathy) |
| Hereditary cerebral hemorrhage with amyloidosis (Icelandic) (HCHWA-I) |
| Familial amyloidotic neuropathy, Senile systemic amyloidosis (ATTR) |
| AL (light chain) amyloidosis (primary systemic amyloidosis) |
| AH (heavy chain) amyloidosis |
| AA (secondary) amyloidosis |
| Aβ amyloidosis |
| AIAPP amyloidosis |
| Aortic medial amyloidosis |
| ApoAI amyloidosis (AApoAI) |
| ApoAII amyloidosis (AApoAII) |
| ApoAIV amyloidosis (AApoAIV) |
| Familial amyloidosis of the Finnish type (FAF) |
| Lysozyme amyloidosis (ALys) |
| Fibrinogen amyloidosis (AFib) |
| Dialysis amyloidosis (Aβ₂M) |
| Medullary thyroid carcinoma (ACal) |
| Cardiac atrial amyloidosis (AANF) |
| Pituitary prolactinoma (APro) |
| LECT2 amyloidosis |
| Hereditary lattice corneal dystrophy |
| Cutaneous lichen amyloidosis (AKer) |
| Mallory bodies |
| Primary cutaneous amyloidosis |
| Corneal lactoferrin amyloidosis |
| Odontogenic (Pindborg) tumor amyloid |
| Seminal vesicle amyloid |
| nephrogenic diabetes insipidus (NDI) |
| Iatrogenic (insulin) |
| Inclusion body myositis/myopathy |
| Cancer |
| Cataracts |
| |
| Pulmonary alveolar proteinosis |
| Cystic Fibrosis |
| Sickle cell disease |
| Critical illness myopathy (CIM) |
| Wilson disease |
| Marfan syndrome |
| Fragile X syndrome |
| Fragile XE syndrome |
| Myotonic dystrophy |
| Retinal ganglion cell degeneration in glaucoma |
| Cerebral β-amyloid angiopathy |
| CADASIL (Cerebral Autosomal Dominant Arteriopathy with Sub-cortical Infarcts and Leukoencephalopathy) |
| Alexander disease |
| Seipinopathies |
| Activator Deficiency/GM2 Gangliosidosis |
| Alpha- mannosidosis |
| Aspartylglucosaminuria |
| Cholesteryl ester storage disease |
| Chronic Hexosaminidase A Deficiency |
| Cystinosis |
| Danon disease |
| Fabry disease |
| Farber disease |
| Fucosidosis |
| Galactosialidosis |
| Gaucher Disease Type I |
| Gaucher Disease Type II |
| Gaucher Disease Type III |
| GM1 gangliosidosis Infantile |
| GM1 gangliosidosis Late infantile/Juvenile |
| GM1 gangliosidosis Adult/Chronic |
| I-Cell disease/Mucolipidosis II |
| Infantile Free Sialic Acid Storage Disease/ISSD |
| Juvenile Hexosaminidase A Deficiency |
| Krabbe disease Infantile Onset |
| Krabbe disease Late Onset |
| Lysosomal acid lipase deficiency Early onset |
| Lysosomal acid lipase deficiency Late onset |
| Metachromatic Leukodystrophy |
| Pseudo-Hurler polydystrophy/Mucolipidosis IIIA |
| MPS I Hurler Syndrome |
| MPS I Scheie Syndrome |
| MPS I Hurler-Scheie Syndrome |
| MPS II Hunter syndrome |
| Sanfilippo syndrome Type A/MPS III A |
| Sanfilippo syndrome Type B/MPS III B |
| Sanfilippo syndrome Type C/MPS III C |
| Sanfilippo syndrome Type D/MPS III D |
| Morquio Type A/MPS IVA |
| Morquio Type B/MPS IVB |
| MPS IX Hyaluronidase Deficiency |
| MPS VI Maroteaux-Lamy |
| MPS VII Sly Syndrome |
| Mucolipidosis I/Sialidosis |
| Mucolipidosis IIIC |
| Mucolipidosis type IV |
| Multiple sulfatase deficiency |
| Niemann-Pick Disease Type A |
| Niemann-Pick Disease Type B |
| Niemann-Pick Disease Type C |
| CLN6 disease - Atypical Late Infantile |
| CLN6 disease - Late Onset variant |
| CLN6 disease - Early Juvenile |
| Batten-Spielmeyer-Vogt/Juvenile NCL/CLN3 disease |
| Finnish Variant Late Infantile CLN5 |
| Jansky-Bielschowsky disease/Late infantile CLN2/TPP1 Disease |
| Kufs/Adult-onset NCL/CLN4 disease |
| Northern Epilepsy/variant late infantile CLN8 |
| Santavuori-Haltia/Infantile CLN1/PPT disease |
| Beta-mannosidosis |
| Pompe disease/Glycogen storage disease type II |
| Pycnodysostosis |
| Sandhoff disease/Adult Onset/GM2 Gangliosidosis |
| Sandhoff disease/GM2 gangliosidosis - Infantile |
| Sandhoff disease/GM2 gangliosidosis - Juvenile |
| Schindler disease |
| Salla disease/Sialic Acid Storage Disease |
| Tay-Sachs/GM2 gangliosidosis |
| Wolman disease |

### Cystic Fibrosis

Cystic Fibrosis (CF), also known as mucoviscidosis, is an autosomal recessive disease that affects mostly the lungs but can also impact other organs, such as the pancreas, liver, kidneys and intestines. The name "cystic fibrosis" refers to the characteristic fibrosis and cysts that form within the pancreas. The disease is characterized by difficulty in breathing and by coughing up sputum due to frequent lung infections.

The most common mutation found in about 70% of CF sufferers worldwide is a deletion of a phenylalanine residue at amino acid position 508 in the 1480 amino acid cystic fibrosis transmembrane conductance regulator (CFTR) protein. This mutation causes misfolding of the protein and its degradation by the cell. Other mutations of the CFTR protein can result in truncated versions of the CFTR protein because their production is ended prematurely. Still other mutations produce mutant CFTR proteins that do not use energy normally; that do not allow chloride, iodide or thiocyanate to cross the membrane normally; or that degrade at a faster rate than normal. CFTR protein mutations can also lead to fewer copies of the CFTR protein being produced. A list of CFTR protein mutation classes and illustrative mutations of each class are disclosed in Table 4.

**Table 4. CF mutation classes and illustrative mutations**

| **CF mutation classes** | **Illustrative mutations** |
|---|---|
| I | W1282X, R553X, G542X |
| II | ΔF508, N1303K |
| III | G551D, G551S, G1349D |
| IV | R117H, R334W, R347P |
| V | 2789+5G>A, A455E |
| VI | 120Δ23, N287Y, 4326ΔITC, 4279insA |

Other CFTR protein mutations include G178R, S549N, S549R, G1244E, S1251N and S1255P.

Accordingly, the present invention further provides methods for treating or preventing cystic fibrosis, comprising administering to a subject in need thereof an effective amount of a Pyrazolopyridazine compound or a non-Pyrazolopyridazine compound. In some embodiments, the subject has a CFTR protein mutation. In one embodiment, the subject has a class I CF mutation. In another embodiment, the subject has a class II CF mutation. In another embodiment, the subject has a class III CF mutation. In another embodiment, the subject has a class IV CF mutation. In another embodiment, the subject has a class V CF mutation. In another embodiment, the subject has a class VI CF mutation.

In one embodiment, the mutation is W1282X, R553X or G542X. In another embodiment, the mutation is ΔF508 or N1303K. In another embodiment, the mutation is G551D, G551S or G1349D. In another embodiment, the mutation is R117H, R334W or R347P. In another embodiment, the mutation is 2789+5G>A or A455E. In another embodiment, the mutation is 120Δ23, N287Y, 4326ΔITC or 4279insA.

In one embodiment, the subject has one or more of the following mutations: W1282X, R553X, G542X, ΔF508, N1303K, G551D, G551S, G1349D, R117H, R334W, R347P, 2789+5G>A, A455E, 120Δ23, N287Y, 4326ΔITC, 4279insA, G178R, S549N, S549R, G1244E, S1251N and S1255P.

### Retinitis Pigmentosa (the treatment or prevention of retinitis pigmentosa with a compound of Formula II is not in accordance with the present invention)

Retinitis Pigmentosa (RP) is an inherited, degenerative eye disease that causes severe vision impairment due to progressive degeneration of the rod photoreceptor cells in the retina. The progressive rod degeneration can be followed by abnormalities in the adjacent retinal pigment epithelium (RPE) and the deterioration of cone photoreceptor cells.

There are multiple genes that, when mutated, can cause RP. Inheritance patterns of RP have been identified as autosomal dominant, autosomal recessive, X-linked, and maternally (mitochondrially) acquired. X-linked RP can be either recessive, affecting primarily only males, or dominant, affecting both males and females. Some digenic (controlled by two genes) and mitochondrial forms of RP are also known.

A mutation of the gene for rhodopsin, a pigment that plays an essential role in the visual transduction cascade enabling vision in low-light conditions, has been identified. The mutation substitutes a proline at amino acid position 23 to a histidine. The rhodopsin gene is a principal protein of photoreceptor outer segments and consists of opsin, a light-sensitive membrane-bound G protein-coupled receptor and a reversibly covalently bound cofactor, retinal. Rhodopsin gene mutations most frequently follow autosomal dominant inheritance patterns.

Up to 150 RP-associated opsin gene mutations have been reported since the P23H mutation in the intradiscal domain of the protein was first reported. These mutations are found throughout the opsin gene and are distributed along the three domains of the protein (the intradiscal, transmembrane, and cytoplasmic domains). Mutations in the opsin gene are most commonly missense mutations and cause misfolding of the rhodopsin protein. The mutation of amino acid 23 in the opsin gene, in which proline is replaced with histidine, accounts for the largest percentage of rhodopsin mutations in the United States. Other mutations associated with RP include T58R, P347L, P347S, as well as deletion of Ile 255. The rare P23A mutation causes autosomal dominant RP.

A list of genes mutated in a subject having RP and the type of RP is presented in Table 5.

**Table 5. Illustrative Retinitis Pigmentosa types and genes affected**

| **Gene** | **Type** |
|---|---|
| RP1 | Retinitis pigmentosa-1 |
| RP2 | Retinitis pigmentosa-2 |
| RPGR | Retinitis pigmentosa-3, Retinitis pigmentosa-15, X-linked |
| RHO | Retinitis pigmentosa-4 |
| | Retinitis pigmentosa-6, X-linked |
| ROM1 | Retinitis pigmentosa-7 |
| RP9 | Retinitis pigmentosa-9 |
| IMPDH1 | Retinitis pigmentosa-10 |
| PRPF31 | Retinitis pigmentosa-11 |
| CRB1 | Retinitis pigmentosa-12, autosomal recessive |
| PRPF8 | Retinitis pigmentosa-13 |
| TULP1 | Retinitis pigmentosa-14 |
| CA4 | Retinitis pigmentosa-17 |
| PRPF3 | Retinitis pigmentosa-18 |
| ABCA4 | Retinitis pigmentosa-19 |
| RPE65 | Retinitis pigmentosa-20 |
| | Retinitis pigmentosa-22 |
| OFD1 | Retinitis pigmentosa-23 |
| | Retinitis pigmentosa-24, X-linked |
| EYS | Retinitis pigmentosa-25 |
| CERKL | Retinitis pigmentosa-26 |
| NRL | Retinitis pigmentosa-27 |
| FAM161A | Retinitis pigmentosa-28 |
| FSCN2 | Retinitis pigmentosa-30 |
| TOPORS | Retinitis pigmentosa-31 |
| | Retinitis pigmentosa-32 |
| SNRNP200 | Retinitis pigmentosa-33 |
| | Retinitis pigmentosa-34, X-linked |
| SEMA4A | Retinitis pigmentosa-35 |
| PRCD | Retinitis pigmentosa-36 |
| NR2E3 | Retinitis pigmentosa-37 |
| MERTK | Retinitis pigmentosa-38 |
| USH2A | Retinitis pigmentosa-39 |
| PDE6B | Retinitis pigmentosa-40 |
| PROM1 | Retinitis pigmentosa-41 |
| KLHL7 | Retinitis pigmentosa-42 |
| PDE6A | Retinitis pigmentosa-43 |
| RGR | Retinitis pigmentosa-44 |
| CNGB1 | Retinitis pigmentosa-45 |
| IDH3B | Retinitis pigmentosa-46 |
| SAG | Retinitis pigmentosa-47 |
| GUCA1B | Retinitis pigmentosa-48 |
| CNGA1 | Retinitis pigmentosa-49 |
| BEST1 | Retinitis pigmentosa-50 |
| TTC8 | Retinitis pigmentosa-51 |
| RDH12 | Retinitis pigmentosa-53 |
| C2orf71 | Retinitis pigmentosa-54 |
| ARL6 | Retinitis pigmentosa-55 |
| IMPG2 | Retinitis pigmentosa-56 |
| PDE6G | Retinitis pigmentosa-57 |
| ZNF513 | Retinitis pigmentosa-58 |
| DHDDS | Retinitis pigmentosa-59 |
| PRPF6 | Retinitis pigmentosa-60 |
| CLRN1 | Retinitis pigmentosa-61 |
| MAK | Retinitis pigmentosa-62 |
| | Retinitis pigmentosa-63, autosomal dominant |
| C8ORF37 | Retinitis pigmentosa-64 |
| RBP3 | Retinitis pigmentosa-66 |
| NEK2 | Retinitis pigmentosa-67 |
| SLC7A14 | Retinitis pigmentosa-68 |
| KIZ | Retinitis pigmentosa-69 |
| PRPF4 | Retinitis pigmentosa-70 |
| PRPH2 | Retinitis pigmentosa, autosomal dominant |
| LRAT | Retinitis pigmentosa, juvenile |
| SPATA7 | Retinitis pigmentosa, juvenile, autosomal recessive |
| AIPL1 | Retinitis pigmentosa, juvenile |
| CRX | Retinitis pigmentosa, autosomal dominant |
| MT-TS2 | Retinitis pigmentosa, mitochondrial |
| RLBP1 | Retinitis pigmentosa, Bothnia retinal dystrophy, Newfoundland rod-cone dystrophy, Pigmentary retinal dystrophy |
| WDR19 | Retinitis pigmentosa, autosomal recessive |

Retinitis pigmentosa may be autosomal dominant, autosomal recessive, X-linked or mitochondrially acquired.

Retinitis pigmentosa (RP) may be RP-1, RP-2, RP-3, RP-4, RP-6, RP-7, RP-9, RP-10, RP-11, RP-12, RP-13, RP-14, RP-15, RP-17, RP-18, RP-19, RP-20, RP-22,RP-23, RP-24, RP-25, RP-26, RP-27, RP-28, RP-30, RP-31, RP-32, RP-33, RP-34, RP-35, RP-36, RP-37, RP-38, RP-39, RP-40, RP-41, RP-42, RP-43, RP-44, RP-45, RP-46, RP-47, RP-48, RP-49, RP-50, RP-51, RP-53, RP-54, RP-55, RP-56, RP-57, RP-58, RP-59, RP-60, RP-61, RP-62, RP-63, RP-64, RP-66, RP-67, RP-68, RP-69 or RP-70.

The subject having retinitis pigmentosa may have a mutation in one or more of the following genes: RP1, RP2, RPGR, RHO, ROM1, RP9, IMPDH1, PRPF31, CRB1, PRPF8, TULP1, CA4, PRPF3, ABCA4, RPE65, OFD1, EYS, CERKL, NRL, FAM161A, FSCN2, TOPORS, SNRNP200, SEMA4A, PRCD, NR2E3, MERTK, USH2A, PDE6B, PROM1, KLHL7, PDE6A, RGR,CNGB1, IDH3B, SAG, GUCA1B, CNGA1, BEST1, TTC8, RDH12, C2orf71, ARL6, IMPG2, PDE6G, ZNF513, DHDDS, PRPF6, CLRN1, MAK, C8ORF37, RBP3, NEK2, SLC7A14, KIZ, PRPF4, PRPH2, LRAT, SPATA7, AIPL1, CRX, MT-TS2, RLBP1 and WDR19.

### Therapeutic or Prophylactic Uses

The Pyrazolopyridazine compounds of Formula II can be administered to a subject as a component of a composition that comprises a pharmaceutically acceptable carrier or vehicle. Non-limiting examples of suitable pharmaceutical carriers or vehicles include starch, glucose, lactose, sucrose, gelatin, malt, rice, flour, chalk, silica gel, magnesium carbonate, magnesium stearate, sodium stearate, glycerol monostearate, talc, sodium chloride, dried skim milk, glycerol, propylene, glycol, water, ethanol, buffered water, and phosphate buffered saline. These compositions can be administered as, for example, drops, solutions, suspensions, tablets, pills, capsules, powders, and sustained-release formulations. In some embodiments, the compositions comprise, for example, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate, and mineral oil. The compositions can additionally comprise lubricating agents, wetting agents, emulsifying and suspending agents, preserving agents, sweetening agents or flavoring agents.

The compositions can comprise an effective amount of the Pyrazolopyridazine compound of Formula II. The compositions can be formulated in a unit dosage form that comprises an effective amount of the Pyrazolopyridazine compound of Formula II. In some embodiments, the compositions comprise, for example, from about 1 ng to about 1,000 mg of a Pyrazolopyridazine compound of Formula II. In some embodiments, the compositions comprise from about 100 mg to about 1,000 mg of a Pyrazolopyridazine compound of Formula II. In some embodiments, the compositions comprise from about 100 mg to about 500 mg of a Pyrazolopyridazine compound of Formula II. In some embodiments, the compositions comprise from about 200 mg to about 300 mg of a Pyrazolopyridazine compound of Formula II .

The dosage of a Pyrazolopyridazine compound of Formula II can vary depending on the symptoms, age, and body weight of the subject, the nature and severity of the proteopathy, the route of administration, and the form of the composition. The compositions described herein can be administered in a single dose or in divided doses. In some embodiments, the dosage of a Pyrazolopyridazine compound of Formula II ranges from about 0.01 ng to about 10 g per kg body mass of the subject, from about 1 ng to about 0.1 g per kg, or from about 100 ng to about 10 mg per kg.

Administration can be, for example, topical, intraaural, intraocular, parenteral, intravenous, intra-arterial, subcutaneous, intramuscular, intracranial, intraorbital, intraventricular, intracapsular, intraspinal, intracisternal, intraperitoneal, intranasal, aerosol, suppository, or oral. Formulations for oral use include tablets containing a Pyrazolopyridazine compound of Formula II in a mixture with non-toxic pharmaceutically acceptable excipients. These excipients can be, for example, inert diluents or fillers (e.g., sucrose and sorbitol), lubricating agents, glidants, and antiadhesives (e.g., magnesium stearate, zinc stearate, stearic acid, silicas, hydrogenated vegetable oils, or talc). Formulations for ocular use can be in the form of eyedrops.

A Pyrazolopyridazine compound of Formula II, or compositions thereof, can be provided in lyophilized form for reconstituting, for instance, in isotonic, aqueous, or saline buffers for parental, subcutaneous, intradermal, intramuscular, or intravenous administration. A composition can also be in the form of a liquid preparation useful for oral, intraaural, nasal, or sublingual administration, such as a suspension, syrup or elixir. A composition can also be in a form suitable for oral administration, such as a capsule, tablet, pill, and chewable solid formulation. A composition can also be prepared as a cream for dermal administration as a liquid, a viscous liquid, a paste, or a powder. A composition can also be prepared as a powder for pulmonary administration with or without an aerosolizing component.

The compositions can be in oral, intraaural, intranasal, sublingual, intraduodenal, subcutaneous, buccal, intracolonic, rectal, vaginal, mucosal, pulmonary, transdermal, intradermal, parenteral, intravenous, intramuscular and ocular dosage forms as well as being able to traverse the blood-brain barrier.

The compositions can be administered by various means known in the art. For example, the compositions can be administered orally, and can be formulated as tablets, capsules, granules, powders or syrups. Alternatively, compositions can be administered parenterally as injections (for example, intravenous, intramuscular or subcutaneous), drop infusion preparations or suppositories. For ophthalmic application compositions can be formulated as eye drops or eye ointments. Aural compositions can be formulated as ear drops, ointments, creams, liquids, gels, or salves for application to the ear, either internally or superficially. These formulations can be prepared by conventional means, and the compositions can be mixed with any conventional additive, such as an excipient, a binder, a disintegrating agent, a lubricant, a solubilizing agent, a suspension aid, an emulsifying agent, or a coating agent.

Compositions can include wetting agents, emulsifiers, and lubricants, coloring agents, release agents, coating agents, sweetening, flavoring and perfuming agents, preservatives and antioxidants.

Compositions can be suitable, for example, for oral, intraaural, intraocular, nasal, topical (including buccal and sublingual), rectal, vaginal, aerosol and/or parenteral administration. The compositions can be provided in a unit dosage form, and can be prepared by any methods known in the art.

Formulations suitable for oral administration may be in the form of capsules, cachets, pills, tablets, lozenges, powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia. Compositions can also be administered as a bolus, electuary, or paste.

Additional examples of pharmaceutically acceptable carriers or vehicles include: (1) fillers or extenders, such as starches, lactose, sucrose, glucose, mannitol, and/or silicic acid; (2) binders, such as carboxymethyl cellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as acetyl alcohol and glycerol monostearate; (8) absorbents, such as kaolin and bentonite clay; (9) lubricants, such as talc, calcium stearate, magnesium stearate, solid polyethylene glycols, sodium lauryl sulfate, and mixtures thereof; (10) coloring agents; and (11) buffering agents. Similar compositions can be employed as fillers in soft- or hard-filled gelatin capsules.

Liquid dosage forms for oral administration include pharmaceutically acceptable emulsions, microemulsions, gels, solutions, suspensions, syrups and elixirs. The liquid dosage form can contain inert diluents commonly used in the art, for example, water or other solvents, solubilizing agents and emulsifiers, such as ethyl alcohol, isopropyl alcohol, diethyl carbonate, ethyl acetate, benzyl alcohol, benzyl benzoate, propylene glycol, 1,3-butylene glycol, oils such as, cottonseed, groundnut, corn, germ, olive, castor and sesame oils, glycerol, tetrahydrofuryl alcohol, polyethylene glycols, fatty acid esters of sorbitan, and mixtures thereof.

Suspension dosage forms can contain suspending, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

The dosage forms for transdermal administration of a subject composition include drops, powders, sprays, ointments, pastes, creams, lotions, gels, solutions, and patches. The ointments, pastes, creams, and gels can contain excipients, such as animal and vegetable fats, oils, waxes, paraffin, starch, tragacanth, cellulose derivatives, polyethylene glycols, silicones, bentonite, silicic acid, talc and zinc oxide, or mixtures thereof.

Powders and sprays can contain excipients such as lactose, talc, silicic acid, aluminum hydroxide, calcium silicates, polyamide powder, or mixtures thereof. Sprays may additionally contain customary propellants, such as chlorofluorohydrocarbons and volatile unsubstituted hydrocarbons, such as butane and propane.

Compositions can be administered by aerosol of solid particles. A non-aqueous (e.g., fluorocarbon propellant) suspension could be used. Sonic nebulizers can be used because they minimize exposure to shear, which might cause degradation.

An aqueous aerosol can be made by formulating an aqueous solution or suspension of a Pyrazolopyridazine compound of Formula II with any conventional pharmaceutically acceptable carriers or vehicles such non-ionic surfactants (Tweens, Pluronics, or polyethylene glycol); proteins such as serum albumin; sorbitan esters; fatty acids; lecithin; amino acids; buffers; salts; sugars; or sugar alcohols.

Compositions suitable for parenteral administration comprise a Pyrazolopyridazine compound of Formula II and one or more pharmaceutically acceptable sterile isotonic aqueous or non-aqueous solutions, dispersions, suspensions, or emulsions, or sterile powders which can be reconstituted into sterile injectable solutions or dispersions just prior to use, which can contain antioxidants, buffers, bacteriostats, or solutes, which render the formulation isotonic with the blood of the subject, and suspending or thickening agents.

## Claims

1. A compound of Formula II for use in a method for treating or preventing a proteopathy, comprising administering to a subject in need thereof an effective amount of the compound of Formula II: 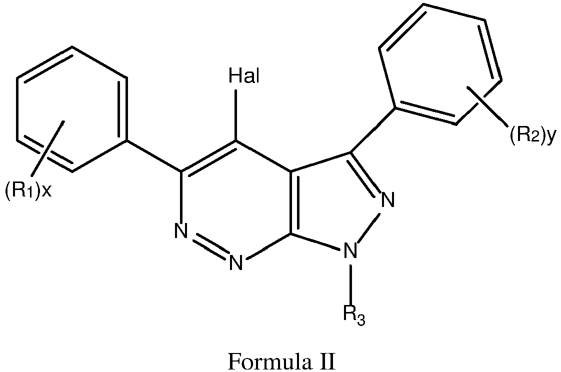
or a pharmaceutically acceptable salt thereof,
wherein Hal is -Cl, -F, -I, or -Br;
x is an integer ranging from 0 to 5;
each R₁ is independently -Cl, -F, -I, -Br, -C₁-C₃ alkyl, -O- C₁-C₃ alkyl, -CN, -CF₃, -C(O)NH(CH₃), or -C≡CCH₂OH;
y is an integer ranging from 0 to 5;
each R₂ is independently -Cl, -F, -Br, -C₁-C₃ alkyl, -O- C₁-C₃ alkyl, -CN, -CF₃, -C(O)NH(CH₃), or -C≡CCH₂OH;
R₃ is -H, -C₁-C₆ alkyl, -(C₁-C₆ alkylene)-OH, -(C₁-C₆ alkylene)-phenyl, -(C₁-C₆ alkylene)-O-(C₁-C₆ alkyl), -C₂-C₆ alkenyl, -(C₁-C₆ alkylene)-C(O)R₄, -(C₁-C₆ alkylene)-R₅, 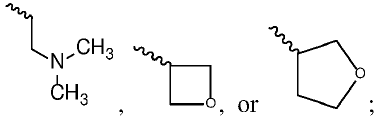
R₄ is -OH, -O-(C₁-C₆ alkyl), -NH₂, -NH(C₁-C₆ alkyl), -NH((C₁-C₆ alkylene)-OH), -NH((C₁-C₆ alkylene)N(C₁-C₆ alkyl)₂), -N(C₁-C₆ alkyl)((C₁-C₆ alkylene)-CN), -N(C₁-C₆ alkyl)((C₁-C₆ alkylene)N(C₁-C₆ alkyl)₂), -NH(C₁-C₆ alkylene)-O-(C₁-C₆ alkyl), 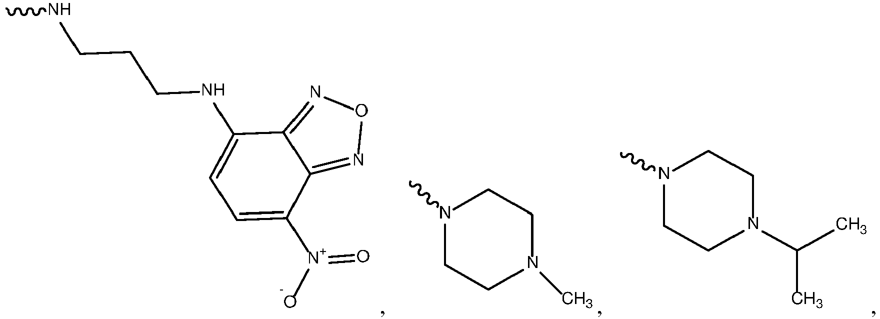 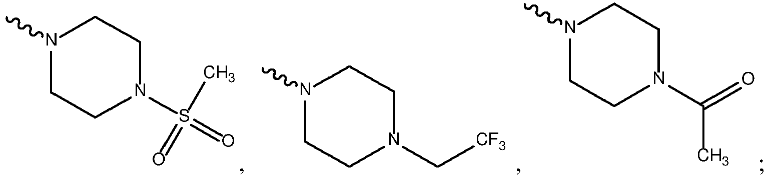 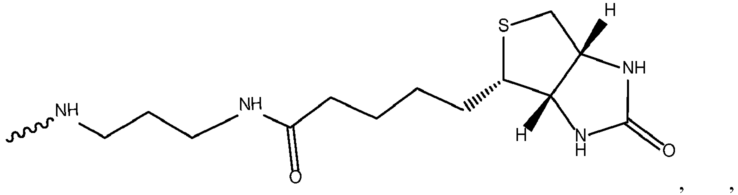 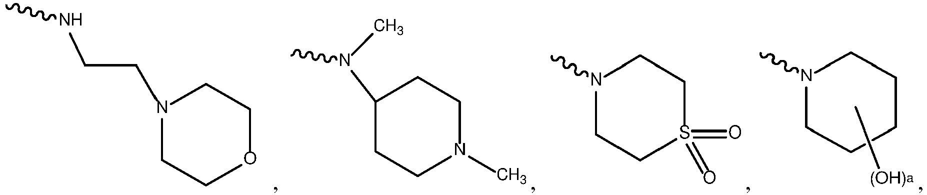 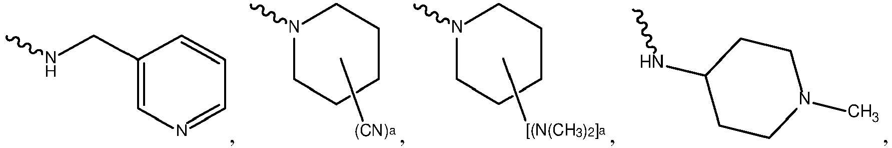 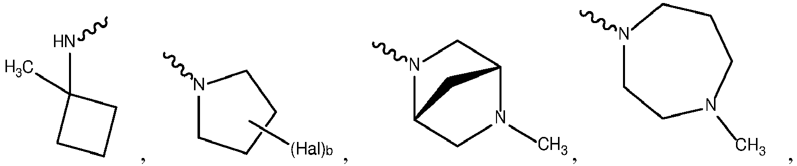 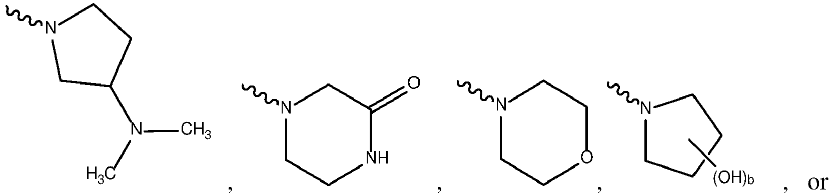 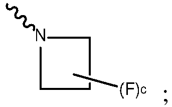
a is an integer ranging from 0 to 10;
b is an integer ranging from 0 to 8;
c is an integer ranging from 0 to 6; and
R₅ is 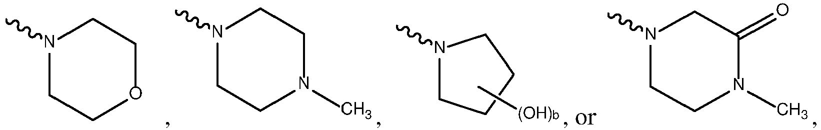 wherein the proteopathy is not retinitis pigmentosa, Leber's congenital amaurosis, a syndromic retinal degeneration, age-related macular degeneration, Usher syndrome or a retinal degenerative disease.

2. The compound for use of claim 1, wherein the compound has the structure: 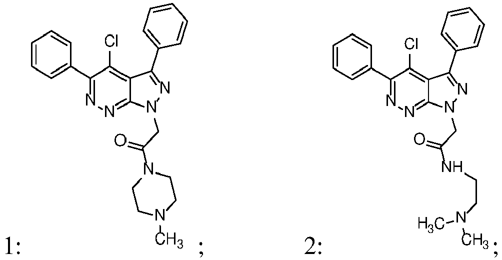 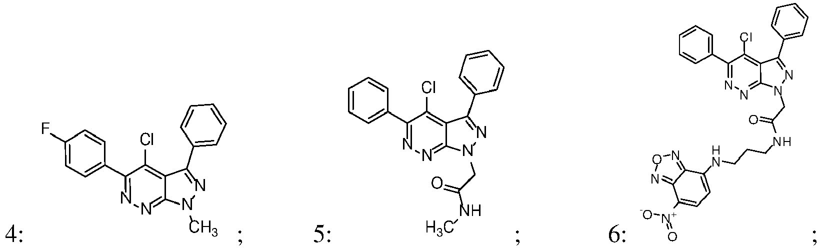 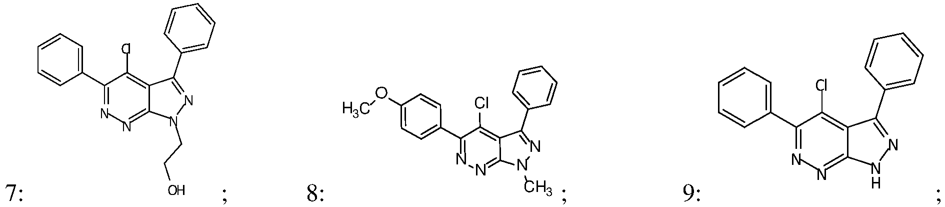 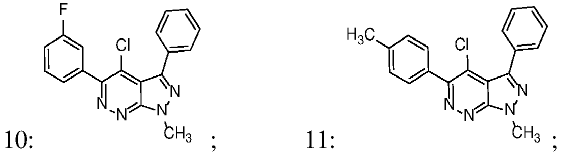 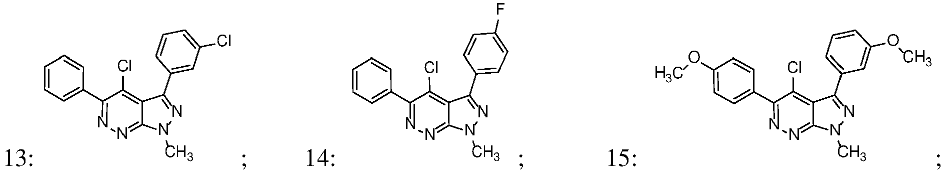 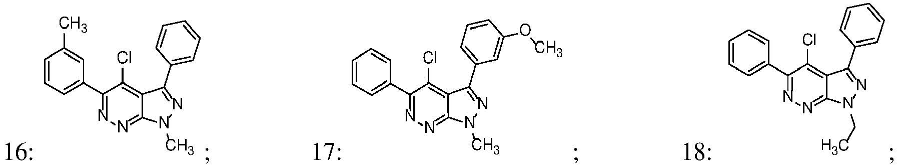 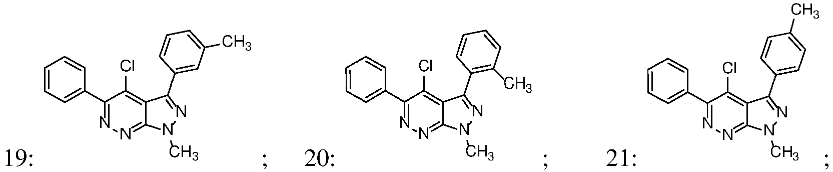 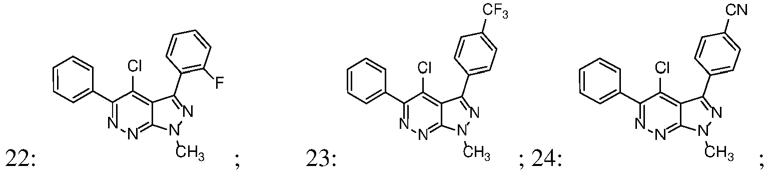 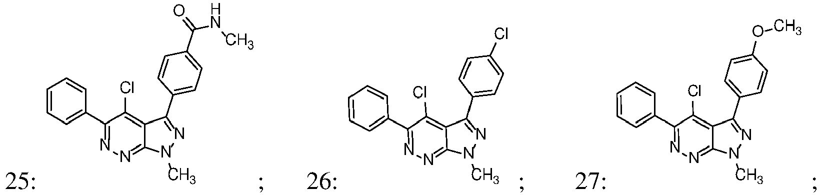 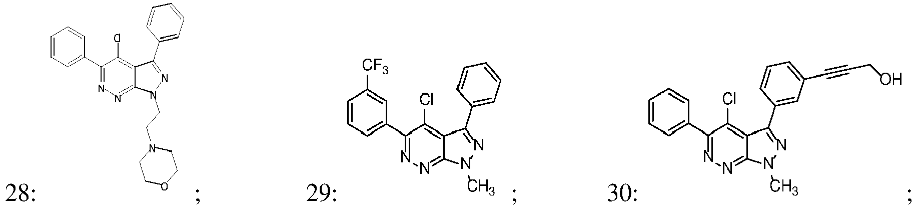 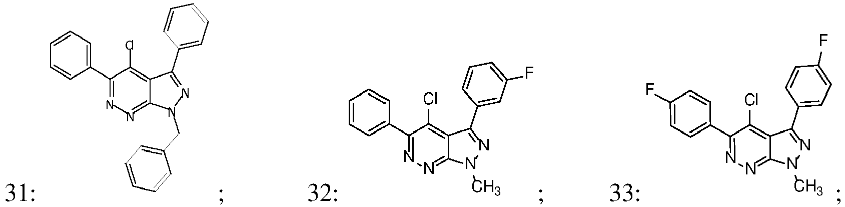 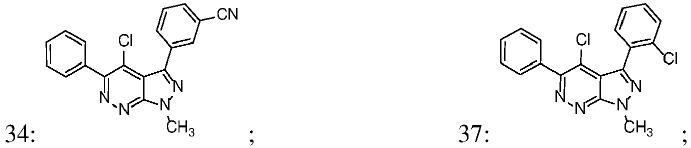 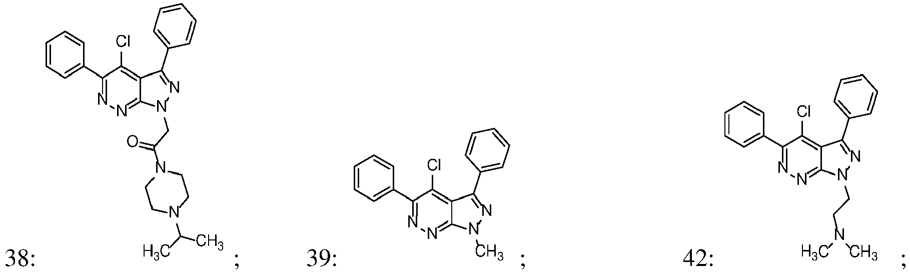 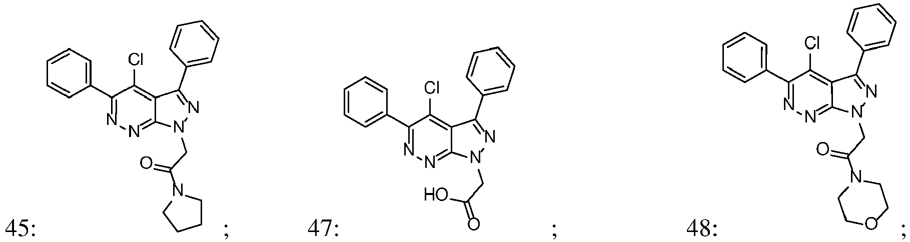 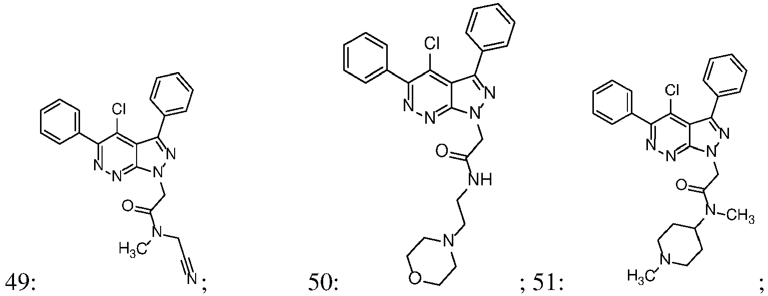 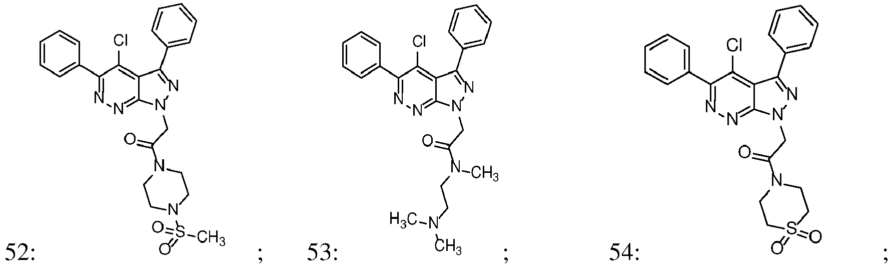 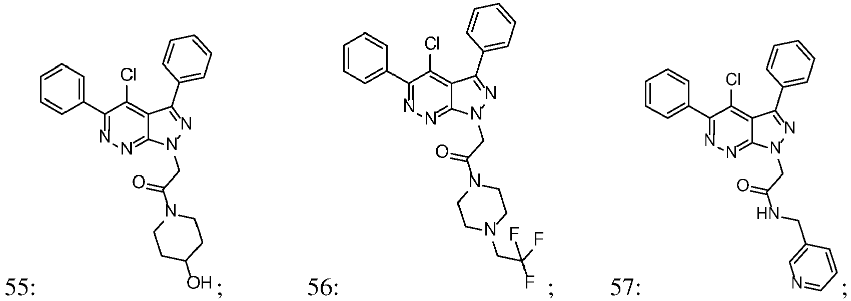 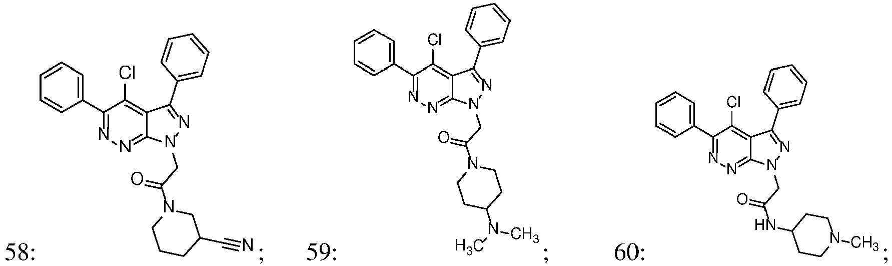 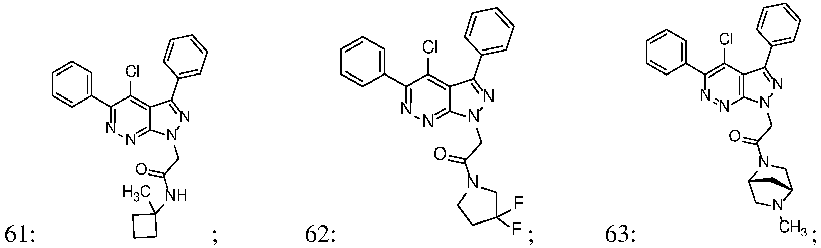 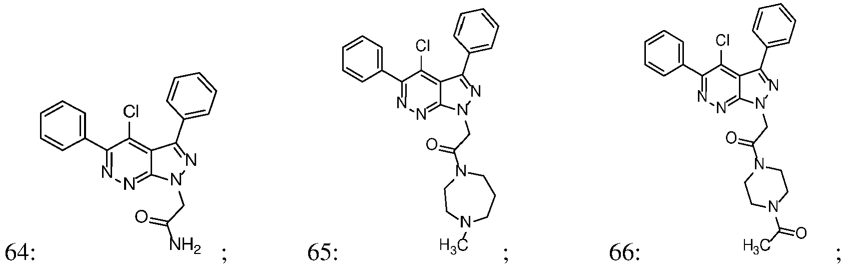 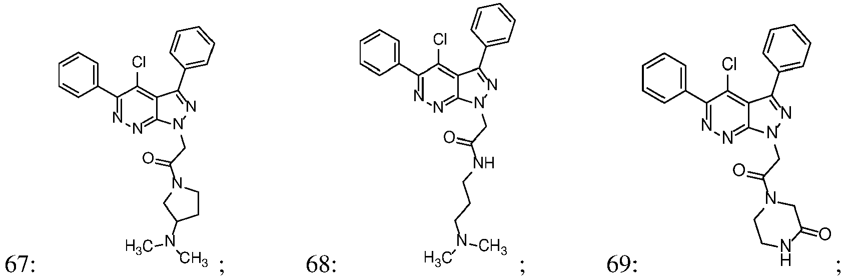 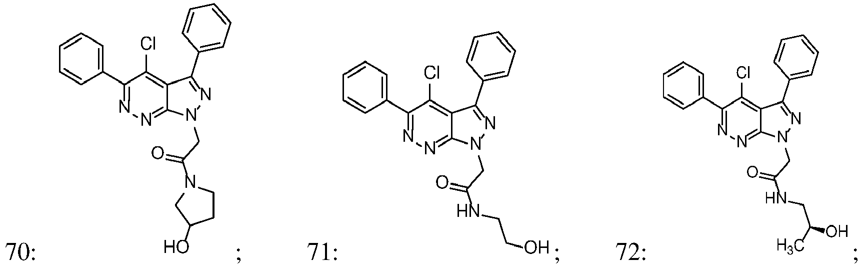 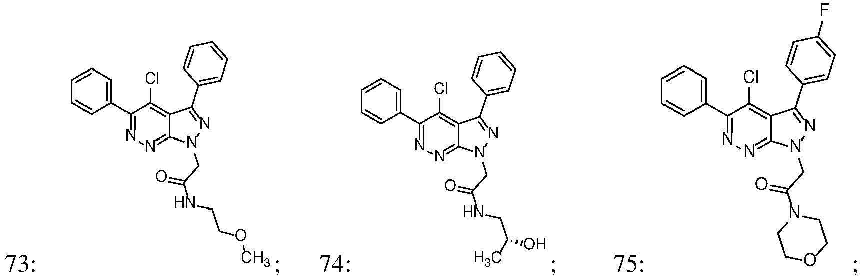 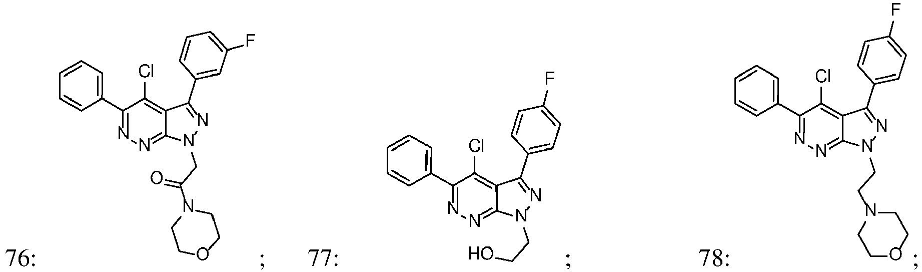 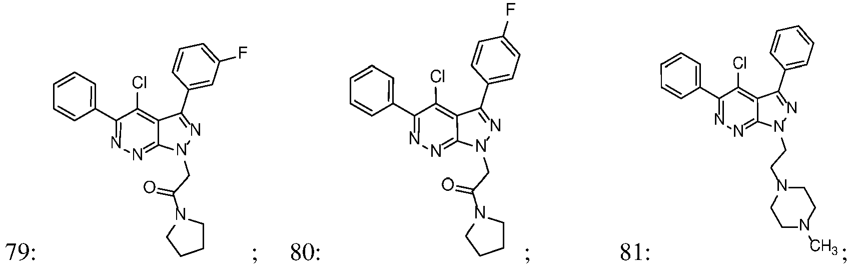 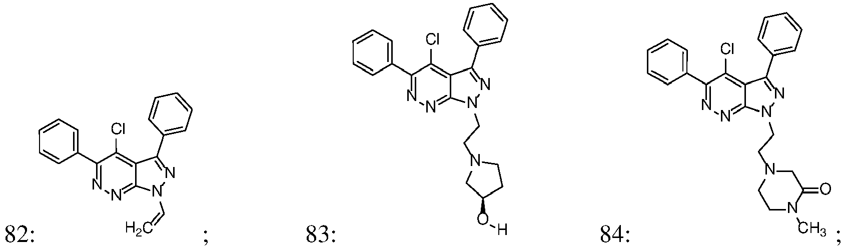 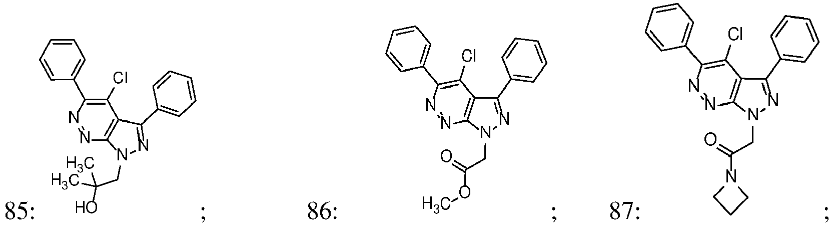 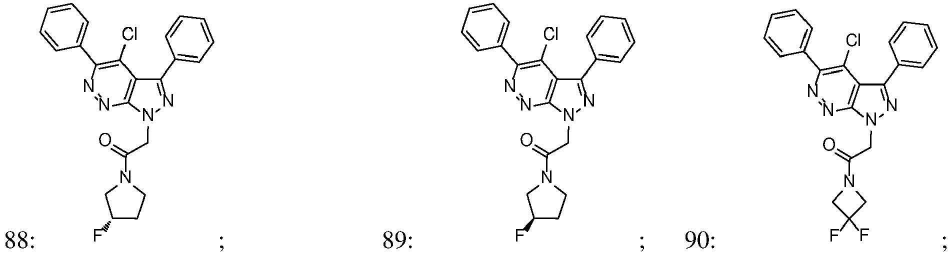 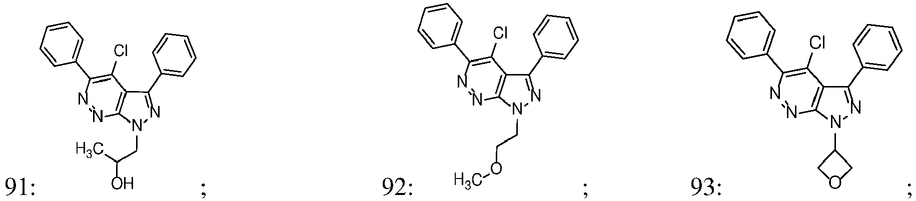 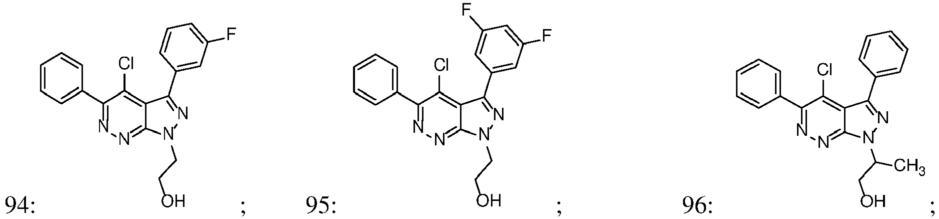 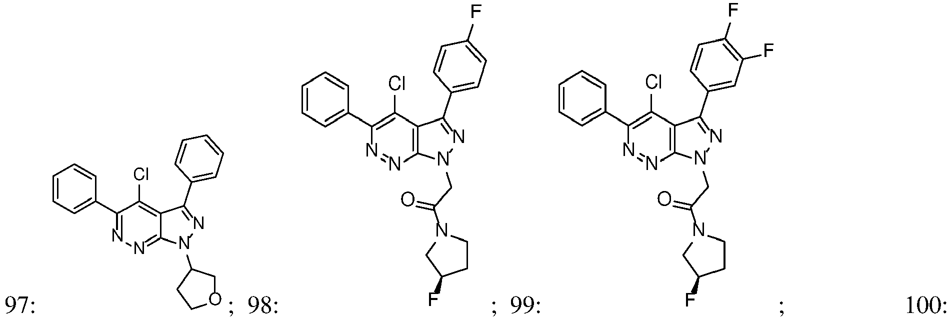 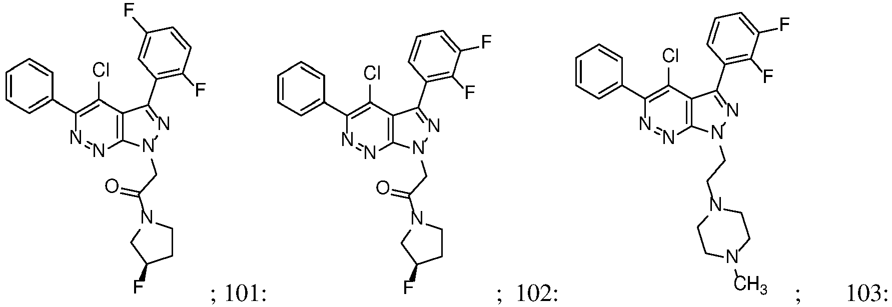 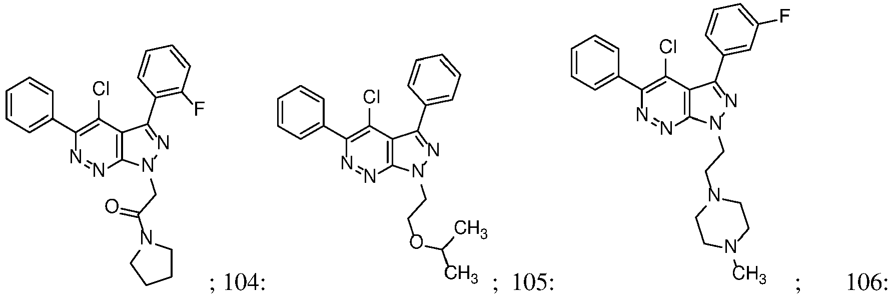 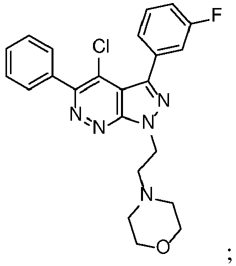 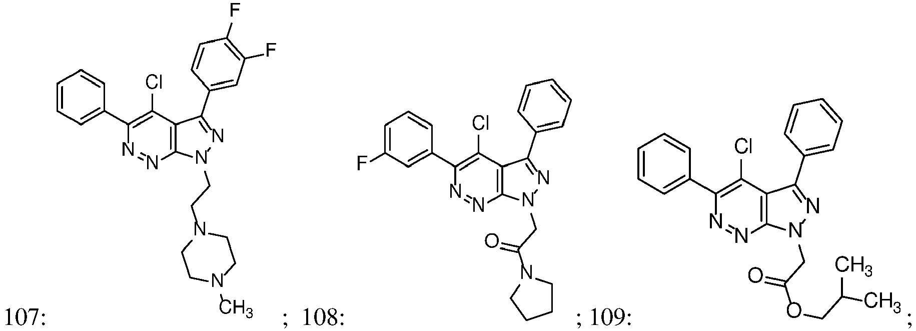 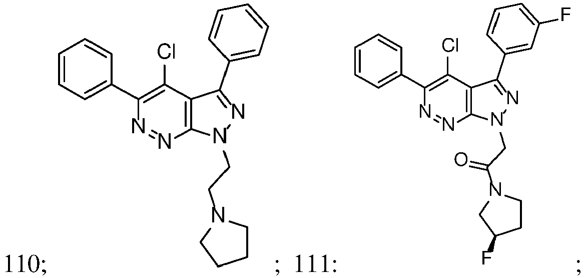 or a pharmaceutically acceptable salt thereof.

3. The compound for use of any one of claims 1-2, wherein the proteopathy is a neurodegenerative disease.

4. The compound for use of claim 3, wherein the neurodegenerative disease is Alzheimer's disease, progressive supranuclear palsy, dementia pugilistica, frontotemporal dementia and parkinsonism linked to chromosome 17 (FTDP-17), Lytico-Bodig disease, tangle-predominant dementia, ganglioma, gangliocytoma, meningioangiomatosis, subacute sclerosing panencephalitis, lead encephalopathy, tuberous sclerosis, Hallervorden-Spatz disease, lipofuscinosis, Pick's disease, corticobasal degeneration, argyrophilic grain disease, Huntington's disease, Parkinson's disease, dementia with Lewy bodies, multiple system atrophy, neuroaxonal dystrophies, dentatorubralpallidoluysian atrophy (DRPLA), spinal-bulbar muscular atrophy (SBMA), spinocerebellar ataxia 1 (SCA 1), SCA 2, SCA 3, SCA 6, SCA 7, SCA 17, prion disease, amyotrophic lateral sclerosis, frontotemporal lobar degeneration (FTLD) or familial encephalopathy with neuroserpin inclusion bodies (FENIB).

5. The compound for use of any one of claims 1-2, wherein the proteopathy is an amyloidosis.

6. The compound for use of claim 5, wherein the amyloidosis is familial British dementia (ABri), familial Danish dementia (ADan), hereditary cerebral haemorrhage with amyloidosis-Icelandic (HCHWA-I), familial amyloidotic neuropathy (ATTR), AL (light chain) primary systemic amyloidosis, AH (heavy chain) amyloidosis, AA secondary amyloidosis, Aβ amyloidosis, aortic medial amyloidosis, LECT2 amyloidosis, AIAPP amyloidosis, apolipoprotein AI amyloidosis (AApoAI), apolipoprotein All amyloidosis (AApoAII), apolipoprotein AIV amyloidosis (AApoAIV), familial amyloidosis of the Finnish type (FAF), fibrinogen amyloidosis (AFib), lysozyme amyloidosis (ALys), dialysis amyloidosis (Aβ₂M), medullary thyroid carcinoma (ACal), cardiac atrial amyloidosis (AANF), pituitary prolactinoma (APro), hereditary lattice corneal dystrophy, cutaneous lichen amyloidosis (AKer), Mallory bodies, primary cutaneous amyloidosis, corneal lactoferrin amyloidosis, odontogenic (Pindborg) tumor amyloid or seminal vesicle amyloid.

7. The compound for use of any one of claims 1-2, wherein the proteopathy is a lysosomal storage disease.

8. The compound for use of claim 7, wherein the lysosomal storage disease is a mucopolysaccharidosis disorder.

9. The compound for use of claim 8, wherein the mucopolysaccharidosis disorder is Pseudo-Hurler polydystrophy/Mucolipidosis IIIA, MPS I Hurler Syndrome, MPS I Scheie Syndrome, MPS I Hurler-Scheie Syndrome, MPS II Hunter syndrome, Sanfilippo syndrome Type A/MPS III A, Sanfilippo syndrome Type B/MPS III B, Sanfilippo syndrome Type C/MPS III C, Sanfilippo syndrome Type D/MPS III D, Morquio Type A/MPS IVA, Morquio Type B/MPS IVB, MPS IX Hyaluronidase Deficiency, MPS VI Maroteaux-Lamy, MPS VII Sly Syndrome, Mucolipidosis I/Sialidosis, Mucolipidosis IIIC or Mucolipidosis type IV.

10. The compound for use of claim 7, wherein the lysosomal storage disease is Pompe disease/glycogen storage disease type II.

11. The compound for use of claim 7, wherein the lysosomal storage disease is activator deficiency/GM2 gangliosidosis, alpha-mannosidosis, aspartylglucosaminuria, cholesteryl ester storage disease,chronic Hexosaminidase A Deficiency, cystinosis, Danon disease, Fabry disease, Farber disease, fucosidosis, galactosialidosis, Gaucher Disease Type I, Gaucher Disease Type II, Gaucher Disease Type III, GM1 gangliosidosis infantile, GM1 gangliosidosis late infantile/uvenile GM1 gangliosidosis adult/chronic, I-Cell disease/Mucolipidosis II, Infantile Free Sialic Acid Storage Disease/ISSD, Juvenile Hexosaminidase A Deficiency, Krabbe disease infantile onset, Krabbe disease late onset, lysosomal acid lipase deficiency early onset, lysosomal acid lipase deficiency Late onset, Metachromatic Leukodystrophy, Pseudo-Hurler polydystrophy/Mucolipidosis IIIA, MPS I Hurler Syndrome, MPS I Scheie Syndrome, MPS I Hurler-Scheie Syndrome, MPS II Hunter syndrome, Sanfilippo syndrome Type A/MPS III A, Sanfilippo syndrome Type B/MPS III B, Sanfilippo syndrome Type C/MPS III C, Sanfilippo syndrome Type D/MPS III D, Morquio Type A/MPS IVA, Morquio Type B/MPS IVB, MPS IX Hyaluronidase Deficiency, MPS VI Maroteaux-Lamy, MPS VII Sly Syndrome, Mucolipidosis I/Sialidosis, Mucolipidosis IIIC, Mucolipidosis type IV, Multiple sulfatase deficiency, Niemann-Pick Disease Type A, Niemann-Pick Disease Type B, Niemann-Pick Disease Type C, CLN6 disease-atypical late infantile, CLN6 disease-late onset variant, CLN6 disease-early juvenile, Batten-Spielmeyer-Vogt/Juvenile NCL/CLN3 disease, Finnish Variant Late Infantile CLN5, Jansky-Bielschowsky disease/Late infantile CLN2/TPP1 Disease, Kufs/Adult-onset NCL/CLN4 disease, Northern Epilepsy/variant late infantile CLN8, Santavuori-Haltia/Infantile CLN1/PPT disease, Beta-mannosidosis, Pompe disease/glycogen storage disease type II, Pycnodysostosis, Sandhoff disease/Adult Onset/GM2 Gangliosidosis, Sandhoff disease/GM2 gangliosidosis - Infantile, Sandhoff disease/GM2 gangliosidosis - Juvenile, Schindler disease, Salla disease/Sialic Acid Storage Disease, Tay-Sachs/GM2 gangliosidosis or Wolman disease.

12. The compound for use of any one of claims 1-11, wherein the compound has the structure 85: 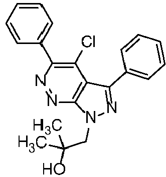 or a pharmaceutically acceptable salt thereof.

13. The compound for use of any one of claims 1-11, wherein the compound has the structure 85:

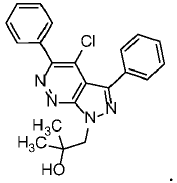

## Patentansprüche

1. Verbindung nach Formel II zur Verwendung in einem Verfahren zur Behandlung oder Verhütung einer Proteinfehlfaltungserkrankung, wobei im Rahmen desselben einem Individuum, welches dessen bedarf, eine wirksame Menge der Verbindung nach Formel II verabreicht wird: 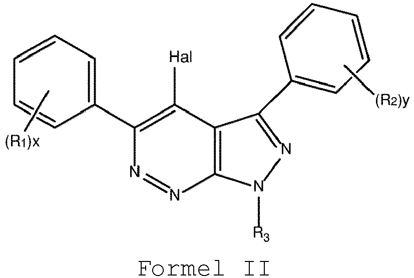
oder ein pharmazeutisch unbedenkliches Salz derselben, wobei Hal für -Cl, -F, -I oder -Br steht;
x eine ganze Zahl im Bereich von 0 bis 5 ist;
jedes R₁ auf unabhängige Weise für -Cl, -F, -I, -Br, -C₁-C₃-Alkyl, -O-C₁-C₃-Alkyl, -CN, -CF₃, -C(O)NH(CH₃) oder - C≡CCH₂OH steht;
y eine ganze Zahl im Bereich von 0 bis 5 ist;
jedes R₂ auf unabhängige Weise für -Cl, -F, -Br, -C₁-C₃-Alkyl, -O-C₁-C₃-Alkyl, -CN, -CF₃, C(O)NH(CH₃) oder - C≡CCH₂OH steht;
R₃ für -H, -C₁-C₆-Alkyl, -(C₁-C₆-Alkylen)-OH, -(C₁-C₆-Alkylen)-phenyl, -(C₁-C₆-Alkylen)-O-(C₁-C₆-alkyl), -C₂-C₆-Alkenyl, -(C₁-C₆-Alkylen)-C(O)R₄, -(C₁-C₆-Alkylen)-R₅, 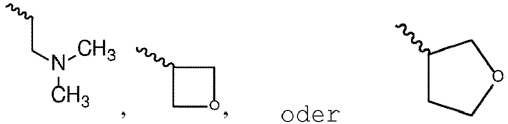 steht;
R₄ für -OH, -O-(C₁-C₆-Alkyl), -NH₂, -NH(C₁-C₆-Alkyl), - NH((C₁-C₆-Alkylen)-OH), -NH((C₁-C₆-Alkylen)N(C₁-C₆-alkyl)₂), -N(C₁-C₆-Alkyl)((C₁-C₆-alkylen)-CN), -N(C₁-C₆-Alkyl)((C₁-C₆-alkylen)N(C₁-C₆-alkyl)₂), -NH(C₁-C₆-Alkylen)-O-(C₁-C₆-alkyl), 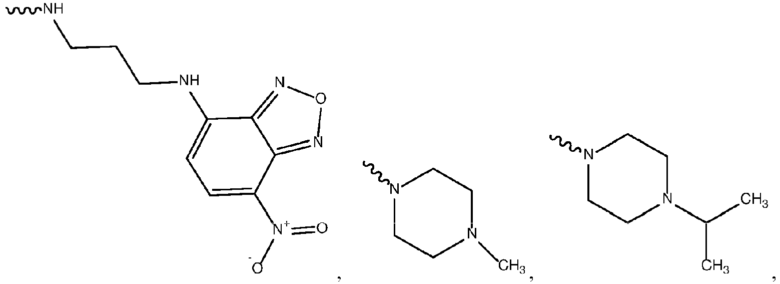 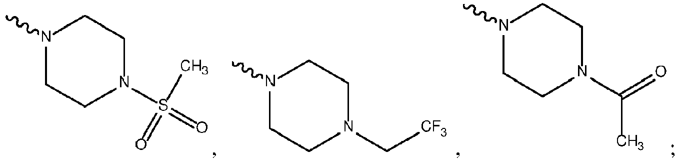 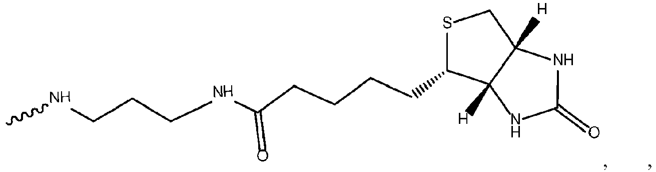 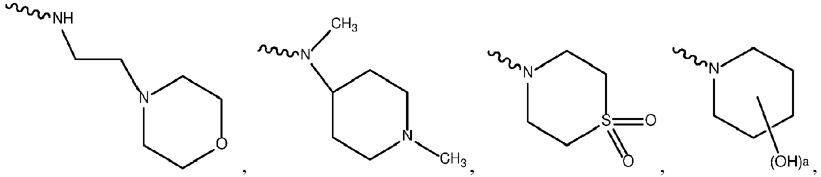 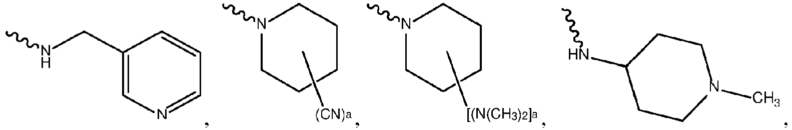 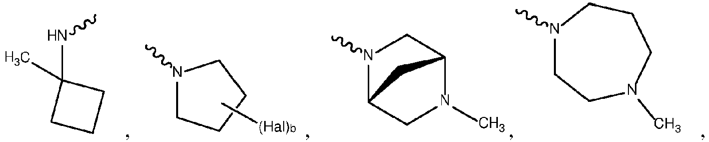 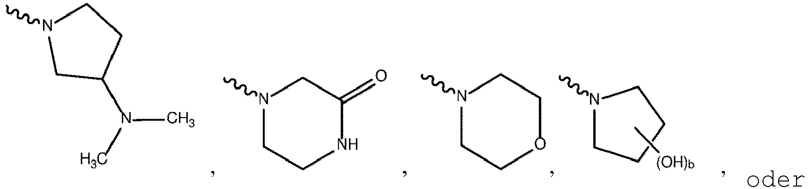 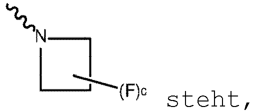
a eine ganze Zahl im Bereich von 0 bis 10 ist;
b eine ganze Zahl im Bereich von 0 bis 8 ist;
c eine ganze Zahl im Bereich von 0 bis 6 ist; und
R₅ für 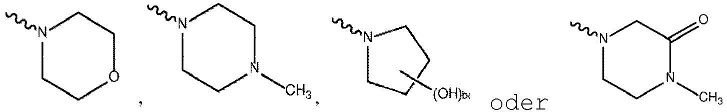 steht,
wobei es sich bei der Proteinfehlfaltungserkrankung nicht um Retinitis pigmentosa, die Lebersche Kongenitale Amaurose, eine syndromartige Entartung der Netzhaut, die altersbedingte Makuladegeneration, das Usher-Syndrom oder eine degenerative Erkrankung der Netzhaut handelt.

2. Verbindung zur Verwendung nach Anspruch 1, wobei die Verbindung die folgende Struktur aufweist: 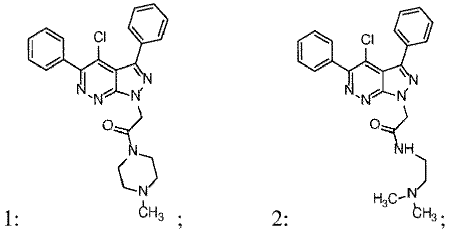 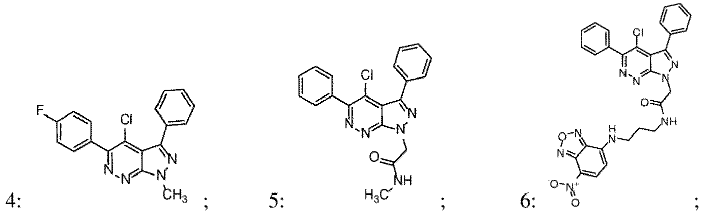 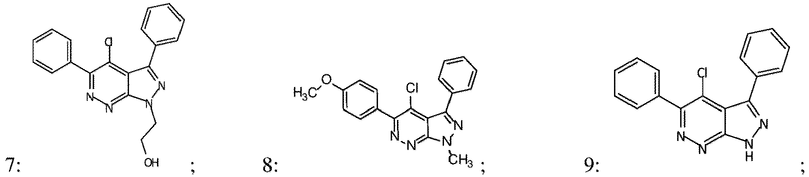 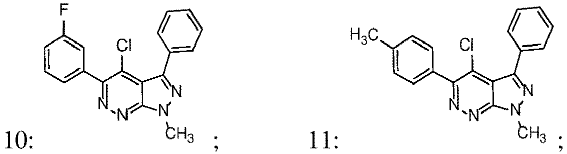 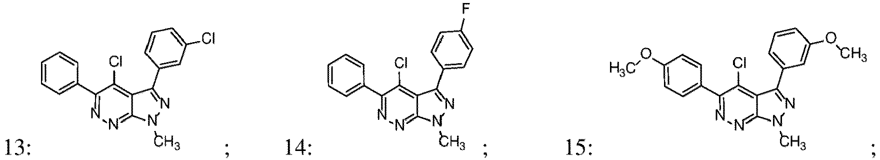 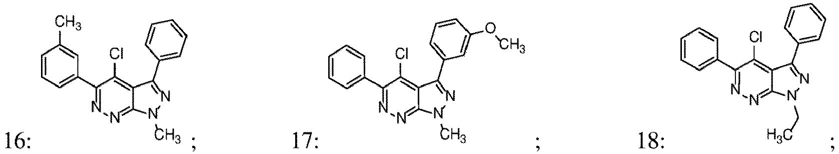 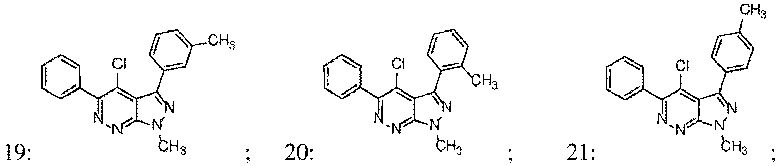 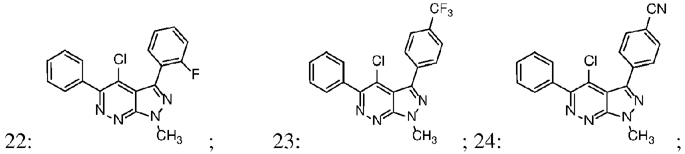 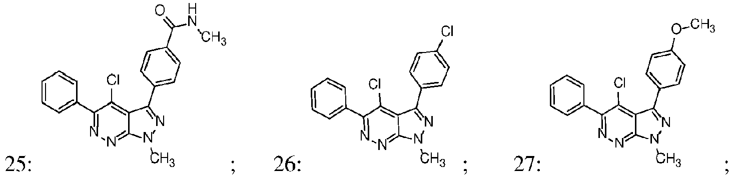 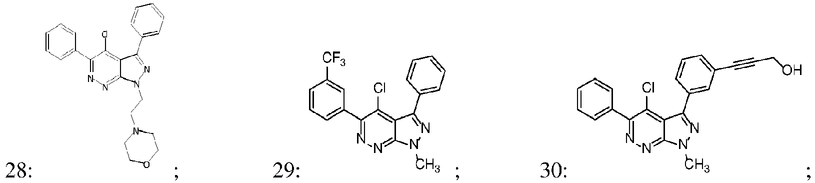 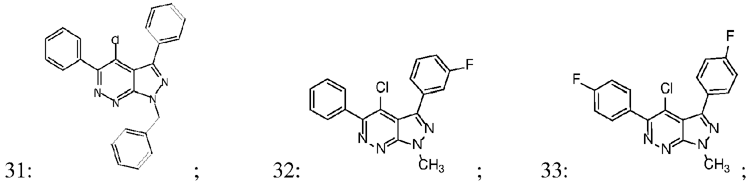 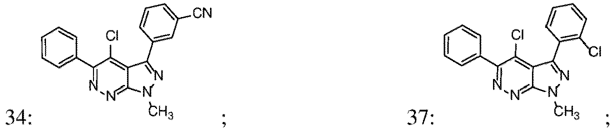 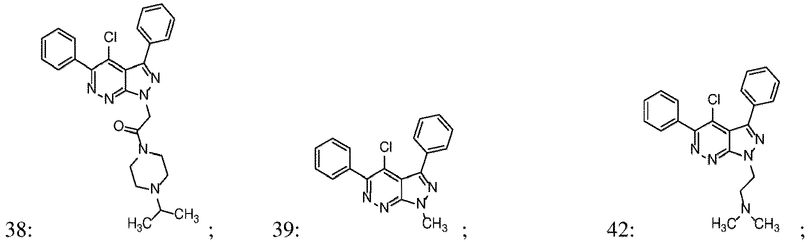 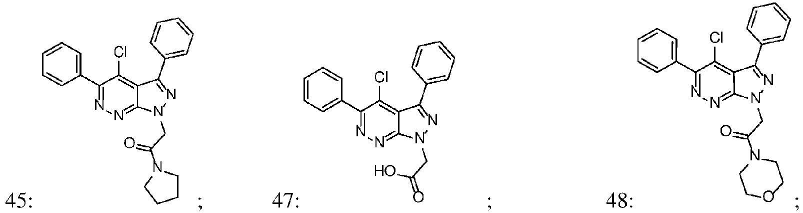 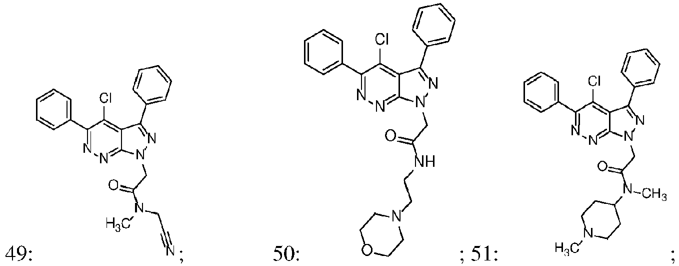 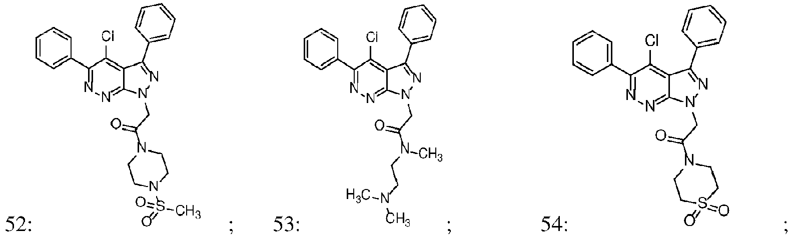 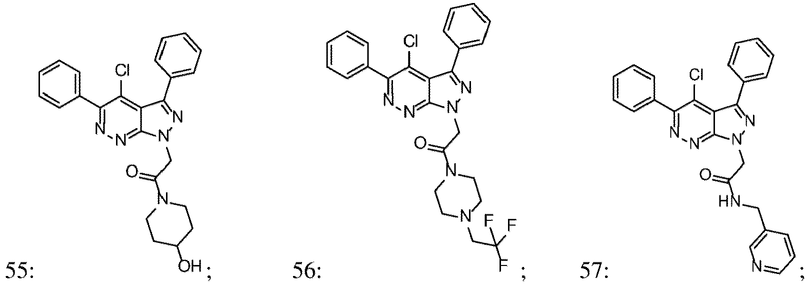 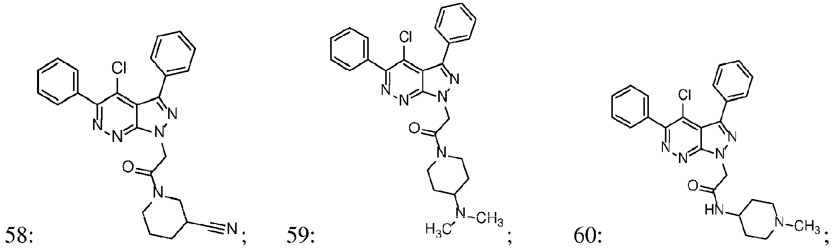 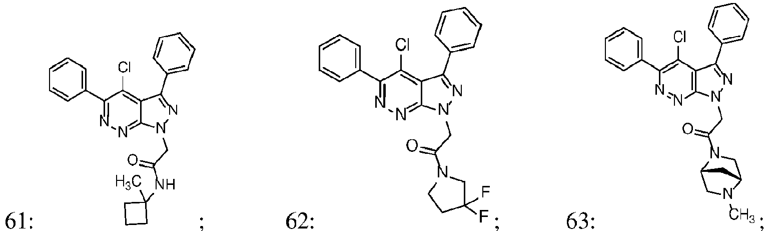 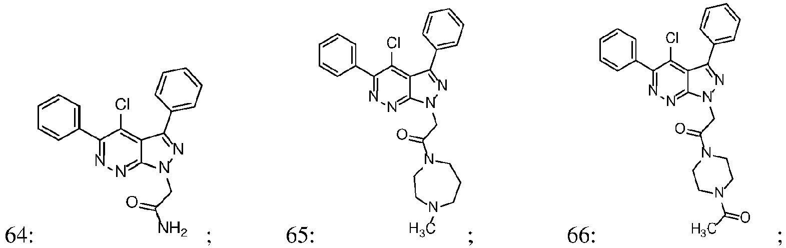 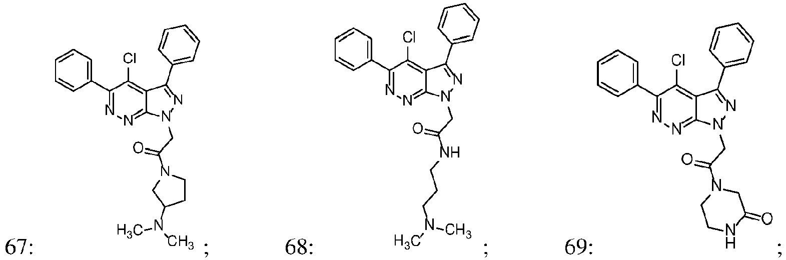 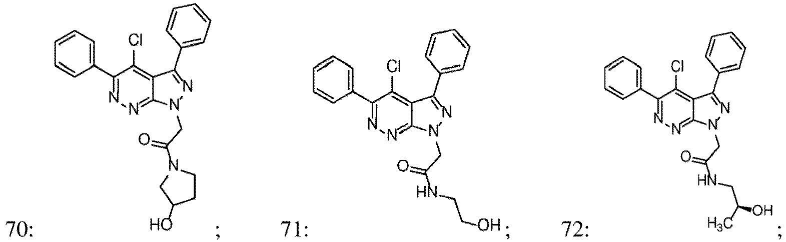 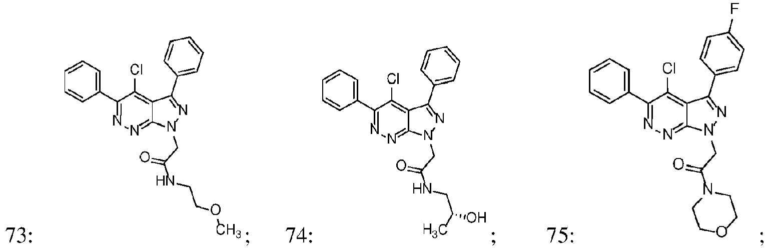 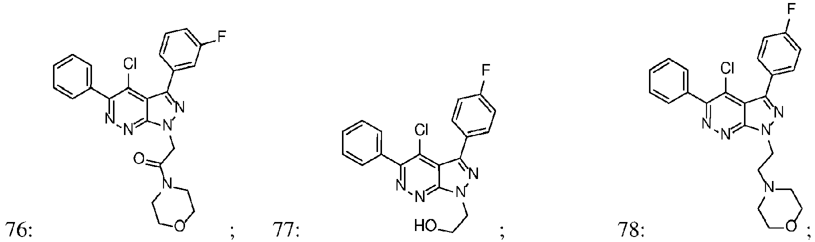 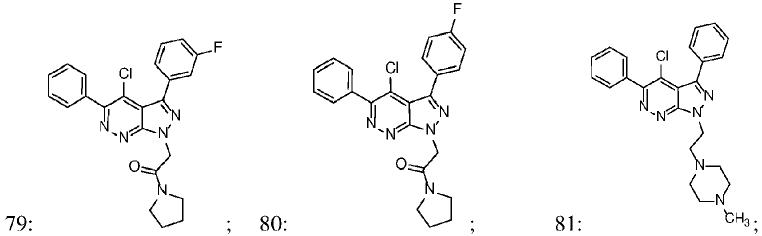 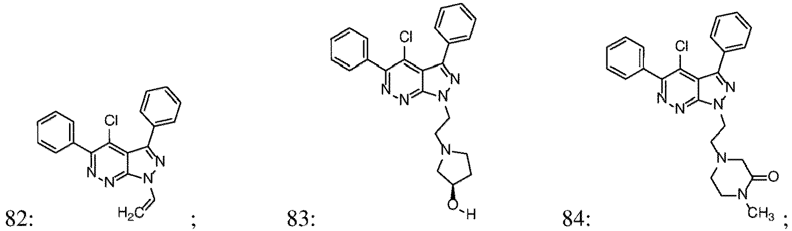 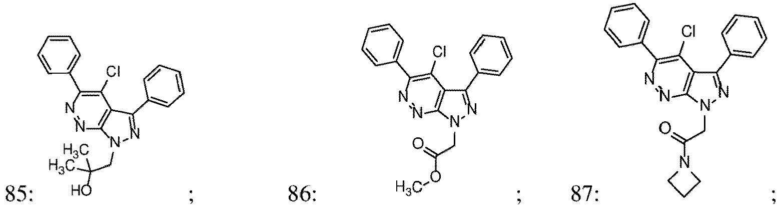 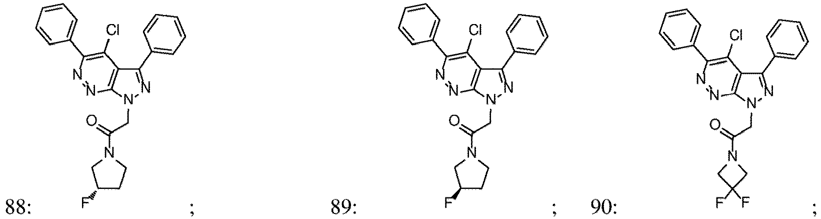 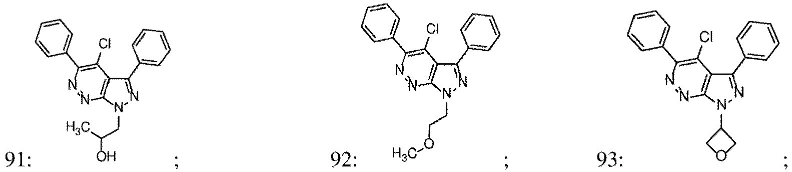 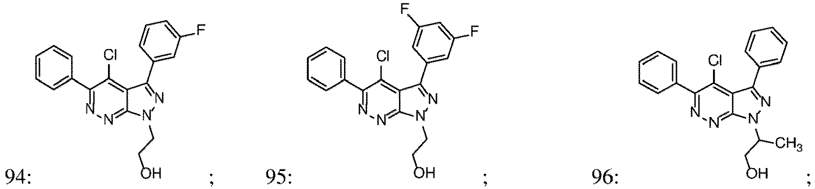 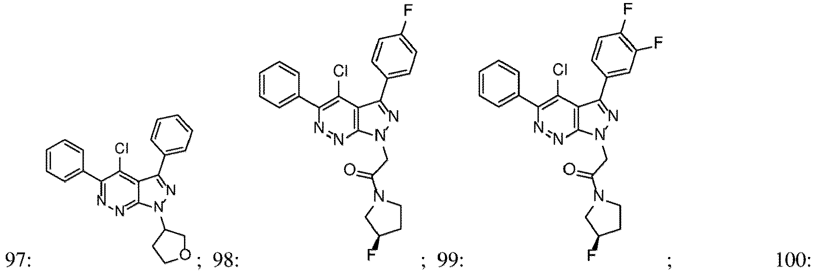 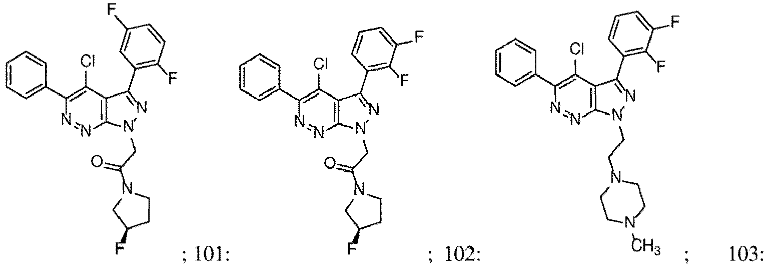 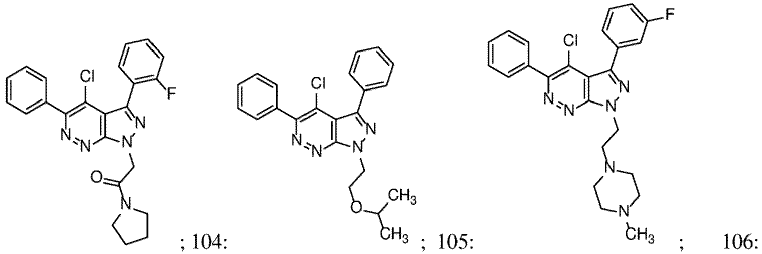 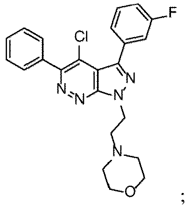 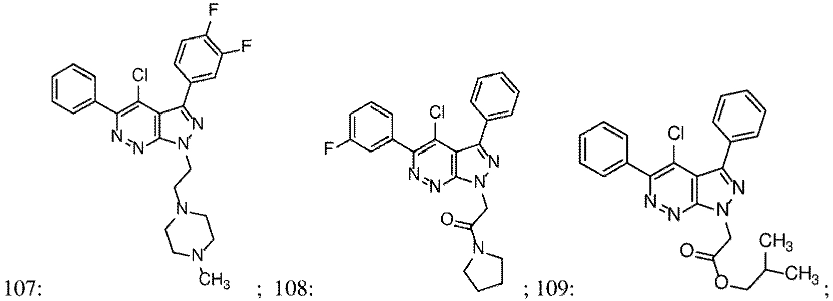 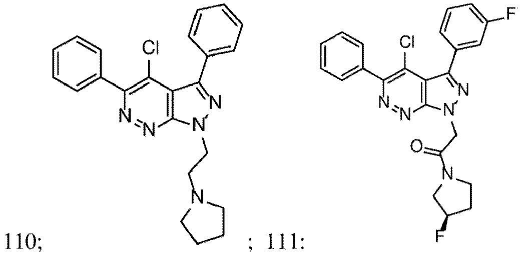 ; oder ein pharmazeutisch unbedenkliches Salz derselben.

3. Verbindung zur Verwendung nach einem beliebigen der Ansprüche 1 bis 2, wobei es sich bei der Proteinfehlfaltungserkrankung um eine neurodegenerative Erkrankung handelt.

4. Verbindung zur Verwendung nach Anspruch 3, wobei es sich bei der neurodegenerativen Erkrankung um die Alzheimer-Krankheit, die Progressive supranukleäre Blickparese, die Chronisch-traumatische Enzephalopathie, die Frontotemporale Demenz und Parkinson-Krankheit in Verbindung mit dem Chromosom 17 (FTDP-17), Lytico-Bodig, knäueldomininierte Demenz, ein Ganglioneurom, ein Gangliozytom, Meningioangiomatose, subakute sklerosierende Panenzephalitis, bleibedingte Enzephalopathie, tuberöse Sklerose, das Hallervorden-Spatz-Syndrom, Lipofuszinose, die Pick-Krankheit, Kortikobasale Degeneration, die Silberkornkrankheit, Chorea Huntington, die Parkinson-Krankheit, Lewy-Körper-Demenz, Multisystematrophie, neuroaxonale Dystrophien, Dentatorubro-Pallidoluysische Atrophie (DRPLA), Spinobulbäre Muskelatrophie Typ Kennedy (SBMA), Spinozerebelläre Ataxie 1 (SCA 1), SCA 2, SCA 3, SCA 6, SCA 7, SCA 17, Prionen-Krankheiten, Amyotrophe Lateralsklerose, Frontotemporale Lobärdegeneration (FTLD) oder familiäre Enzephalopathie mit Neuroserpin-Einschlusskörpern (FENIB) handelt.

5. Verbindung zur Verwendung nach einem beliebigen der Ansprüche 1 bis 2, wobei es sich bei der Proteinfehlfaltungserkrankung um eine Amyloidose handelt.

6. Verbindung zur Verwendung nach Anspruch 5, wobei es sich bei der Amyloidose um familiäre britische Demenz (ABri), familiäre dänische Demenz (ADan), erbliche zerebrale Hämorrhagie mit Amyloidose-isländisch (HCHWA-I),familiäre amyloidotische Neuropathie (ATTR), AL (Leichtkette) primäre systemische Amyloidose, AH (Schwerkette) Amyloidose, AA sekundäre Amyloidose, Aß-Amyloidose, mediale Amyloidose der Aorta, LECT2-Amyloidose, AIAPP-Amyloidose, Apolipoprotein-AI-Amyloidose, AIAPP-Amyloidose, Apolipoprotein-AI-Amyloidose (AApoAI), Apolipoprotein-AII-Amyloidose (AApoAII), Apolipoprotein-AIV-Amyloidose (AApoAIV), familiäre Amyloidose des finnischen Typs (FAF), Fibrinogen-Amyloidose (AFib), Lysozym-Amyloidose (ALys), Dialyse-Amyloidose (Aβ₂M), das medulläre Schilddrüsenkarzinom (ACal), kardiale atriale Amyloidose (AANF), das Prolaktinom der Hypophyse (APro), erbliche gittrige Hornhautdystrophie, flechtenartige Amyloidose der Haut (AKer), Mallory-Körper, primäre Amyloidose der Haut, Lactoferrin-Amyloidose der Hornhaut, ein Amyloid odontogener (Pindborg-) Tumore oder ein Amyloid der Samenvesikel handelt.

7. Verbindung zur Verwendung nach einem beliebigen der Ansprüche 1 bis 2, wobei es sich bei der Proteinfehlfaltungserkrankung um eine lysosomale Speicherkrankheit handelt.

8. Verbindung zur Verwendung nach Anspruch 7, wobei es sich bei der lysosomalen Speicherkrankheit um eine Mukopolysaccharidose-Störung handelt.

9. Verbindung zur Verbindung nach Anspruch 8, wobei es sich bei der Mukopolysaccharidose-Störung um Pseudo-Hurler-Polydystrophie / Mukolipidose IIIA, MPS I Morbus Hurler, MPS I Morbus Scheie, MPS I Morbus Hurler/Scheie, MPS II Hunter-Syndrom, das Sanfilippo-Syndrom vom Typ A/MPS III A, das Sanfilippo-Syndrom vom Typ B / MPS III B, das Sanfilippo-Syndrom vom Typ C / MPS III C, das Sanfilippo-Syndrom vom Typ D / MPS III D, Morbus Morquio vom Typ A / MPS IVA, Morbus Morquio vom Typ B / MPS IVB, MPS IX Hyaluronidase-Mangel, MPS VI Maroteaux-Lamy-Syndrom, MPS VII Sly-Syndrom, Mukolipidose I / Sialidose, Mukolipidose IIIC oder um Mukolipidose Typ IV handelt.

10. Verbindung zur Verwendung nach Anspruch 7, wobei es sich bei der lysosomalen Speicherkrankheit um Morbus Pompe / eine Glykogenspeicherkrankheit vom Typ II handelt.

11. Verbindung zur Verwendung nach Anspruch 7, wobei es sich bei der lysosomalen Speicherkrankheit um Aktivatormangel / GM2-Gangliosidose, alpha-Mannosidose, Aspartylglucosaminurie, die Cholesterinester-Speicherkrankheit, chronischen Hexosaminidase-A-Mangel, Cystinose, Morbus Danon, Morbus Fabry, das Farber-Syndrom, Fucosidose, Galactosialidose, Morbus Gaucher Typ I, Morbus Gaucher Typ II, Morbus Gaucher Typ III, GM1-Gangliosidose des Kindes, GM1-Gangliosidose der späten Kindheit/Jugend, GM1-Gangliosidose des Erwachsenen / chronischer Art, die I-Zellkrankheit / Mukolipidose II, die Speicherkrankheit freier Sialinsäure des Kindes / ISSD, juvenilen Hexosaminidase-A-Mangel, Morbus Krabbe mit Einsetzen in der Kindheit, spät einsetzenden Morbus Krabbe, früh einsetzenden Mangel an lysosomaler saurer Lipase, spät einsetzenden Mangel an lysosomaler saurer Lipase, metachromatische Leukodystrophie, Pseudo-Hurler-Polydystrophie / Mucolipidose IIIA, MPS I Morbus Hurler, MPS I Morbus Scheie, MPS I Morbus Hurler/Scheie, MPS II Hunter-Syndrom, Sanfilippo-Syndrom Typ A / MPS III A, Sanfilippo-Syndrom Typ B / MPS III B, Sanfilippo-Syndrom Typ C / MPS III C, Sanfilippo-Syndrom Typ D / MPS III D, Morbus Morquio Typ A / MPS WA, Morbus Morquio Typ B / MPS WB, MPS IX Hyaluronidase-Mangel, MPS VI Maroteaux-Lamy-Syndrom, MPS VII Sly-Syndrom, Mukolipidose I / Sialidose, Mukolipidose IIIC, Mukolipidose Typ IV, multiplen Sulfatase-Mangel, die Niemann-Pick Krankheit vom Typ A, die Niemann-Pick-Krankheit vom Typ B, die Niemann-Pick-Krankheit vom Typ C, die atypische CLN6-Krankkeit der späten Kindheit, die spät einsetzende Variante der CLN6-Krankheit, die CLN6 Krankheit der frühen Jugend, die Batten-Spielmeyer-Vogt- / NCL/CLN3-Krankheit der Jugend, die finnische CLN5-Variante der späten Jugend, Morbus Jansky-Bielschowsky / CLN2/TPP1-Krankheit der späten Jugend, Kufs- / NCL/CLN4-Krankheit mit Einsetzen im Erwachsenenalter, nördliche Epilepsie / CLN8-Variante der späten Kindheit, Santavuori-Haltia- / CLN1/PPT-Krankheit der Kindheit, beta-Mannosidose, Morbus Pompe / eine Glykogenspeicherkrankheit vom Typ II, Pyknodysostose, Sandhoff-Krankheit / Einsetzen im Erwachsenenalter / GM2-Gangliosidose, die Sandhoff-Krankheit / GM2-Gangliosidose - Kindheit, die Sandhoff-Krankheit / GM2-Gangliosidose - Jugend, Morbus Schindler, die Salla-Erkrankung / Sialinsäure-Speicherkrankheit, Morbus Tay-Sachs / GM2-Gangliosidose oder um Morbus Wolman handelt.

12. Verbindung zur Verwendung einem beliebigen der Ansprüche 1 bis 11, wobei die Verbindung die Struktur 85 aufweist: 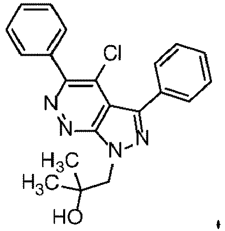 oder ein pharmazeutisch unbedenkliches Salz derselben.

13. Verbindung zur Verwendung einem beliebigen der Ansprüche 1 bis 11, wobei die Verbindung die Struktur 85 aufweist:

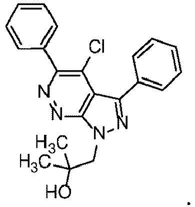

## Revendications

1. Composé de formule II destiné à être utilisé dans un procédé de traitement ou de prévention d'une protéinopathie, comprenant l'administration d'une quantité efficace du composé de formule II à un sujet le nécessitant : 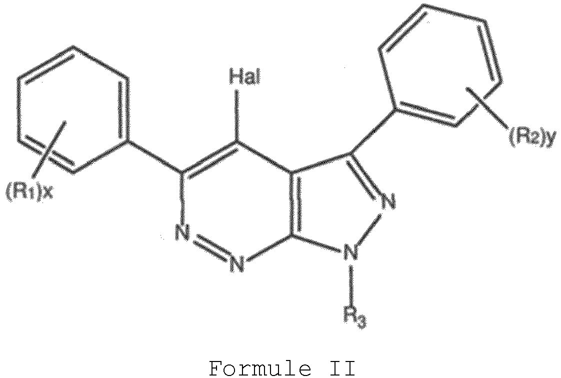
ou d'un sel de celui-ci acceptable d'un point de vue pharmaceutique,
dans lequel Hal est -Cl, -F, -I ou -Br ;
x est un entier allant de 0 à 5 ;
chaque R₁ est indépendamment -Cl, -F, -I, -Br, -C₁-C₃ alkyle, -O-C₁-C₃ alkyle, -CN, -CF₃, -C(O)NH(CH₃) ou - C≡CCH₂OH ;
y est un entier allant de 0 à 5 ;
chaque R₂ est indépendamment -Cl, -F, -Br, -C₁-C₃ alkyle, -O-C₁-C₃ alkyle, -CN, -CF₃, -C(O)NH(CH₃) ou - C≡CCH₂OH ;
R₃ est -H, -C₁-C₆ alkyle, -(C₁-C₆ alkylène)-OH, -(C₁-C₆ alkylène)-phényle, -(C₁-C₆ alkylène)-O-(C₁-C₆ alkyle), - C₂-C₆ alcényle, -(C₁-C₆ alkylène)-C(O)R₄, -(C₁-C₆ alkylène)-R₅, 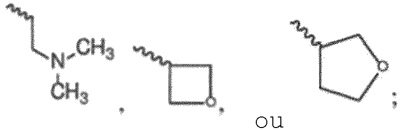
R₄ est -OH, -O-(C₁-C₆ alkyle), -NH₂, -NH(C₁-C₆ alkyle), - NH((C₁-C₆ alkylène)-OH), -NH((C₁-C₆ alkylène)-N(C₁-C₆ alkyle)₂), -N(C₁-C₆ alkyle)((C₁-C₆ alkylène)-CN), -N(C₁-C₆ alkyle)((C₁-C₆ alkylène)N(C₁-C₆ alkyle)₂), -NH(C₁-C₆ alkylène)-O-(C₁-C₆ alkyle), 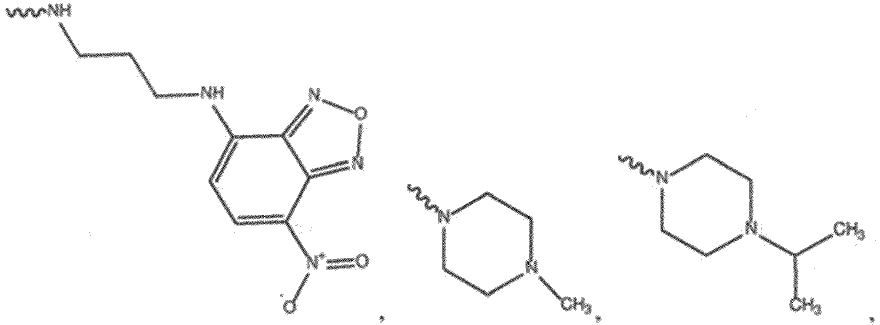 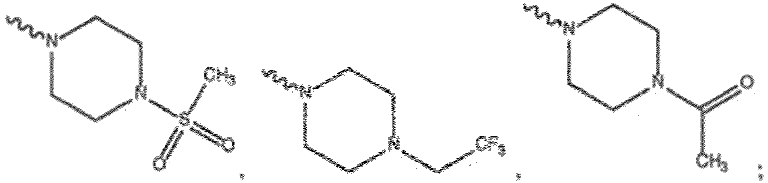 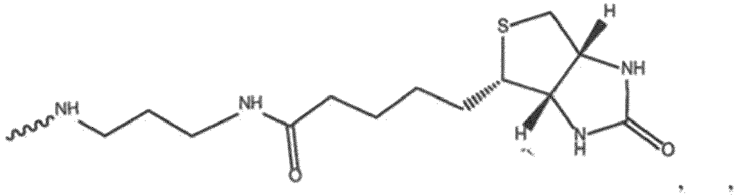 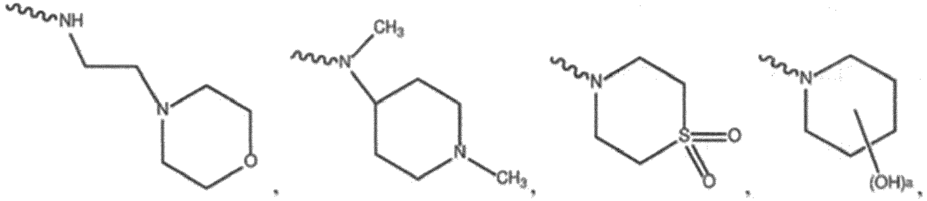 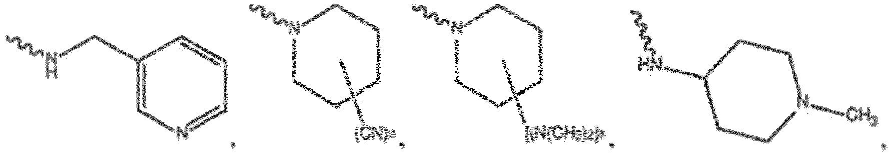 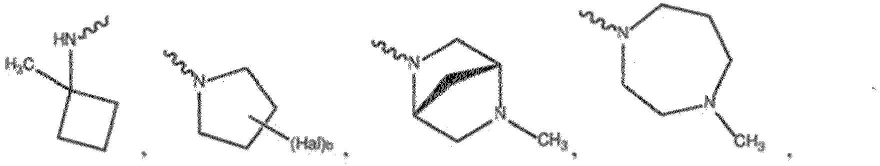 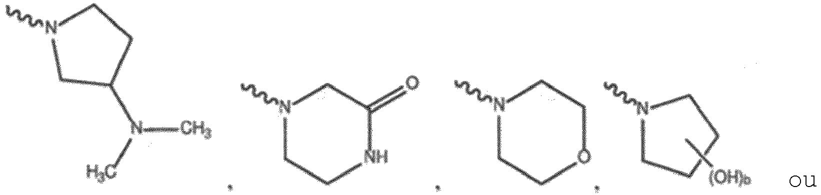 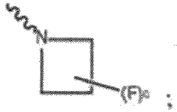
a est un entier allant de 0 à 10 ;
b est un entier allant de 0 à 8 ;
c est un entier allant de 0 à 6 ; et
R₅ est 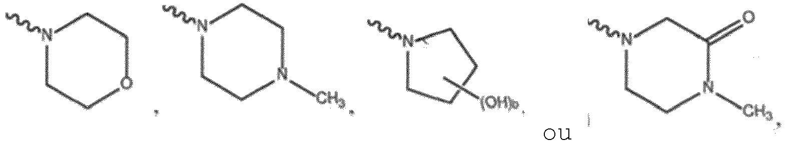
dans lequel la protéinopathie n'est pas une rétinite pigmentaire, l'amaurose congénitale de Leber, une dégénérescence rétinienne syndromique, la dégénérescence maculaire liée à l'âge, le syndrome d'Usher ou une maladie dégénérative de la rétine.

2. Composé destiné à l'utilisation de la revendication 1, le composé ayant la structure : 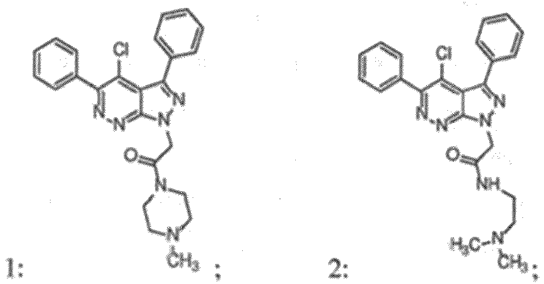 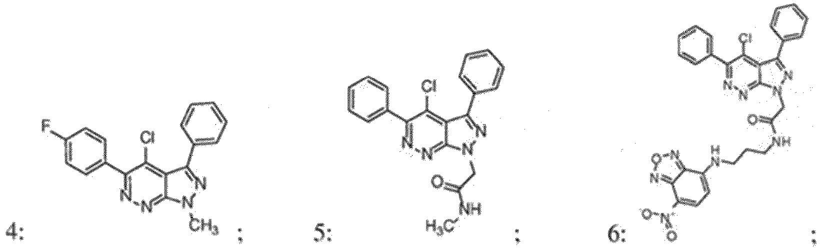 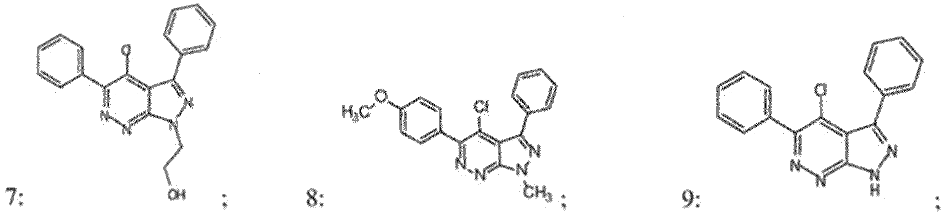 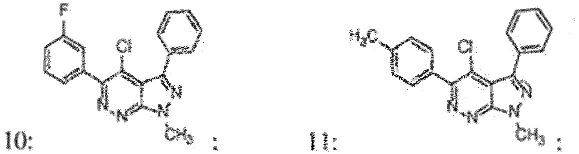 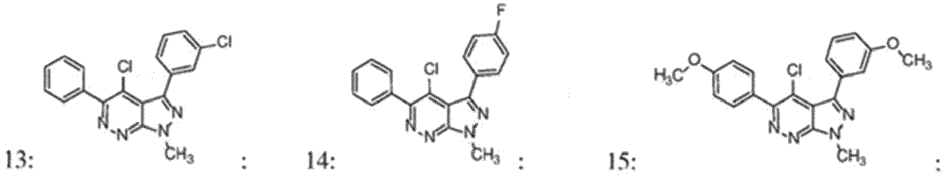 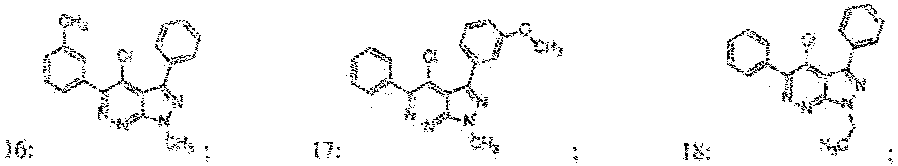 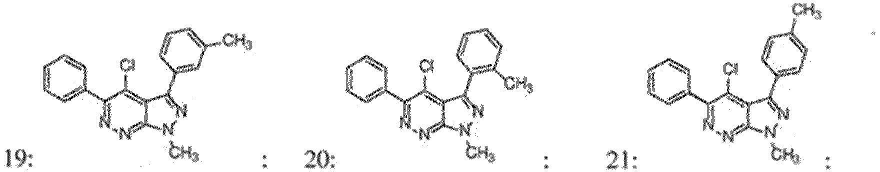 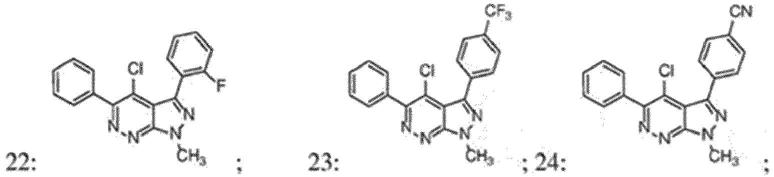 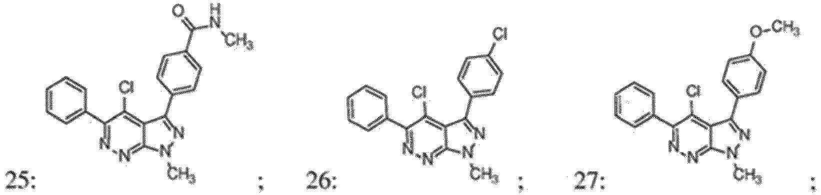 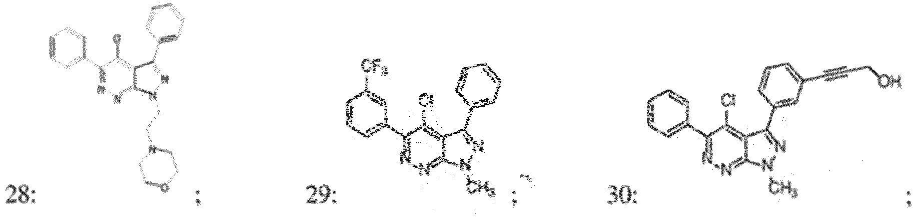 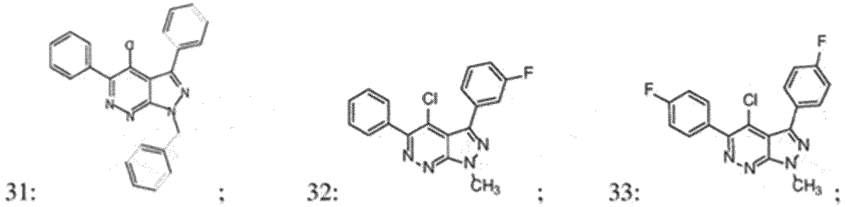 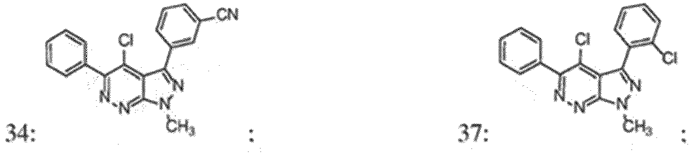 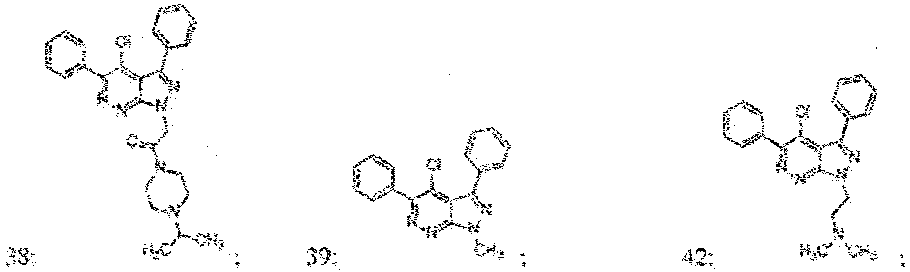 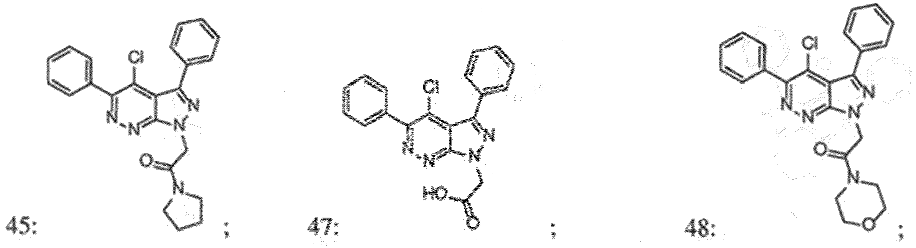 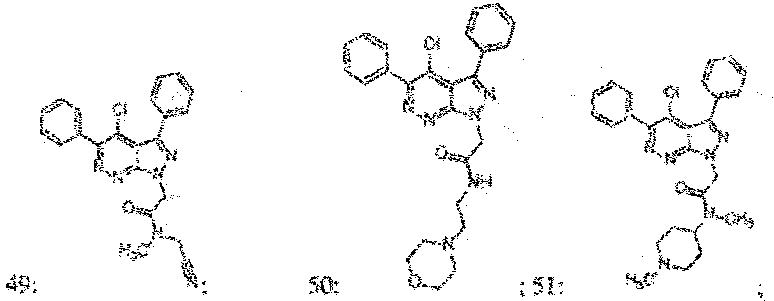 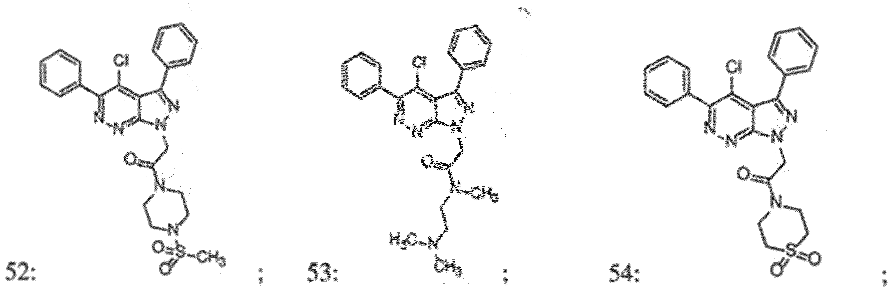 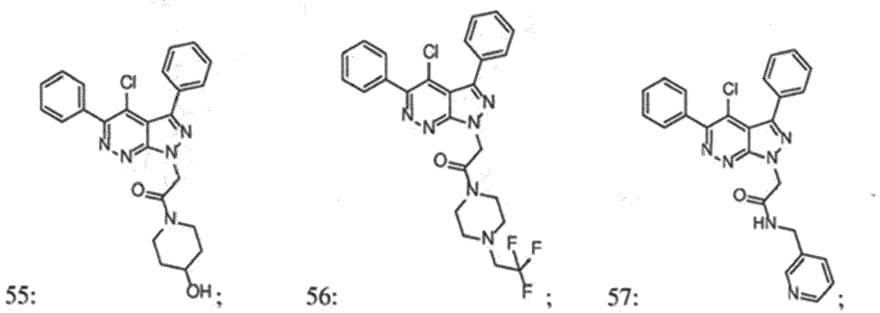 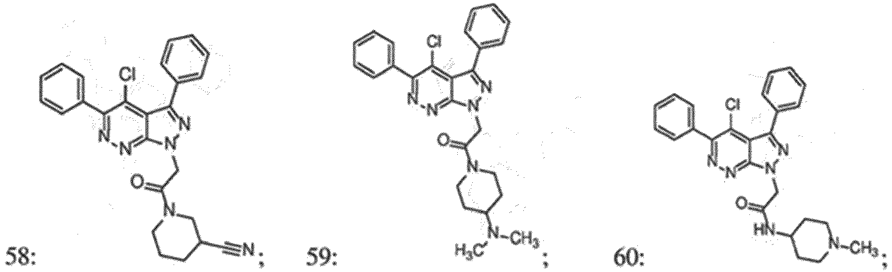 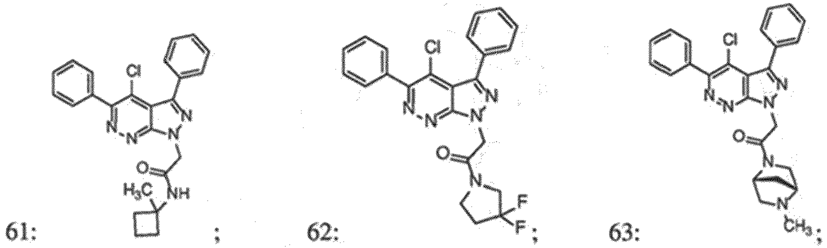 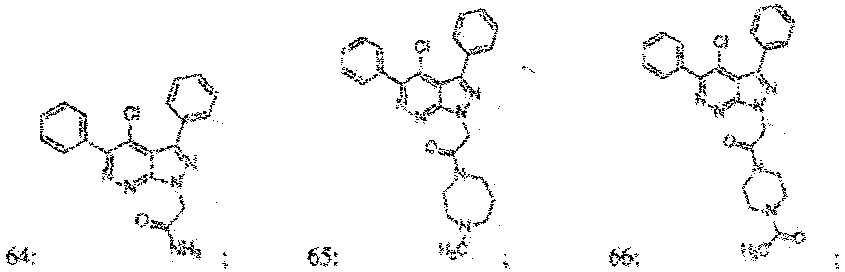 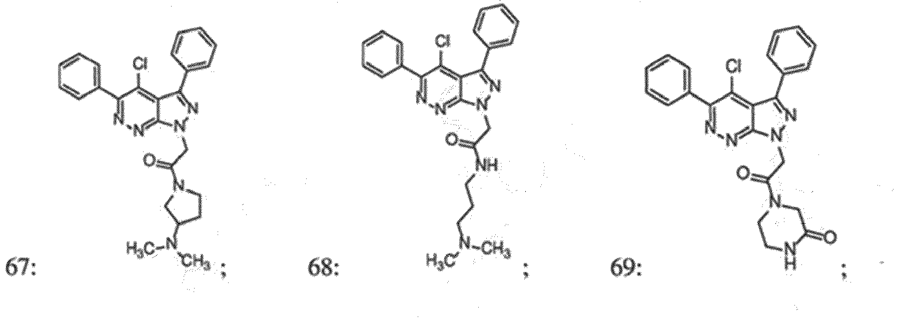 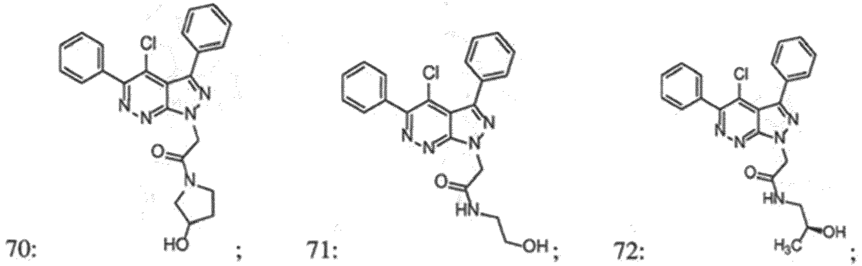 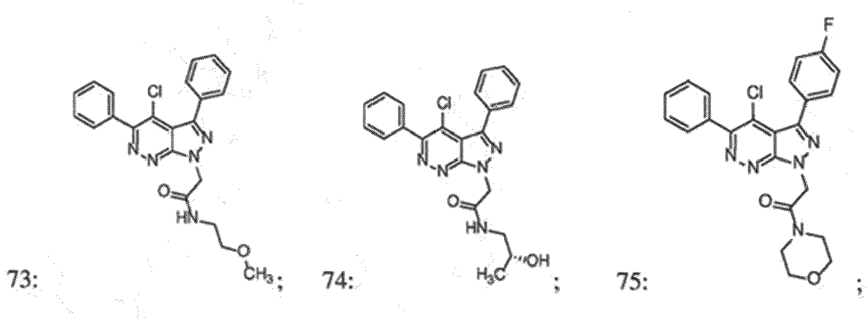 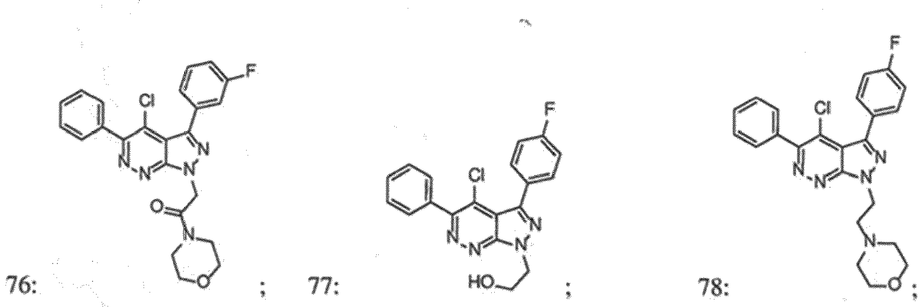 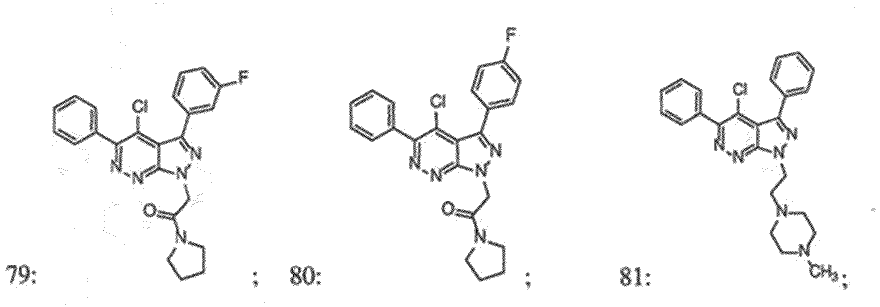 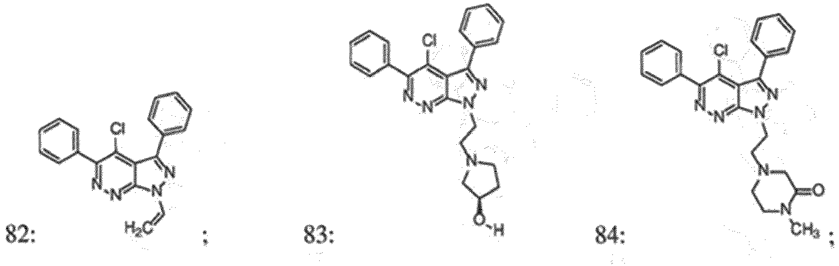 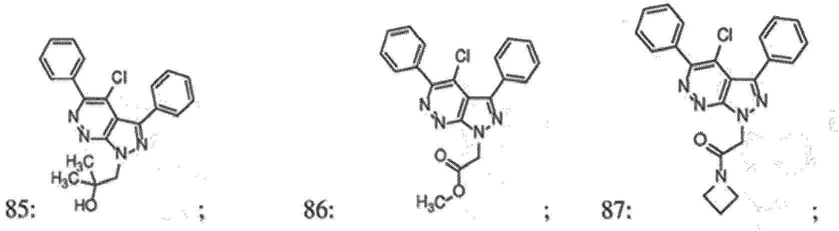 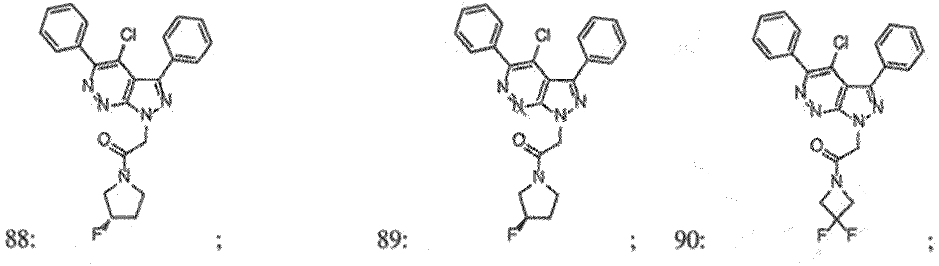 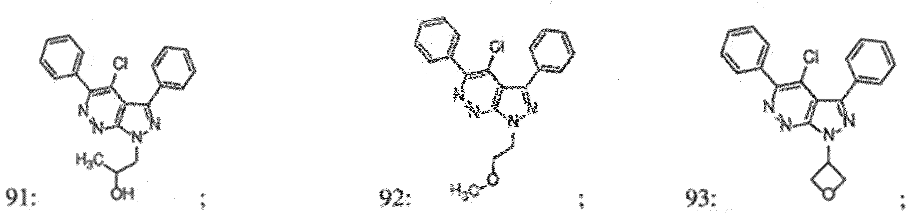 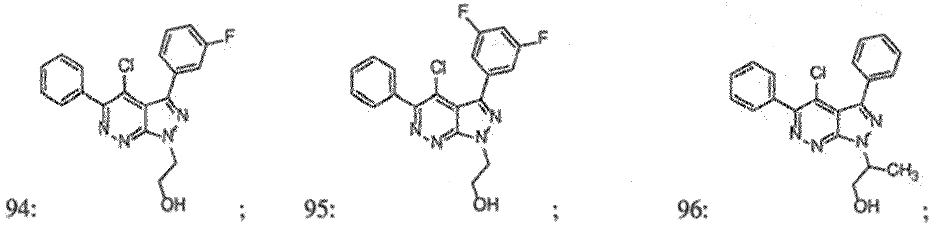 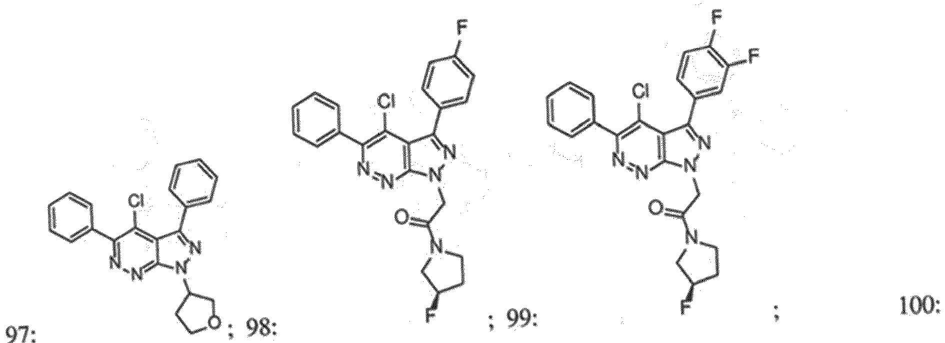 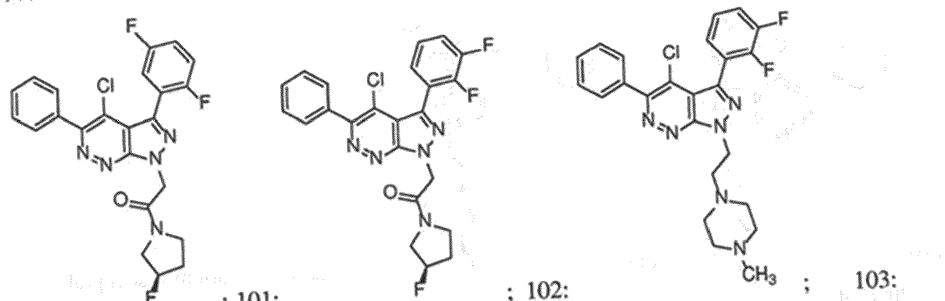 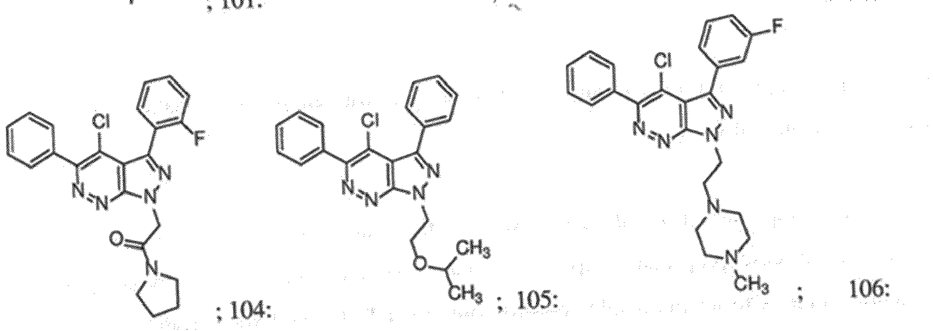 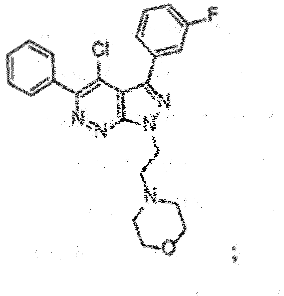 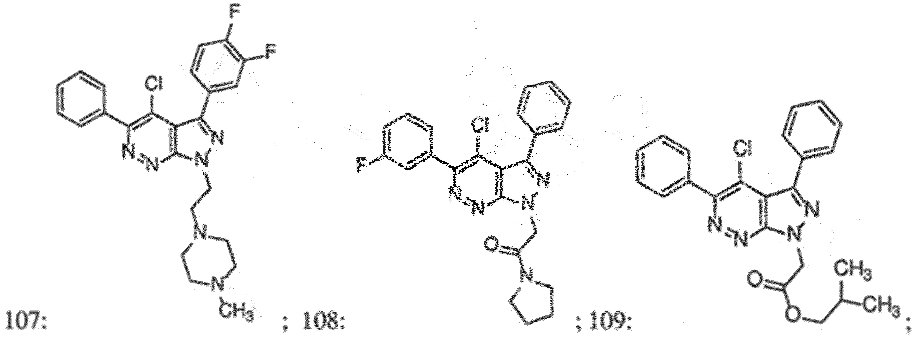 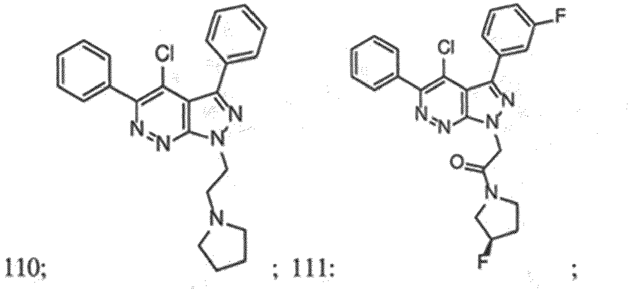 ou sel de celui-ci acceptable d'un point de vue pharmaceutique.

3. Composé destiné à l'utilisation de l'une quelconque des revendications 1-2, dans lequel la protéinopathie est une maladie neurodégénérative.

4. Composé destiné à l'utilisation de la revendication 3, dans lequel la maladie neurodégénérative est la maladie d'Alzheimer, une paralysie supra-nucléaire progressive, la démence pugilistique, une démence fronto-temporale et un parkinsonisme lié au chromosome 17 (FTDP-17), la maladie de Lytico-Bodig, une démence à prédominance d'enchevêtrements, un gangliome, un gangliocytome, une angiomatose méningée, une panencéphalite sclérosante subaiguë, une encéphalopathie due au plomb, la sclérose tubéreuse, la maladie de Hallervorden-Spatz, une lipofuscinose, la maladie de Pick, une dégénérescence cortico-basale, la maladie des grains argyrophiles, la maladie de Huntington, la maladie de Parkinson, une démence à corps de Lewy, une atrophie systémique multiple, des dystrophies neuro-axonales, l'atrophie dentato-rubro-pallido-luysienne (DRPLA), une atrophie musculaire spino-bulbaire (AMSB), l'ataxie spinocérébelleuse 1 (ASC 1), l'ASC 2, l'ASC 3, l'ASC 6, l'ASC 7, l'ASC 17, une maladie à prion, la sclérose latérale amyotrophique, la dégénérescence lobaire fronto-temporale (DLFT) ou une encéphalopathie familiale avec corps d'inclusion de neuroserpine (FENIB).

5. Composé destiné à l'utilisation de l'une quelconque des revendications 1-2, dans lequel la protéinopathie est une amylose.

6. Composé destiné à l'utilisation de la revendication 5, dans lequel l'amylose est la démence familiale type britannique (ABri), la démence familiale type danoise (ADan), une hémorragie cérébrale héréditaire avec amylose type hollandais (HCHWA-I), une neuropathie amyloïde familiale (ATTR), une amylose AL systémique primitive (chaîne légère), une amylose AH (chaîne lourde), une amylose AA secondaire, une amylose Aβ, une amylose médiale aortique, une amylose à LECT2, une amylose à AIAPP, une amylose à apolipoprotéine AI (AApoAI), une amylose à apolipoprotéine AII (AApoAII), une amylose à apolipoprotéine AIV (AApoAIV), l'amylose familiale de type finnoise (FAF), une amylose à fibrinogène (AFib), une amylose à lysozyme (ALys), une amylose liée à une dialyse (Aβ₂M), un carcinome médullaire thyroïdien (ACal), une amylose cardiaque atriale (AANF), un prolactinome pituitaire (APro), une dystrophie cornéenne grillagée héréditaire, le lichen amyloïde cutané (AKer), les corps de Mallory, une amylose cutanée primitive, une amylose cornéenne à lactoferrine, l'amyloïde odontogénique d'une tumeur de Pindborg ou l'amyloïde des vésicules séminales.

7. Composé destiné à l'utilisation de l'une quelconque des revendications 1-2, dans lequel la protéinopathie est une maladie du stockage lysosomal.

8. Composé destiné à l'utilisation de la revendication 7, dans lequel la maladie du stockage lysosomal est un trouble de type muco-polysaccharidose.

9. Composé destiné à l'utilisation de la revendication 8, dans lequel le trouble de type muco-polysaccharidose est une pseudo-polydystrophie de Hurler/mucolipidose IIIA, le syndrome MPS I de Hurler, le syndrome MPS I de Scheie, le syndrome MPS I de Hurler-Scheie, le syndrome MPS II de Hunter, le syndrome de Sanfilippo de type A/MPS III A, le syndrome de Sanfilippo de type B/MPS III B, le syndrome de Sanfilippo de type C/MPS III C, le syndrome de Sanfilippo de type D/MPS III D, le Morquio de type A/MPS IVA, le Morquio de type B/MPS IVB, une déficience en hyaluronidase de MPS IX, la MPS VI de Maroteaux-Lamy, le syndrome MPS VII de Sly, la mucolipidose I/sialidose, la mucolipidose IIIC ou la mucolipidose de type IV.

10. Composé destiné à l'utilisation de la revendication 7, dans lequel la maladie du stockage lysosomal est la maladie de Pompe/maladie du stockage du glycogène de type II.

11. Composé destiné à l'utilisation de la revendication 7, dans lequel la maladie du stockage lysosomal est une déficience d'activateur/gangliosidose à GM2, une alpha-mannosidose, une aspartylglucosaminurie, la maladie du stockage des esters de cholestéryle, une déficience chronique d'hexosaminidase A, une cystinose, la maladie de Danon, la maladie de Fabry, la maladie de Farber, une fucosidose, une galacto-sialidose, la maladie de Gaucher de type I, la maladie de Gaucher de type II, la maladie de Gaucher de type III, une gangliosidose à GM1 infantile, une gangliosidose à GM1 infantile/juvénile tardive, une gangliosidose à GM1 adulte/chronique, la maladie cellule I/mucolipidose II, la maladie du stockage de l'acide sialique libre/ISSD infantile, une déficience juvénile d'hexosaminidase A, la maladie de Krabbe à apparition infantile, la maladie de Krabbe à apparition tardive, une déficience en acide lipase lysosomale à apparition précoce, une déficience en acide lipase lysosomale à apparition tardive, une leucodystrophie métachromatique, une pseudo-polydystrophie de Hurler/mucolipidose IIIA, le syndrome MPS I de Hurler, le syndrome MPS I de Scheie, le syndrome MPS I de Hurler-Scheie, le syndrome MPS II de Hunter, le syndrome de Sanfilippo de type A/MPS III A, le syndrome de Sanfilippo de type B/MPS III B, le syndrome de Sanfilippo de type C/MPS III C, le syndrome de Sanfilippo de type D/MPS III D, le Morquio de type A/MPS IVA, le Morquio de type B/MPS IVB, une déficience en hyaluronidase de MPS IX, la MPS VI de Maroteaux-Lamy, le syndrome MPS VII de Sly, la mucolipidose I/sialidose, la mucolipidose IIIC, la mucolipidose de type IV, une déficience en sulfatase multiple, la maladie de Niemann-Pick de type A, la maladie de Niemann-Pick de type B, la maladie de Niemann-Pick de type C, la maladie CLN6 atypique infantile tardive, un variant de la maladie CLN6 à apparition tardive, la maladie CLN6 juvénile précoce, la maladie NCL/CLN3 juvénile de Batten-Spielmeyer-Vogt, la CLN5 infantile tardive - variant finnois, la maladie de Jansky-Bielschowsky/maladie CLN2/TPP1 infantile tardive, la maladie NCL/CLN4 de Kufs à apparition chez l'adulte, le variant de la CLN8 infantile tardive/à épilepsie nordique, la maladie CLN1/PPT infantile/Santavuori-Haltia, une bêta-mannosidose, la maladie de Pompe/maladie du stockage du glycogène de type II, la pycnodysostose, la maladie de Sandhoff/à apparition chez l'adulte/gangliosidose à GM2, la maladie de Sandhoff/gangliosidose à GM2-infantile, la maladie de Sandhoff/gangliosidose à GM2-juvénile, la maladie de Schindler, la maladie de Salla/maladie du stockage de l'acide sialique, la gangliosidose à GM2/Tay-Sachs ou la maladie de Wolman.

12. Composé destiné à l'utilisation de l'une quelconque des revendications 1-11, le composé ayant la structure 85 : 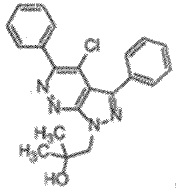 ou sel de celui-ci acceptable d'un point de vue pharmaceutique.

13. Composé destiné à l'utilisation de l'une quelconque des revendications 1-11, le composé ayant la structure 85 :
